# EUROPÄISCHE PATENTSCHRIFT

(11) **EP 1 293 562 B1**
(45) Veröffentlichungstag und Bekanntmachung des Hinweises auf die Patenterteilung: **22.11.2006**
(21) Anmeldenummer: 02019172.2
(22) Anmeldetag: 02.09.2002
(51) Int. Cl.: C12N 9/02, C12R 1/645, C12N 15/53, C12N 15/80, C12N 15/82, C12Q 1/26, C12N 5/10, C07K 16/40, G01N 33/50

(54) **Glyoxal Oxidasen aus Pilzen**
Fungal glyoxal oxidases
Glyoxal oxydases fongiques

(30) Priorität: 13.09.2001 DE 10145095; 04.12.2001 DE 10159375; 16.05.2002 DE 10221725
(43) Veröffentlichungstag der Anmeldung: 19.03.2003
(73) Patentinhaber: Bayer CropScience AG, 40789 Monheim (DE)
(72) Erfinder: Aichinger, Christian, Dr., 50939 Köln (DE); Schreier, Peter, Prof. Dr., 50674 Köln (DE); Leuthner, Brigitta, Dr., 40764 Langenfeld (DE); Adamczewski, Martin, Dr., 51067 Köln (DE); Hillebrand, Stefan, Dr., 41462 Neuss (DE); Kuck, Karl-Heinz, Dr., 40764 Langenfeld (DE); van Kan, Johannes, Arnoldus, Laurentius, Dr., NL-3911 JP Rhenen (NL); Visser, Jaap, Dr., 6703 CK Wageningen (NL); Stefanato, Francesca, Maria, Dr., 8057 Zürich (CH); Kahmann, Regine, Prof. Dr., 35037 Marburg (DE); Bölker, Michael, Prof. Dr., 35037 Marburg (DE)

(56) Entgegenhaltungen:
- WO-A-98/37203
- US-A- 3 689 536
- DATABASE EMBL [Online] BITTON F. ET AL.: "Botrytis cinerea strain T4 cDNA library under conditions of nitrogen deprivation" Database accession no. AL113811 XP002222291
- KERSTEN PHILIP J ET AL: "Phanerochaete chrysosporium glyoxal oxidase is encoded by two allelic variants: Structure, genomic organization, and heterologous expression of glx1 and glx2." JOURNAL OF BACTERIOLOGY, Bd. 177, Nr. 21, 1995, Seiten 6106-6110, XP002218567 ISSN: 0021-9193
- WHITTAKER MEI M ET AL: "Glyoxal oxidase from Phanerochaete chrysosporium is a new radical-copper oxidase." JOURNAL OF BIOLOGICAL CHEMISTRY, Bd. 271, Nr. 2, 1996, Seiten 681-687, XP002218568 ISSN: 0021-9258
- KELLEHER F M ET AL: "RE-EXAMINATION OF THE PRODUCTS OF THE ACTION OF GALACTOSE OXIDASE EVIDENCE FOR THE CONVERSION OF RAFFINOSE TO 6 CARBOXYRAFFINOSE" JOURNAL OF BIOLOGICAL CHEMISTRY, Bd. 261, Nr. 24, 1986, Seiten 11045-11048, XP002222290 ISSN: 0021-9258
- BÖLKER M ET AL: "Tagging pathogenicity genes in Ustilago maydis by restriction enzyme-mediated integration (REMI)." MOLECULAR & GENERAL GENETICS: MGG. GERMANY 20 SEP 1995, Bd. 248, Nr. 5, 20. September 1995 (1995-09-20), Seiten 547-552, XP001118302 ISSN: 0026-8925
- KAHMANN REGINE ET AL: "REMI (restriction enzyme mediated integration) and its impact on the isolation of pathogenicity genes in fungi attacking plants." EUROPEAN JOURNAL OF PLANT PATHOLOGY, Bd. 105, Nr. 3, April 1999 (1999-04), Seiten 221-229, XP009001384 ISSN: 0929-1873

### SEQUENCE LISTING

<110> Bayer AG
<120> Glyoxaloxidasen aus Pilzen
<130> Le A 35 261
<160> 12
<170> Patent In version 3.0
<210> 1
   <211> 2589
   <212> DNA
   <213> Ustilago maydis
<220>
   <221> CDS
   <222> (1)..(2589)
<400> 1
   atg acg agg cac ctc tcc tca tcc tcg agg cgc tcc tcg ctc gcc aaa        48
   Met Thr Arg His Leu Ser Ser Ser Ser Arg Arg Ser Ser Leu Ala Lys
   1               5                   10                  15
   agc gcc atg acc ctc gca acc ctt tct ctc gcc cta acc tcg tgc gca        96
   Ser Ala Met Thr Leu Ala Thr Leu Ser Leu Ala Leu Thr Ser Cys Ala
               20                  25                  30
   tcg gcc gcc agc aag gcc ggc tca tac gag gtt gtc aac acc aac tca       144
   Ser Ala Ala Ser Lys Ala Gly Ser Tyr Glu Val Val Asn Thr Asn Ser
           35                  40                  45
   ctc gcc tcg gcc atg atg ctc ggt tta atg gac gag gac aac gtc ttt       192
   Leu Ala Ser Ala Met Met Leu Gly Leu Met Asp Glu Asp Asn Val Phe
       50                  55                  60
   att ctc gac aaa gct gaa aac aac tcg gct cgt ctc gcc gat ggc cgt       240
   Ile Leu Asp Lys Ala Glu Asn Asn Ser Ala Arg Leu Ala Asp Gly Arg
   65                  70                  75                  80
   cat gtc tgg ggt tct ttc tac aag ctt tcc gac aat tcg gtc acc ggc       288
   His Val Trp Gly Ser Phe Tyr Lys Leu Ser Asp Asn Ser Val Thr Gly
                   85                  90                  95
   acc gcc gtc cag acc aac act ttc tgt gcc tct ggt gcc acc ttg gga       336
   Thr Ala Val Gln Thr Asn Thr Phe Cys Ala Ser Gly Ala Thr Leu Gly
               100                 105                 110
   aat ggt tct tgg ctt gta gct ggc ggc aac cag gcc gta ggt tac ggt      384
   Asn Gly Ser Trp Leu Val Ala Gly Gly Asn Gln Ala Val Gly Tyr Gly
           115                 120                 125
   ggc gct gca cag gcc cag gag atc aac ccc tac tcg gac ttc gac gga      432
   Gly Ala Ala Gln Ala Gln Glu Ile Asn Pro Tyr Ser Asp Phe Asp Gly
       130                 135                 140
   act agg gcg att cgt ctg ctc gaa ccc aac tcg cag acg tgg atc gac      480
   Thr Arg Ala Ile Arg Leu Leu Glu Pro Asn Ser Gln Thr Trp Ile Asp
   145                 150                 155                 160
   tcg ccc agt aca act gtc gca cag gtc aac atg ctc cag caa ccc cgt      528
   Ser Pro Ser Thr Thr Val Ala Gln Val Asn Met Leu Gln Gln Pro Arg
                   165                 170                 175
   tgg tac ccc ggt atc gag gtt ctt gaa gac ggt agc gtt atc ttt atc      576
   Trp Tyr Pro Gly Ile Glu Val Leu Glu Asp Gly Ser Val Ile Phe Ile
               180                 185                 190
   gga ggt gcc gtc tcg ggc ggc tac att aat cgc aac acg cct acc act      624
   Gly Gly Ala Val Ser Gly Gly Tyr Ile Asn Arg Asn Thr Pro Thr Thr
           195                 200                 205
   gat cct ctt tac cag aat gga ggc gct aac ccc acc tac gaa tac ttt      672
   Asp Pro Leu Tyr Gln Asn Gly Gly Ala Asn Pro Thr Tyr Glu Tyr Phe
       210                 215                 220
   ccc tcc aag acc acc gga aac cta ccc atc tgt aac ttt atg gct cag      720
   Pro Ser Lys Thr Thr Gly Asn Leu Pro Ile Cys Asn Phe Met Ala Gln
   225                 230                 235                 240
   act aac ggt ctc aac atg tac ccg cac acc tac ctc atg ccc tct ggc      768
   Thr Asn Gly Leu Asn Met Tyr Pro His Thr Tyr Leu Met Pro Ser Gly
                   245                 250                 255
   aag atc ttc atg cag gcc aac gtc agt acc atc ctc tgg gac cac gtc      816
   Lys Ile Phe Met Gln Ala Asn Val Ser Thr Ile Leu Trp Asp His Val
               260                 265                 270
   aac aac act cag atc gac ctt ccc gac atg cct ggc gga gtc gtg cgc      864
   Asn Asn Thr Gln Ile Asp Leu Pro Asp Met Pro Gly Gly Val Val Arg
           275                 280                 285
   gtc tac ccc gcc tcg gct gcc act gcc atg ctg cca ctc act cct cag      912
   Val Tyr Pro Ala Ser Ala Ala Thr Ala Met Leu Pro Leu Thr Pro Gln
       290                 295                 300
   aat cag tac aca cct acc atc ctg ttt tgc ggt ggt agt gtc atg agc      960
   Asn Gln Tyr Thr Pro Thr Ile Leu Phe Cys Gly Gly Ser Val Met Ser
   305                 310                 315                 320
   gac cag atg tgg ggc aac tac agt ggt ccc ggt ggc aac att ctc ggt     1008
   Asp Gln Met Trp Gly Asn Tyr Ser Gly Pro Gly Gly Asn Ile Leu Gly
                   325                 330                 335
   ctc caa gcc tct gat gac tgc tcg tcc atc aac ccc gag gac aat cag     1056
   Leu Gln Ala Ser Asp Asp Cys Ser Ser Ile Asn Pro Glu Asp Asn Gln
               340                 345                 350
   ggc aac cag atc act gac gct cag tac gtc cag gag ggg cgg ctt ccc     1104
   Gly Asn Gln Ile Thr Asp Ala Gln Tyr Val Gln Glu Gly Arg Leu Pro
               355                 360                 365
   gaa ggt cgt tcc atg gga cag ttc atc cac ctc cct gac ggt acc atg      1152
   Glu Gly Arg Ser Met Gly Gln Phe Ile His Leu Pro Asp Gly Thr Met
           370                 375                 380
   gtc gtc ctc aac ggc gcc aac aag gga act gcc ggc tat tcg aac cag      1200
   Val Val Leu Asn Gly Ala Asn Lys Gly Thr Ala Gly Tyr Ser Asn Gln
   385                 390                 395                 400
   aca tgg aac acc atc cag tac aac ggt cgc acc gtc gtc acc gaa ggt      1248
   Thr Trp Asn Thr Ile Gln Tyr Asn Gly Arg Thr Val Val Thr Glu Gly
                   405                 410                 415
   ctt tcg cag gat ccc act tac gtt ccc gtc atc tat gac ccg tcc aag      1296
   Leu Ser Gln Asp Pro Thr Tyr Val Pro Val Ile Tyr Asp Pro Ser Lys
               420                 425                 430
   ccc aga ggt cag cgt ctc tcc aat gct aat ctc aag cct tcc acc att      1344
   Pro Arg Gly Gln Arg Leu Ser Asn Ala Asn Leu Lys Pro Ser Thr Ile
           435                 440                 445
   gct cgt ctc tac cac tcg agc gct att ttg ctc ccc gat ggt tcc gtc      1392
   Ala Arg Leu Tyr His Ser Ser Ala Ile Leu Leu Pro Asp Gly Ser Val
       450                 455                 460
   atg gtt gca ggt tcc aac ccg cat cag gat gtt gcg ctc gac atg ccc      1440
   Met Val Ala Gly Ser Asn Pro His Gln Asp Val Ala Leu Asp Met Pro
   465                 470                 475                 480
   acc ggc acc acg cct cag gct ttc aac acc acc tac gag gtt gaa aag      1488
   Thr Gly Thr Thr Pro Gln Ala Phe Asn Thr Thr Tyr Glu Val Glu Lys
                   485                 490                 495
   tgg tac cct cct tac tgg gac tcg cca cgc cct tac cca cag ggc gtg      1536
   Trp Tyr Pro Pro Tyr Trp Asp Ser Pro Arg Pro Tyr Pro Gln Gly Val
               500                 505                 510
   ccc aat tcg gtg ctg tac ggc ggc agt cct ttc aac att acc gtc aac      1584
   Pro Asn Ser Val Leu Tyr Gly Gly Ser Pro Phe Asn Ile Thr Val Asn
           515                 520                 525
   ggt acc ttt atg ggt gac tcg gcc aac gcc aag gca gcc aac acc aag      1632
   Gly Thr Phe Met Gly Asp Ser Ala Asn Ala Lys Ala Ala Asn Thr Lys
       530                 535                 540
   ttt gcc atc att cgt acc ggt ttc tcc acc cac gcc atg aac atg ggg      1680
   Phe Ala Ile Ile Arg Thr Gly Phe Ser Thr His Ala Met Asn Met Gly
   545                 550                 555                 560
   cag cgc gcc gtc tac ctc gac tac acc tac acc gtt aac gat gac gcc      1728
   Gln Arg Ala Val Tyr Leu Asp Tyr Thr Tyr Thr Val Asn Asp Asp Ala
                   565                 570                 575
   tcg gtc acc tac atg gtc aac cct ttg ccc aac act aag gct atg aac      1776
   Ser Val Thr Tyr Met Val Asn Pro Leu Pro Asn Thr Lys Ala Met Asn
               580                 585                 590
   cgc ctc ttt gtg cct ggc ccg gcc ttc ttc tac gtc acc gtc ggt ggc      1824
   Arg Leu Phe Val Pro Gly Pro Ala Phe Phe Tyr Val Thr Val Gly Gly
           595                 600                 605
   gtg cca agc cat ggc aag ctg atc atg gtg gga act tcc ccc act ggc      1872
   Val Pro Ser His Gly Lys Leu Ile Met Val Gly Thr Ser Pro Thr Gly
       610                 615                 620
   act ggc aac gtc ccc ttc act cct cag ctc ggg tct gca ctc gtc gcg     1920
   Thr Gly Asn Val Pro Phe Thr Pro Gln Leu Gly Ser Ala Leu Val Ala
   625                 630                 635                 640
   ctt ccc cct gct gtc aac agc acc aaa ttc aca gcc tcc ctc ccc aag     1968
   Leu Pro Pro Ala Val Asn Ser Thr Lys Phe Thr Ala Ser Leu Pro Lys
                   645                 650                 655
   gct ggc agc agc tct tcc tcc gag ttt ggc ctc ggc aag atc att ggt     2016
   Ala Gly Ser Ser Ser Ser Ser Glu Phe Gly Leu Gly Lys Ile Ile Gly
               660                 665                 670
   atc gct gtt gct ggc gcc gca gtt ttg gcc ctc att gct ctc ggc tgt     2064
   Ile Ala Val Ala Gly Ala Ala Val Leu Ala Leu Ile Ala Leu Gly Cys
           675                 680                 685
   tgt ctg tgg agg cgc aag ggc agg agc cat agc gac aag gct gcc tcg     2112
   Cys Leu Trp Arg Arg Lys Gly Arg Ser His Ser Asp Lys Ala Ala Ser
       690                 695                 700
   cgc cag tcg gct gcc cct tgg acc agc cgc gac ctt ggc tcg ggt ccc     2160
   Arg Gln Ser Ala Ala Pro Trp Thr Ser Arg Asp Leu Gly Ser Gly Pro
   705                 710                 715                 720
   gag tac aag cgt gtc gac act cct gtc gga tcc atc agc ggt ggt cgc     2208
   Glu Tyr Lys Arg Val Asp Thr Pro Val Gly Ser Ile Ser Gly Gly Arg
                   725                 730                 735
   ttt ggg gcc gcc agg atg gac agc tcg aat acg ttt gag agc tat cgg     2256
   Phe Gly Ala Ala Arg Met Asp Ser Ser Asn Thr Phe Glu Ser Tyr Arg
               740                 745                 750
   ttg cac gac cag gtc agc acg agc gaa agc aag gag gcg att ggc agc     2304
   Leu His Asp Gln Val Ser Thr Ser Glu Ser Lys Glu Ala Ile Gly Ser
           755                 760                 765
   tac tac gac caa cct cgc agc ggc agc cgt ggc ggc tac gct cct agc     2352
   Tyr Tyr Asp Gln Pro Arg Ser Gly Ser Arg Gly Gly Tyr Ala Pro Ser
       770                 775                 780
   ccg ctc gcc tac gac caa cac gga cgt ggc gcc tcg caa ggc cag tac     2400
   Pro Leu Ala Tyr Asp Gln His Gly Arg Gly Ala Ser Gln Gly Gln Tyr
   785                 790                 795                 800
   cac cag caa ggc tgg ggc gaa tac cac gct ggc gat gct ggt gca tac     2448
   His Gln Gln Gly Trp Gly Glu Tyr His Ala Gly Asp Ala Gly Ala Tyr
                   805                 810                 815
   tac gag gac aac act agc agg tac ggc agc ggt ggc ggt gga cac agc     2496
   Tyr Glu Asp Asn Thr Ser Arg Tyr Gly Ser Gly Gly Gly Gly His Ser
               820                 825                 830
   tac gat gat tac tcg cac cag caa tac caa cag cag cat tac tat gac     2544
   Tyr Asp Asp Tyr Ser His Gln Gln Tyr Gln Gln Gln His Tyr Tyr Asp
           835                 840                 845
   agc cca ggt cat cag cac caa gga agc tac tct agt cga cgc taa         2589
   Ser Pro Gly His Gln His Gln Gly Ser Tyr Ser Ser Arg Arg
       850                 855                 860
<210> 2
   <211> 862
   <212> PRT
   <213> Ustilago maydis
<400> 2
   Met Thr Arg His Leu Ser Ser Ser Ser Arg Arg Ser Ser Leu Ala Lys
   1               5                   10                  15
   Ser Ala Met Thr Leu Ala Thr Leu Ser Leu Ala Leu Thr Ser Cys Ala
               20                  25                  30
   Ser Ala Ala Ser Lys Ala Gly Ser Tyr Glu Val Val Asn Thr Asn Ser
           35                  40                  45
   Leu Ala Ser Ala Met Met Leu Gly Leu Met Asp Glu Asp Asn Val Phe
       50                  55                  60
   Ile Leu Asp Lys Ala Glu Asn Asn Ser Ala Arg Leu Ala Asp Gly Arg
   65                  70                  75                  80
   His Val Trp Gly Ser Phe Tyr Lys Leu Ser Asp Asn Ser Val Thr Gly
                   85                  90                  95
   Thr Ala Val Gln Thr Asn Thr Phe Cys Ala Ser Gly Ala Thr Leu Gly
               100                 105                 110
   Asn Gly Ser Trp Leu Val Ala Gly Gly Asn Gln Ala Val Gly Tyr Gly
           115                 120                 125
   Gly Ala Ala Gln Ala Gln Glu Ile Asn Pro Tyr Ser Asp Phe Asp Gly
       130                 135                 140
   Thr Arg Ala Ile Arg Leu Leu Glu Pro Asn Ser Gln Thr Trp Ile Asp
   145                 150                 155                 160
   Ser Pro Ser Thr Thr Val Ala Gln Val Asn Met Leu Gln Gln Pro Arg
                   165                 170                 175
   Trp Tyr Pro Gly Ile Glu Val Leu Glu Asp Gly Ser Val Ile Phe Ile
               180                 185                 190
   Gly Gly Ala Val Ser Gly Gly Tyr Ile Asn Arg Asn Thr Pro Thr Thr
           195                 200                 205
   Asp Pro Leu Tyr Gln Asn Gly Gly Ala Asn Pro Thr Tyr Glu Tyr Phe
       210             215             220
   Pro Ser Lys Thr Thr Gly Asn Leu Pro Ile Cys Asn Phe Met Ala Gln
   225             230             235             240
   Thr Asn Gly Leu Asn Met Tyr Pro His Thr Tyr Leu Met Pro Ser Gly
               245             250             255
   Lys Ile Phe Met Gln Ala Asn Val Ser Thr Ile Leu Trp Asp His Val
           260             265             270
   Asn Asn Thr Gln Ile Asp Leu Pro Asp Met Pro Gly Gly Val Val Arg
       275             280             285
   Val Tyr Pro Ala Ser Ala Ala Thr Ala Met Leu Pro Leu Thr Pro Gln
       290             295             300
   Asn Gln Tyr Thr Pro Thr Ile Leu Phe Cys Gly Gly Ser Val Met Ser
   305             310             315             320
   Asp Gln Met Trp Gly Asn Tyr Ser Gly Pro Gly Gly Asn Ile Leu Gly
               325             330             335
   Leu Gln Ala Ser Asp Asp Cys Ser Ser Ile Asn Pro Glu Asp Asn Gln
           340             345             350
   Gly Asn Gln Ile Thr Asp Ala Gln Tyr Val Gln Glu Gly Arg Leu Pro
       355             360             365
   Glu Gly Arg Ser Met Gly Gln Phe Ile His Leu Pro Asp Gly Thr Met
       370             375             380
   Val Val Leu Asn Gly Ala Asn Lys Gly Thr Ala Gly Tyr Ser Asn Gln
   385             390             395             400
   Thr Trp Asn Thr Ile Gln Tyr Asn Gly Arg Thr Val Val Thr Glu Gly
               405             410             415
   Leu Ser Gln Asp Pro Thr Tyr Val Pro Val Ile Tyr Asp Pro Ser Lys
           420             425             430
   Pro Arg Gly Gln Arg Leu Ser Asn Ala Asn Leu Lys Pro Ser Thr Ile
       435             440             445
   Ala Arg Leu Tyr His Ser Ser Ala Ile Leu Leu Pro Asp Gly Ser Val
   450 455 460
   Met Val Ala Gly Ser Asn Pro His Gln Asp Val Ala Leu Asp Met Pro
   465 470 475 480
   Thr Gly Thr Thr Pro Gln Ala Phe Asn Thr Thr Tyr Glu Val Glu Lys
   485 490 495
   Trp Tyr Pro Pro Tyr Trp Asp Ser Pro Arg Pro Tyr Pro Gln Gly Val
   500 505 510
   Pro Asn Ser Val Leu Tyr Gly Gly Ser Pro Phe Asn Ile Thr Val Asn
   515 520 525
   Gly Thr Phe Met Gly Asp Ser Ala Asn Ala Lys Ala Ala Asn Thr Lys
   530 535 540
   Phe Ala Ile Ile Arg Thr Gly Phe Ser Thr His Ala Met Asn Met Gly
   545 550 555 560
   Gln Arg Ala Val Tyr Leu Asp Tyr Thr Tyr Thr Val Asn Asp Asp Ala
   565 570 575
   Ser Val Thr Tyr Met Val Asn Pro Leu Pro Asn Thr Lys Ala Met Asn
   580 585 590
   Arg Leu Phe Val Pro Gly Pro Ala Phe Phe Tyr Val Thr Val Gly Gly
   595 600 605
   Val Pro Ser His Gly Lys Leu Ile Met Val Gly Thr Ser Pro Thr Gly
   610 615 620
   Thr Gly Asn Val Pro Phe Thr Pro Gln Leu Gly Ser Ala Leu Val Ala
   625 630 635 640
   Leu Pro Pro Ala Val Asn Ser Thr Lys Phe Thr Ala Ser Leu Pro Lys
   645 650 655
   Ala Gly Ser Ser Ser Ser Ser Glu Phe Gly Leu Gly Lys Ile Ile Gly
   660 665 670
   Ile Ala Val Ala Gly Ala Ala Val Leu Ala Leu Ile Ala Leu Gly Cys
   675 680 685
   Cys Leu Trp Arg Arg Lys Gly Arg Ser His Ser Asp Lys Ala Ala Ser
   690 695 700
   Arg Gln Ser Ala Ala Pro Trp Thr Ser Arg Asp Leu Gly Ser Gly Pro
   705                 710                 715                 720
   Glu Tyr Lys Arg Val Asp Thr Pro Val Gly Ser Ile Ser Gly Gly Arg
                   725                 730                 735
   Phe Gly Ala Ala Arg Met Asp Ser Ser Asn Thr Phe Glu Ser Tyr Arg
               740                 745                 750
   Leu His Asp Gln Val Ser Thr Ser Glu Ser Lys Glu Ala Ile Gly Ser
           755                 760                 765
   Tyr Tyr Asp Gln Pro Arg Ser Gly Ser Arg Gly Gly Tyr Ala Pro Ser
       770                 775                 780
   Pro Leu Ala Tyr Asp Gln His Gly Arg Gly Ala Ser Gln Gly Gln Tyr
   785                 790                 795                 800
   His Gln Gln Gly Trp Gly Glu Tyr His Ala Gly Asp Ala Gly Ala Tyr
                   805                 810                 815
   Tyr Glu Asp Asn Thr Ser Arg Tyr Gly Ser Gly Gly Gly Gly His Ser
               820                 825                 830
   Tyr Asp Asp Tyr Ser His Gln Gln Tyr Gln Gln Gln His Tyr Tyr Asp
           835                 840                 845
   Ser Pro Gly His Gln His Gln Gly Ser Tyr Ser Ser Arg Arg
       850                 855                 860
<210> 3
   <211> 2923
   <212> DNA
   <213> Ustilago maydis
<220>
   <221> CDS
   <222> (238)..(2823)
<400> 3
   acgttccttc tcccttttcc tcgctttcac cactgcctcg acgttccttc tttggcttct      60
   gcagttctga ctgttgccac tttttcgtcc cctccgtctc gcctttgatt tatcaccacc    120
   gcgcactcat tggctgcggc gaattaccac gctttgggct cacgccatcc atcgctcagc    180
   cacatttcca ttcaatatca ctgagctctg tcttccagaa aggatcgttt acacacc       237
   atg acg agg cac ctc tcc tca tcc tcg agg cgc tcc tcg ctc gcc aaa      285
   Met Thr Arg His Leu Ser Ser Ser Ser Arg Arg Ser Ser Leu Ala Lys
   1               5                   10                  15
   agc gcc atg acc ctc gca acc ctt tct ctc gcc cta acc tcg tgc gca      333
   Ser Ala Met Thr Leu Ala Thr Leu Ser Leu Ala Leu Thr Ser Cys Ala
               20                  25                  30
   tcg gcc gcc agc aag gcc ggc tca tac gag gtt gtc aac acc aac tca      381
   Ser Ala Ala Ser Lys Ala Gly Ser Tyr Glu Val Val Asn Thr Asn Ser
           35                  40                  45
   ctc gcc tcg gcc atg atg ctc ggt tta atg gac gag gac aac gtc ttt      429
   Leu Ala Ser Ala Met Met Leu Gly Leu Met Asp Glu Asp Asn Val Phe
       50                  55                  60
   att ctc gac aaa gct gaa aac aac tcg gct cgt ctc gcc gat ggc cgt      477
   Ile Leu Asp Lys Ala Glu Asn Asn Ser Ala Arg Leu Ala Asp Gly Arg
   65                  70                  75                  80
   cat gtc tgg ggt tct ttc tac aag ctt tcc gac aat tcg gtc acc ggc      525
   His Val Trp Gly Ser Phe Tyr Lys Leu Ser Asp Asn Ser Val Thr Gly
                   85                  90                  95
   acc gcc gtc cag acc aac act ttc tgt gcc tct ggt gcc acc ttg gga      573
   Thr Ala Val Gln Thr Asn Thr Phe Cys Ala Ser Gly Ala Thr Leu Gly
               100                 105                 110
   aat ggt tct tgg ctt gta gct ggc ggc aac cag gcc gta ggt tac ggt      621
   Asn Gly Ser Trp Leu Val Ala Gly Gly Asn Gln Ala Val Gly Tyr Gly
           115                 120                 125
   ggc gct gca cag gcc cag gag atc aac ccc tac tcg gac ttc gac gga      669
   Gly Ala Ala Gln Ala Gln Glu Ile Asn Pro Tyr Ser Asp Phe Asp Gly
       130                 135                 140
   act agg gcg att cgt ctg ctc gaa ccc aac tcg cag acg tgg atc gac      717
   Thr Arg Ala Ile Arg Leu Leu Glu Pro Asn Ser Gln Thr Trp Ile Asp
   145                 150                 155                 160
   tcg ccc agt aca act gtc gca cag gtc aac atg ctc cag caa ccc cgt      765
   Ser Pro Ser Thr Thr Val Ala Gln Val Asn Met Leu Gln Gln Pro Arg
                   165                 170                 175
   tgg tac ccc ggt atc gag gtt ctt gaa gac ggt agc gtt atc ttt atc      813
   Trp Tyr Pro Gly Ile Glu Val Leu Glu Asp Gly Ser Val Ile Phe Ile
               180                 185                 190
   gga ggt gcc gtc tcg ggc ggc tac att aat cgc aac acg cct acc act      861
   Gly Gly Ala Val Ser Gly Gly Tyr Ile Asn Arg Asn Thr Pro Thr Thr
           195                 200                 205
   gat cct ctt tac cag aat gga ggc gct aac ccc acc tac gaa tac ttt      909
   Asp Pro Leu Tyr Gln Asn Gly Gly Ala Asn Pro Thr Tyr Glu Tyr Phe
       210                 215                 220
   ccc tcc aag acc acc gga aac cta ccc atc tgt aac ttt atg gct cag      957
   Pro Ser Lys Thr Thr Gly Asn Leu Pro Ile Cys Asn Phe Met Ala Gln
   225                 230                 235                 240
   act aac ggt ctc aac atg tac ccg cac acc tac ctc atg ccc tct ggc     1005
   Thr Asn Gly Leu Asn Met Tyr Pro His Thr Tyr Leu Met Pro Ser Gly
                   245                 250                 255
   aag atc ttc atg cag gcc aac gtc agt acc atc ctc tgg gac cac gtc      1053
   Lys Ile Phe Met Gln Ala Asn Val Ser Thr Ile Leu Trp Asp His Val
               260                 265                 270
   aac aac act cag atc gac ctt ccc gac atg cct ggc gga gtc gtg cgc      1101
   Asn Asn Thr Gln Ile Asp Leu Pro Asp Met Pro Gly Gly Val Val Arg
           275                 280                 285
   gtc tac ccc gcc tcg gct gcc act gcc atg ctg cca ctc act cct cag      1149
   Val Tyr Pro Ala Ser Ala Ala Thr Ala Met Leu Pro Leu Thr Pro Gln
       290                 295                 300
   aat cag tac aca cct acc atc ctg ttt tgc ggt ggt agt gtc atg agc      1197
   Asn Gln Tyr Thr Pro Thr Ile Leu Phe Cys Gly Gly Ser Val Met Ser
   305                 310                 315                 320
   gac cag atg tgg ggc aac tac agt ggt ccc ggt ggc aac att ctc ggt      1245
   Asp Gln Met Trp Gly Asn Tyr Ser Gly Pro Gly Gly Asn Ile Leu Gly
                   325                 330                 335
   ctc caa gcc tct gat gac tgc tcg tcc atc aac ccc gag gac aat cag      1293
   Leu Gln Ala Ser Asp Asp Cys Ser Ser Ile Asn Pro Glu Asp Asn Gln
               340                 345                 350
   ggc aac cag atc act gac gct cag tac gtc cag gag ggg cgg ctt ccc      1341
   Gly Asn Gln Ile Thr Asp Ala Gln Tyr Val Gln Glu Gly Arg Leu Pro
           355                 360                 365
   gaa ggt cgt tcc atg gga cag ttc atc cac ctc cct gac ggt acc atg      1389
   Glu Gly Arg Ser Met Gly Gln Phe Ile His Leu Pro Asp Gly Thr Met
       370                 375                 380
   gtc gtc ctc aac ggc gcc aac aag gga act gcc ggc tat tcg aac cag      1437
   Val Val Leu Asn Gly Ala Asn Lys Gly Thr Ala Gly Tyr Ser Asn Gln
   385                 390                 395                 400
   aca tgg aac acc atc cag tac aac ggt cgc acc gtc gtc acc gaa ggt      1485
   Thr Trp Asn Thr Ile Gln Tyr Asn Gly Arg Thr Val Val Thr Glu Gly
                   405                 410                 415
   ctt tcg cag gat ccc act tac gtt ccc gtc atc tat gac ccg tcc aag      1533
   Leu Ser Gln Asp Pro Thr Tyr Val Pro Val Ile Tyr Asp Pro Ser Lys
               420                 425                 430
   ccc aga ggt cag cgt ctc tcc aat gct aat ctc aag cct tcc acc att      1581
   Pro Arg Gly Gln Arg Leu Ser Asn Ala Asn Leu Lys Pro Ser Thr Ile
           435                 440                 445
   gct cgt ctc tac cac tcg agc gct att ttg ctc ccc gat ggt tcc gtc      1629
   Ala Arg Leu Tyr His Ser Ser Ala Ile Leu Leu Pro Asp Gly Ser Val
       450                 455                 460
   atg gtt gca ggt tcc aac ccg cat cag gat gtt gcg ctc gac atg ccc      1677
   Met Val Ala Gly Ser Asn Pro His Gln Asp Val Ala Leu Asp Met Pro
   465                 470                 475                 480
   acc ggc acc acg cct cag gct ttc aac acc acc tac gag gtt gaa aag      1725
   Thr Gly Thr Thr Pro Gln Ala Phe Asn Thr Thr Tyr Glu Val Glu Lys
                   485                 490                 495
   tgg tac cct cct tac tgg gac tcg cca cgc cct tac cca cag ggc gtg      1773
   Trp Tyr Pro Pro Tyr Trp Asp Ser Pro Arg Pro Tyr Pro Gln Gly Val
                   500                     505                     510
   ccc aat tcg gtg ctg tac ggc ggc agt cct ttc aac att acc gtc aac       1821
   Pro Asn Ser Val Leu Tyr Gly Gly Ser Pro Phe Asn Ile Thr Val Asn
           515                 520                     525
   ggt acc ttt atg ggt gac tcg gcc aac gcc aag gca gcc aac acc aag       1869
   Gly Thr Phe Met Gly Asp Ser Ala Asn Ala Lys Ala Ala Asn Thr Lys
       530                 535                     540
   ttt gcc atc att cgt acc ggt ttc tcc acc cac gcc atg aac atg ggg       1917
   Phe Ala Ile Ile Arg Thr Gly Phe Ser Thr His Ala Met Asn Met Gly
   545                 550                     555                     560
   cag cgc gcc gtc tac ctc gac tac acc tac acc gtt aac gat gac gcc       1965
   Gln Arg Ala Val Tyr Leu Asp Tyr Thr Tyr Thr Val Asn Asp Asp Ala
                   565                     570                     575
   tcg gtc acc tac atg gtc aac cct ttg ccc aac act aag gct atg aac       2013
   Ser Val Thr Tyr Met Val Asn Pro Leu Pro Asn Thr Lys Ala Met Asn
               580                     585                     590
   cgc ctc ttt gtg cct ggc ccg gcc ttc ttc tac gtc acc gtc ggt ggc       2061
   Arg Leu Phe Val Pro Gly Pro Ala Phe Phe Tyr Val Thr Val Gly Gly
           595                     600                     605
   gtg cca agc cat ggc aag ctg atc atg gtg gga act tcc ccc act ggc       2109
   Val Pro Ser His Gly Lys Leu Ile Met Val Gly Thr Ser Pro Thr Gly
       610                     615                     620
   act ggc aac gtc ccc ttc act cct cag ctc ggg tct gca ctc gtc gcg       2157
   Thr Gly Asn Val Pro Phe Thr Pro Gln Leu Gly Ser Ala Leu Val Ala
   625                     630                     635                     640
   ctt ccc cct gct gtc aac agc acc aaa ttc aca gcc tcc ctc ccc aag       2205
   Leu Pro Pro Ala Val Asn Ser Thr Lys Phe Thr Ala Ser Leu Pro Lys
                   645                     650                     655
   gct ggc agc agc tct tcc tcc gag ttt ggc ctc ggc aag atc att ggt       2253
   Ala Gly Ser Ser Ser Ser Ser Glu Phe Gly Leu Gly Lys Ile Ile Gly
               660                     665                     670
   atc gct gtt gct ggc gcc gca gtt ttg gcc ctc att gct ctc ggc tgt       2301
   Ile Ala Val Ala Gly Ala Ala Val Leu Ala Leu Ile Ala Leu Gly Cys
           675                     680                     685
   tgt ctg tgg agg cgc aag ggc agg agc cat agc gac aag gct gcc tcg       2349
   Cys Leu Trp Arg Arg Lys Gly Arg Ser His Ser Asp Lys Ala Ala Ser
       690                     695                     700
   cgc cag tcg gct gcc cct tgg acc agc cgc gac ctt ggc tcg ggt ccc       2397
   Arg Gln Ser Ala Ala Pro Trp Thr Ser Arg Asp Leu Gly Ser Gly Pro
   705                     710                     715                     720
   gag tac aag cgt gtc gac act cct gtc gga tcc atc agc ggt ggt cgc       2445
   Glu Tyr Lys Arg Val Asp Thr Pro Val Gly Ser Ile Ser Gly Gly Arg
                   725                     730                     735
   ttt ggg gcc gcc agg atg gac agc tcg aat acg ttt gag agc tat cgg       2493
   Phe Gly Ala Ala Arg Met Asp Ser Ser Asn Thr Phe Glu Ser Tyr Arg
               740                     745                     750
   ttg cac gac cag gtc agc acg agc gaa agc aag gag gcg att ggc agc       2541
   Leu His Asp Gln Val Ser Thr Ser Glu Ser Lys Glu Ala Ile Gly Ser
           755                 760                 765
   tac tac gac caa cct cgc agc ggc agc cgt ggc ggc tac gct cct agc      2589
   Tyr Tyr Asp Gln Pro Arg Ser Gly Ser Arg Gly Gly Tyr Ala Pro Ser
       770                 775                 780
   ccg ctc gcc tac gac caa cac gga cgt ggc gcc tcg caa ggc cag tac      2637
   Pro Leu Ala Tyr Asp Gln His Gly Arg Gly Ala Ser Gln Gly Gln Tyr
   785                 790                 795                 800
   cac cag caa ggc tgg ggc gaa tac cac gct ggc gat gct ggt gca tac      2685
   His Gln Gln Gly Trp Gly Glu Tyr His Ala Gly Asp Ala Gly Ala Tyr
                   805                 810                 815
   tac gag gac aac act agc agg tac ggc agc ggt ggc ggt gga cac agc      2733
   Tyr Glu Asp Asn Thr Ser Arg Tyr Gly Ser Gly Gly Gly Gly His Ser
               820                 825                 830
   tac gat gat tac tcg cac cag caa tac caa cag cag cat tac tat gac      2781
   Tyr Asp Asp Tyr Ser His Gln Gln Tyr Gln Gln Gln His Tyr Tyr Asp
           835                 840                 845
   agc cca ggt cat cag cac caa gga agc tac tct agt cga cgc              2823
   Ser Pro Gly His Gln His Gln Gly Ser Tyr Ser Ser Arg Arg
       850                 855                 860
   taagccccga aaaacgctgc tggtgctttg tcagtcagtg catgggggat cctctagagt   2883
   cgacctgcag gcatgcaagc ttggcactgg ccgtcgtttt                          2923
<210> 4
   <211> 862
   <212> PRT
   <213> Ustilago maydis
<400> 4
   Met Thr Arg His Leu Ser Ser Ser Ser Arg Arg Ser Ser Leu Ala Lys
   1               5                   10                  15
   Ser Ala Met Thr Leu Ala Thr Leu Ser Leu Ala Leu Thr Ser Cys Ala
               20                  25                  30
   Ser Ala Ala Ser Lys Ala Gly Ser Tyr Glu Val Val Asn Thr Asn Ser
           35                  40                  45
   Leu Ala Ser Ala Met Met Leu Gly Leu Met Asp Glu Asp Asn Val Phe
       50                  55                  60
   Ile Leu Asp Lys Ala Glu Asn Asn Ser Ala Arg Leu Ala Asp Gly Arg
   65                  70                  75                  80
   His Val Trp Gly Ser Phe Tyr Lys Leu Ser Asp Asn Ser Val Thr Gly
                   85                  90                  95
   Thr Ala Val Gln Thr Asn Thr Phe Cys Ala Ser Gly Ala Thr Leu Gly
               100                 105                 110
   Asn Gly Ser Trp Leu Val Ala Gly Gly Asn Gln Ala Val Gly Tyr Gly
           115                 120                 125
   Gly Ala Ala Gln Ala Gln Glu Ile Asn Pro Tyr Ser Asp Phe Asp Gly
       130                 135                 140
   Thr Arg Ala Ile Arg Leu Leu Glu Pro Asn Ser Gln Thr Trp Ile Asp
   145                 150                 155                 160
   Ser Pro Ser Thr Thr Val Ala Gln Val Asn Met Leu Gln Gln Pro Arg
                   165                 170                 175
   Trp Tyr Pro Gly Ile Glu Val Leu Glu Asp Gly Ser Val Ile Phe Ile
               180                 185                 190
   Gly Gly Ala Val Ser Gly Gly Tyr Ile Asn Arg Asn Thr Pro Thr Thr
           195                 200                 205
   Asp Pro Leu Tyr Gln Asn Gly Gly Ala Asn Pro Thr Tyr Glu Tyr Phe
       210                 215                 220
   Pro Ser Lys Thr Thr Gly Asn Leu Pro Ile Cys Asn Phe Met Ala Gln
   225                 230                 235                 240
   Thr Asn Gly Leu Asn Met Tyr Pro His Thr Tyr Leu Met Pro Ser Gly
                   245                 250                 255
   Lys Ile Phe Met Gln Ala Asn Val Ser Thr Ile Leu Trp Asp His Val
               260                 265                 270
   Asn Asn Thr Gln Ile Asp Leu Pro Asp Met Pro Gly Gly Val Val Arg
           275                 280                 285
   Val Tyr Pro Ala Ser Ala Ala Thr Ala Met Leu Pro Leu Thr Pro Gln
       290                 295                 300
   Asn Gln Tyr Thr Pro Thr Ile Leu Phe Cys Gly Gly Ser Val Met Ser
   305                 310                 315                 320
   Asp Gln Met Trp Gly Asn Tyr Ser Gly Pro Gly Gly Asn Ile Leu Gly
                   325                 330                 335
   Leu Gln Ala Ser Asp Asp Cys Ser Ser Ile Asn Pro Glu Asp Asn Gln
               340                 345                 350
   Gly Asn Gln Ile Thr Asp Ala Gln Tyr Val Gln Glu Gly Arg Leu Pro
           355                 360                 365
   Glu Gly Arg Ser Met Gly Gln Phe Ile His Leu Pro Asp Gly Thr Met
       370                 375                 380
   Val Val Leu Asn Gly Ala Asn Lys Gly Thr Ala Gly Tyr Ser Asn Gln
   385                 390                 395                 400
   Thr Trp Asn Thr Ile Gln Tyr Asn Gly Arg Thr Val Val Thr Glu Gly
                   405                 410                 415
   Leu Ser Gln Asp Pro Thr Tyr Val Pro Val Ile Tyr Asp Pro Ser Lys
               420                 425                 430
   Pro Arg Gly Gln Arg Leu Ser Asn Ala Asn Leu Lys Pro Ser Thr Ile
           435                 440                 445
   Ala Arg Leu Tyr His Ser Ser Ala Ile Leu Leu Pro Asp Gly Ser Val
       450                 455                 460
   Met Val Ala Gly Ser Asn Pro His Gln Asp Val Ala Leu Asp Met Pro
   465                 470                 475                 480
   Thr Gly Thr Thr Pro Gln Ala Phe Asn Thr Thr Tyr Glu Val Glu Lys
                   485                 490                 495
   Trp Tyr Pro Pro Tyr Trp Asp Ser Pro Arg Pro Tyr Pro Gln Gly Val
               500                 505                 510
   Pro Asn Ser Val Leu Tyr Gly Gly Ser Pro Phe Asn Ile Thr Val Asn
           515                 520                 525
   Gly Thr Phe Met Gly Asp Ser Ala Asn Ala Lys Ala Ala Asn Thr Lys
       530                 535                 540
   Phe Ala Ile Ile Arg Thr Gly Phe Ser Thr His Ala Met Asn Met Gly
   545                 550                 555                 560
   Gln Arg Ala Val Tyr Leu Asp Tyr Thr Tyr Thr Val Asn Asp Asp Ala
                   565                 570                 575
   Ser Val Thr Tyr Met Val Asn Pro Leu Pro Asn Thr Lys Ala Met Asn
   580 585 590
   Arg Leu Phe Val Pro Gly Pro Ala Phe Phe Tyr Val Thr Val Gly Gly
   595 600 605
   Val Pro Ser His Gly Lys Leu Ile Met Val Gly Thr Ser Pro Thr Gly
   610 615 620
   Thr Gly Asn Val Pro Phe Thr Pro Gln Leu Gly Ser Ala Leu Val Ala
   625 630 635 640
   Leu Pro Pro Ala Val Asn Ser Thr Lys Phe Thr Ala Ser Leu Pro Lys
   645 650 655
   Ala Gly Ser Ser Ser Ser Ser Glu Phe Gly Leu Gly Lys Ile Ile Gly
   660 665 670
   Ile Ala Val Ala Gly Ala Ala Val Leu Ala Leu Ile Ala Leu Gly Cys
   675 680 685
   Cys Leu Trp Arg Arg Lys Gly Arg Ser His Ser Asp Lys Ala Ala Ser
   690 695 700
   Arg Gln Ser Ala Ala Pro Trp Thr Ser Arg Asp Leu Gly Ser Gly Pro
   705 710 715 720
   Glu Tyr Lys Arg Val Asp Thr Pro Val Gly Ser Ile Ser Gly Gly Arg
   725 730 735
   Phe Gly Ala Ala Arg Met Asp Ser Ser Asn Thr Phe Glu Ser Tyr Arg
   740 745 750
   Leu His Asp Gln Val Ser Thr Ser Glu Ser Lys Glu Ala Ile Gly Ser
   755 760 765
   Tyr Tyr Asp Gln Pro Arg Ser Gly Ser Arg Gly Gly Tyr Ala Pro Ser
   770 775 780
   Pro Leu Ala Tyr Asp Gln His Gly Arg Gly Ala Ser Gln Gly Gln Tyr
   785 790 795 800
   His Gln Gln Gly Trp Gly Glu Tyr His Ala Gly Asp Ala Gly Ala Tyr
   805 810 815
   Tyr Glu Asp Asn Thr Ser Arg Tyr Gly Ser Gly Gly Gly Gly His Ser
   820 825 830
   Tyr Asp Asp Tyr Ser His Gln Gln Tyr Gln Gln Gln His Tyr Tyr Asp
           835                 840                 845
   Ser Pro Gly His Gln His Gln Gly Ser Tyr Ser Ser Arg Arg
       850                 855                 860
<210> 5
   <211> 1614
   <212> DNA
   <213> Ustilago maydis
<220>
   <221> CDS
   <222> (1)..(1614)
<400> 5
   atg gag gtg cgt tcc aac acg ttc tgt gcc ggc ggt atg acg ctg ggc       48
   Met Glu Val Arg Ser Asn Thr Phe Cys Ala Gly Gly Met Thr Leu Gly
   1               5                   10                  15
   gac ggc agt tgg ctc gtc acg ggc gga aac aag gcg gtt acc acg aat       96
   Asp Gly Ser Trp Leu Val Thr Gly Gly Asn Lys Ala Val Thr Thr Asn
               20                  25                  30
   ggc gcg act gct aag gca ggt gct gga tac ggc gct tac aat ggc ggt      144
   Gly Ala Thr Ala Lys Ala Gly Ala Gly Tyr Gly Ala Tyr Asn Gly Gly
           35                  40                  45
   aag gca ctg cga ttc ctt agc cct tgc gac aac atg caa tgt cag tgg      192
   Lys Ala Leu Arg Phe Leu Ser Pro Cys Asp Asn Met Gln Cys Gln Trp
       50                  55                  60
   aac gac caa aac agc aat cag ctc aac atg gag agg tgg tat cct acc      240
   Asn Asp Gln Asn Ser Asn Gln Leu Asn Met Glu Arg Trp Tyr Pro Thr
   65                  70                  75                  80
   gta gag cct cta gcc gat gga tcc aat atc atc ctt gga ggc atg cgc      288
   Val Glu Pro Leu Ala Asp Gly Ser Asn Ile Ile Leu Gly Gly Met Arg
                   85                  90                  95
   gac ggt ggc ttt gtt cca agc cag ggc tct aat gtt cct act tac gag      336
   Asp Gly Gly Phe Val Pro Ser Gln Gly Ser Asn Val Pro Thr Tyr Glu
               100                 105                 110
   ttc tac cct cct aag agt ggc gga gct agt att aat ttg cca atc ctg      384
   Phe Tyr Pro Pro Lys Ser Gly Gly Ala Ser Ile Asn Leu Pro Ile Leu
           115                 120                 125
   caa cgt act gta ccc ctc tca ctc tac ccg atc gcg tat ctc atg tcg      432
   Gln Arg Thr Val Pro Leu Ser Leu Tyr Pro Ile Ala Tyr Leu Met Ser
       130                 135                 140
   tcc ggt gag gtg ttt atc caa gcc gga agg gag gcg atc ctt tgg aat      480
   Ser Gly Glu Val Phe Ile Gln Ala Gly Arg Glu Ala Ile Leu Trp Asn
   145                 150                 155                 160
   tac gac cag cag agc gag cgc gca ttt gcc aag att cca ggt gct cct        528
   Tyr Asp Gln Gln Ser Glu Arg Ala Phe Ala Lys Ile Pro Gly Ala Pro
                   165                 170                 175
   cgt gtc tat cct gcc tct ggt ggc tcg gct atg ctt cct cta act ccg        576
   Arg Val Tyr Pro Ala Ser Gly Gly Ser Ala Met Leu Pro Leu Thr Pro
               180                 185                 190
   gca gac gat tac aag gag acc atc ctc ttc tgc ggt ggt acg agc ttg        624
   Ala Asp Asp Tyr Lys Glu Thr Ile Leu Phe Cys Gly Gly Thr Ser Leu
           195                 200                 205
   ggc aag gtc tcg aac tgg ggt aac gag ggt gga ccc tcg atc ccc ata        672
   Gly Lys Val Ser Asn Trp Gly Asn Glu Gly Gly Pro Ser Ile Pro Ile
       210                 215                 220
   tct cag gtt ccc gca tcg acg tcg tgc gag cag atc agc cca ttc cag        720
   Ser Gln Val Pro Ala Ser Thr Ser Cys Glu Gln Ile Ser Pro Phe Gln
   225                 230                 235                 240
   ggt gga aac tgg gaa tcg gtc gac gat ttg ccc gag cgt cgt tcc atg        768
   Gly Gly Asn Trp Glu Ser Val Asp Asp Leu Pro Glu Arg Arg Ser Met
                   245                 250                 255
   ggt caa ttt atc aac ctg ccc gac ggc acc ctg tgg ttc ggc aac ggt        816
   Gly Gln Phe Ile Asn Leu Pro Asp Gly Thr Leu Trp Phe Gly Asn Gly
               260                 265                 270
   gtc acc act ggc gtt gct ggt tac agc acc gac ccc aac tct gtc ggc        864
   Val Thr Thr Gly Val Ala Gly Tyr Ser Thr Asp Pro Asn Ser Val Gly
           275                 280                 285
   aaa ccg gtg ggc gag tcg tat ggc gac aac ccg tcg tac cag cct ctc        912
   Lys Pro Val Gly Glu Ser Tyr Gly Asp Asn Pro Ser Tyr Gln Pro Leu
       290                 295                 300
   gta tac gac ccc aag gca agc cga ggc aac cga tgg aag cgc gtc gga        960
   Val Tyr Asp Pro Lys Ala Ser Arg Gly Asn Arg Trp Lys Arg Val Gly
   305                 310                 315                 320
   agc acc aac att ggt cga ctc tat cat tcg tct gct acg ctg ctt ccg       1008
   Ser Thr Asn Ile Gly Arg Leu Tyr His Ser Ser Ala Thr Leu Leu Pro
                   325                 330                 335
   gat tcg tct atc ctc gtt gct ggt tcc aac cct aat gct gac gtc aac       1056
   Asp Ser Ser Ile Leu Val Ala Gly Ser Asn Pro Asn Ala Asp Val Asn
               340                 345                 350
   cac cat gtc aag tgg aag acg gaa tac cgc att gaa cga tgg tac cca       1104
   His His Val Lys Trp Lys Thr Glu Tyr Arg Ile Glu Arg Trp Tyr Pro
           355                 360                 365
   gac ttc tac gat cag cct cgg ccc tcg aac gac ggt ctc cct agc tct       1152
   Asp Phe Tyr Asp Gln Pro Arg Pro Ser Asn Asp Gly Leu Pro Ser Ser
       370                 375                 380
   ttc tcg tac ggc ggt caa ggc ttt acc atc agg ctc agt tct gca gca       1200
   Phe Ser Tyr Gly Gly Gln Gly Phe Thr Ile Arg Leu Ser Ser Ala Ala
   385                 390                 395                 400
   cag gcg cag aag gcc aag gtg gtc ctg att cga act gga ttt tcc acg       1248
   Gln Ala Gln Lys Ala Lys Val Val Leu Ile Arg Thr Gly Phe Ser Thr
                   405                     410                     415
   cat ggc atg aat atg ggt caa cgc atg atc gag ctc aag tcg aca cat      1296
   His Gly Met Asn Met Gly Gln Arg Met Ile Glu Leu Lys Ser Thr His
               420                     425                     430
   cgg ggc agc aag ctc tac gta gcg cag ctt cca ccc aat ccg aac ctg      1344
   Arg Gly Ser Lys Leu Tyr Val Ala Gln Leu Pro Pro Asn Pro Asn Leu
           435                     440                     445
   ttt gct ccc ggt cct gcg ctc gcg ttc gtt gta gtc gat ggc gtt ccg      1392
   Phe Ala Pro Gly Pro Ala Leu Ala Phe Val Val Val Asp Gly Val Pro
       450                     455                     460
   agt caa gga aag atg gtc atg gtg ggc aac gga aag atc ggc gag cag      1440
   Ser Gln Gly Lys Met Val Met Val Gly Asn Gly Lys Ile Gly Glu Gln
   465                     470                     475                     480
   cct gtc gat gca gag agc gtg ctg ccc ggc tcg acc gcc ccg atg aac      1488
   Pro Val Asp Ala Glu Ser Val Leu Pro Gly Ser Thr Ala Pro Met Asn
                   485                     490                     495
   gac atg ttt caa aga cga cag aat gcg tcc cag acc gaa cgc gat gtg      1536
   Asp Met Phe Gln Arg Arg Gln Asn Ala Ser Gln Thr Glu Arg Asp Val
               500                     505                     510
   gct tcc agt cac aac caa gtg ctc cac cga agc ggc ttg cat gcc cgt      1584
   Ala Ser Ser His Asn Gln Val Leu His Arg Ser Gly Leu His Ala Arg
           515                     520                     525
   cat caa aag ggt ggc gtc gat cgt tat tga                              1614
   His Gln Lys Gly Gly Val Asp Arg Tyr
       530                     535
<210> 6
   <211> 537
   <212> PRT
   <213> Ustilago maydis
<400> 6
   Met Glu Val Arg Ser Asn Thr Phe Cys Ala Gly Gly Met Thr Leu Gly
   1               5                   10                  15
   Asp Gly Ser Trp Leu Val Thr Gly Gly Asn Lys Ala Val Thr Thr Asn
               20                  25                  30
   Gly Ala Thr Ala Lys Ala Gly Ala Gly Tyr Gly Ala Tyr Asn Gly Gly
           35                  40                  45
   Lys Ala Leu Arg Phe Leu Ser Pro Cys Asp Asn Met Gln Cys Gln Trp
       50                  55                  60
   Asn Asp Gln Asn Ser Asn Gln Leu Asn Met Glu Arg Trp Tyr Pro Thr
   65                  70                  75                  80
   Val Glu Pro Leu Ala Asp Gly Ser Asn Ile Ile Leu Gly Gly Met Arg
                   85                  90                  95
   Asp Gly Gly Phe Val Pro Ser Gln Gly Ser Asn Val Pro Thr Tyr Glu
               100                 105                 110
   Phe Tyr Pro Pro Lys Ser Gly Gly Ala Ser Ile Asn Leu Pro Ile Leu
           115                 120                 125
   Gln Arg Thr Val Pro Leu Ser Leu Tyr Pro Ile Ala Tyr Leu Met Ser
       130                 135                 140
   Ser Gly Glu Val Phe Ile Gln Ala Gly Arg Glu Ala Ile Leu Trp Asn
   145                 150                 155                 160
   Tyr Asp Gln Gln Ser Glu Arg Ala Phe Ala Lys Ile Pro Gly Ala Pro
                   165                 170                 175
   Arg Val Tyr Pro Ala Ser Gly Gly Ser Ala Met Leu Pro Leu Thr Pro
               180                 185                 190
   Ala Asp Asp Tyr Lys Glu Thr Ile Leu Phe Cys Gly Gly Thr Ser Leu
           195                 200                 205
   Gly Lys Val Ser Asn Trp Gly Asn Glu Gly Gly Pro Ser Ile Pro Ile
       210                 215                 220
   Ser Gln Val Pro Ala Ser Thr Ser Cys Glu Gln Ile Ser Pro Phe Gln
   225                 230                 235                 240
   Gly Gly Asn Trp Glu Ser Val Asp Asp Leu Pro Glu Arg Arg Ser Met
                   245                 250                 255
   Gly Gln Phe Ile Asn Leu Pro Asp Gly Thr Leu Trp Phe Gly Asn Gly
               260                 265                 270
   Val Thr Thr Gly Val Ala Gly Tyr Ser Thr Asp Pro Asn Ser Val Gly
           275                 280                 285
   Lys Pro Val Gly Glu Ser Tyr Gly Asp Asn Pro Ser Tyr Gln Pro Leu
       290                 295                 300
   Val Tyr Asp Pro Lys Ala Ser Arg Gly Asn Arg Trp Lys Arg Val Gly
   305                 310                 315                 320
   Ser Thr Asn Ile Gly Arg Leu Tyr His Ser Ser Ala Thr Leu Leu Pro
                   325                 330                 335
   Asp Ser Ser Ile Leu Val Ala Gly Ser Asn Pro Asn Ala Asp Val Asn
               340                 345                 350
   His His Val Lys Trp Lys Thr Glu Tyr Arg Ile Glu Arg Trp Tyr Pro
           355                 360                 365
   Asp Phe Tyr Asp Gln Pro Arg Pro Ser Asn Asp Gly Leu Pro Ser Ser
       370                 375                 380
   Phe Ser Tyr Gly Gly Gln Gly Phe Thr Ile Arg Leu Ser Ser Ala Ala
   385                 390                 395                 400
   Gln Ala Gln Lys Ala Lys Val Val Leu Ile Arg Thr Gly Phe Ser Thr
                   405                 410                 415
   His Gly Met Asn Met Gly Gln Arg Met Ile Glu Leu Lys Ser Thr His
               420                 425                 430
   Arg Gly Ser Lys Leu Tyr Val Ala Gln Leu Pro Pro Asn Pro Asn Leu
           435                 440                 445
   Phe Ala Pro Gly Pro Ala Leu Ala Phe Val Val Val Asp Gly Val Pro
       450                 455                 460
   Ser Gln Gly Lys Met Val Met Val Gly Asn Gly Lys Ile Gly Glu Gln
   465                 470                 475                 480
   Pro Val Asp Ala Glu Ser Val Leu Pro Gly Ser Thr Ala Pro Met Asn
                   485                 490                 495
   Asp Met Phe Gln Arg Arg Gln Asn Ala Ser Gln Thr Glu Arg Asp Val
               500                 505                 510
   Ala Ser Ser His Asn Gln Val Leu His Arg Ser Gly Leu His Ala Arg
           515                 520                 525
   His Gln Lys Gly Gly Val Asp Arg Tyr
       530                 535
<210> 7
   <211> 1902
   <212> DNA
   <213> Ustilago maydis
<220>
   <221> CDS
   <222> (1)..(1902)
<400> 7
   atg gct gca tcg tcc atg gcg gct aca cca gga gga agc gag atc gtc        48
   Met Ala Ala Ser Ser Met Ala Ala Thr Pro Gly Gly Ser Glu Ile Val
   1               5                   10                  15
   ggc tcg tcc gcc gtc tca ggc atg atg ctc ttc aac agc gcc cca ggc        96
   Gly Ser Ser Ala Val Ser Gly Met Met Leu Phe Asn Ser Ala Pro Gly
               20                  25                  30
   aaa gtc atc atc ctc gac aag acc gaa ggc aat gca gcc cgc atc aac       144
   Lys Val Ile Ile Leu Asp Lys Thr Glu Gly Asn Ala Ala Arg Ile Asn
           35                  40                  45
   ggc cat cct gct tgg gga gag gag tgg gac acc gag gct cgc acc agt       192
   Gly His Pro Ala Trp Gly Glu Glu Trp Asp Thr Glu Ala Arg Thr Ser
       50                  55                  60
   cgt ctg atg aac gtc gtc acc aac acg ttt tgt gca ggc ggt atg tcg       240
   Arg Leu Met Asn Val Val Thr Asn Thr Phe Cys Ala Gly Gly Met Ser
   65                  70                  75                  80
   ctc ggc aac ggc acc tgg gct gtc ttt gga ggc aat gag aac gtc ggg       288
   Leu Gly Asn Gly Thr Trp Ala Val Phe Gly Gly Asn Glu Asn Val Gly
                   85                  90                  95
   ccc gga ggc aac tcg acc acc cca cgt ttc agc acc aca gcg cct tac       336
   Pro Gly Gly Asn Ser Thr Thr Pro Arg Phe Ser Thr Thr Ala Pro Tyr
               100                 105                 110
   tat gat ggc gat gga ggc gct gct gct cgt ttc tac act ccc aat tct       384
   Tyr Asp Gly Asp Gly Gly Ala Ala Ala Arg Phe Tyr Thr Pro Asn Ser
           115                 120                 125
   cag ggc acc tcc gat tgg gat gat ggt aac cac tac atg cag agg cgc       432
   Gln Gly Thr Ser Asp Trp Asp Asp Gly Asn His Tyr Met Gln Arg Arg
       130                 135                 140
   aga tgg tat cca act gtc gaa gct ctc ggt gat ggc acg ctc tgg ata       480
   Arg Trp Tyr Pro Thr Val Glu Ala Leu Gly Asp Gly Thr Leu Trp Ile
   145                 150                 155                 160
   gga ggc ggt gaa gac tat gga ggt tac gtt gca gac gaa gga cag aac       528
   Gly Gly Gly Glu Asp Tyr Gly Gly Tyr Val Ala Asp Glu Gly Gln Asn
                   165                 170                 175
   caa ccc aac ttt gag tac tgg ccg cca aga ggc gcc gcc atc aac atg       576
   Gln Pro Asn Phe Glu Tyr Trp Pro Pro Arg Gly Ala Ala Ile Asn Met
               180                 185                 190
   gac ttt ctt acc cag act ttg cca atg aac ctg tat cct ttg gcg tgg       624
   Asp Phe Leu Thr Gln Thr Leu Pro Met Asn Leu Tyr Pro Leu Ala Trp
           195                 200                 205
   ctc atg gca tcc ggt cgc ttg ttt gtc cag gca ggg cag gat gcg atc       672
   Leu Met Ala Ser Gly Arg Leu Phe Val Gln Ala Gly Gln Asp Ala Ile
       210                 215                 220
   ctg tac gac ttg gag agc aat tcg gtt gcc aaa ggt ctt ccg tcc acc      720
   Leu Tyr Asp Leu Glu Ser Asn Ser Val Ala Lys Gly Leu Pro Ser Thr
   225                 230                 235                 240
   acg gga ccc atg aaa gtt tac ccg gct tca gcg ggc gta gct atg ttg      768
   Thr Gly Pro Met Lys Val Tyr Pro Ala Ser Ala Gly Val Ala Met Leu
                   245                 250                 255
   cca ctg aca ccc gcg aac aac tat tcg caa gag gtg ctc ttc tgt ggc      816
   Pro Leu Thr Pro Ala Asn Asn Tyr Ser Gln Glu Val Leu Phe Cys Gly
               260                 265                 270
   ggc gtg cag cga ccg ctt aac gaa tgg ggt aac ggt gcg ggt cct ctg      864
   Gly Val Gln Arg Pro Leu Asn Glu Trp Gly Asn Gly Ala Gly Pro Leu
           275                 280                 285
   tac aac cca ctt ccg ttt gcg gca agc aag gtg tgc gag cgc atc acg      912
   Tyr Asn Pro Leu Pro Phe Ala Ala Ser Lys Val Cys Glu Arg Ile Thr
       290                 295                 300
   ccc gag gcc gac aat ccg acg tgg gag cag gac gac gat ctg atc aat      960
   Pro Glu Ala Asp Asn Pro Thr Trp Glu Gln Asp Asp Asp Leu Ile Asn
   305                 310                 315                 320
   ggt cga tct atg ggc act ttt gtc tat ctg ccc gac gga aag ctg tgg     1008
   Gly Arg Ser Met Gly Thr Phe Val Tyr Leu Pro Asp Gly Lys Leu Trp
                   325                 330                 335
   ttt gga caa ggg gtg cgt atg ggt acc ggg ggc tat tca ggt cag cct     1056
   Phe Gly Gln Gly Val Arg Met Gly Thr Gly Gly Tyr Ser Gly Gln Pro
               340                 345                 350
   tac aac aag aac att ggt att tcg ttg ggc gac caa ccg gac ttc cag     1104
   Tyr Asn Lys Asn Ile Gly Ile Ser Leu Gly Asp Gln Pro Asp Phe Gln
           355                 360                 365
   ccg atg ctc tac gat cct tca gcg gcg aag ggc tcg cgt ttt tcg aca     1152
   Pro Met Leu Tyr Asp Pro Ser Ala Ala Lys Gly Ser Arg Phe Ser Thr
       370                 375                 380
   act ggc cta gcg cag atg cag gtg caa agg atg tac cat tcg acc gcc     1200
   Thr Gly Leu Ala Gln Met Gln Val Gln Arg Met Tyr His Ser Thr Ala
   385                 390                 395                 400
   atc ttg ctc gag gac ggc tcc gtg ctc act tcc ggc tcc aac cct aac     1248
   Ile Leu Leu Glu Asp Gly Ser Val Leu Thr Ser Gly Ser Asn Pro Asn
                   405                 410                 415
   gcc gac gtt tcg ctt agt aac gca gcc aac tac acc aac acc gag tac     1296
   Ala Asp Val Ser Leu Ser Asn Ala Ala Asn Tyr Thr Asn Thr Glu Tyr
               420                 425                 430
   cgt ctg gag cag tgg tac ccg ttg tgg tac aac gag ccc agg cct acg     1344
   Arg Leu Glu Gln Trp Tyr Pro Leu Trp Tyr Asn Glu Pro Arg Pro Thr
           435                 440                 445
   cag ccc aac gtc act cag att gct tac ggt ggt ggt tcc ttt gac gtg     1392
   Gln Pro Asn Val Thr Gln Ile Ala Tyr Gly Gly Gly Ser Phe Asp Val
       450                 455                 460
   ccg ctc tct gaa tcg gac ctc tcg aac aac att acc aac atc aag aca      1440
   Pro Leu Ser Glu Ser Asp Leu Ser Asn Asn Ile Thr Asn Ile Lys Thr
   465                 470                 475                 480
   gcc aag atg gtt att att cgg tcc gga ttc gcg aca cac ggt gtc aac      1488
   Ala Lys Met Val Ile Ile Arg Ser Gly Phe Ala Thr His Gly Val Asn
                   485                 490                 495
   ttt gga cag cgc tac ctc gag ctc aat tcg acc tac act gcc ttt cag      1536
   Phe Gly Gln Arg Tyr Leu Glu Leu Asn Ser Thr Tyr Thr Ala Phe Gln
               500                 505                 510
   aat ggc agc gtt gga ggc acg ctg cac gtg tcc aac atg ccg cct aac      1584
   Asn Gly Ser Val Gly Gly Thr Leu His Val Ser Asn Met Pro Pro Asn
           515                 520                 525
   gct aac ctt ttc cag cct ggg ccg gcc atg gca ttt ttg gta atc aac      1632
   Ala Asn Leu Phe Gln Pro Gly Pro Ala Met Ala Phe Leu Val Ile Asn
       530                 535                 540
   ggt gtg cct tcc cac ggt cag cac gta atg atc ggc act ggc cag ctg      1680
   Gly Val Pro Ser His Gly Gln His Val Met Ile Gly Thr Gly Gln Leu
   545                 550                 555                 560
   ggc gac cag aat gtg atg gct tcg acg gtg ctt cct gcc tca cag gat      1728
   Gly Asp Gln Asn Val Met Ala Ser Thr Val Leu Pro Ala Ser Gln Asp
                   565                 570                 575
   cca cca gca ccg aga acg ggt agt agt gga tct ggc tcg aaa gga tcc      1776
   Pro Pro Ala Pro Arg Thr Gly Ser Ser Gly Ser Gly Ser Lys Gly Ser
               580                 585                 590
   aac ggc tcg aat gga tcc aac ggt act ctg aag gac tcg ccc aat ggt      1824
   Asn Gly Ser Asn Gly Ser Asn Gly Thr Leu Lys Asp Ser Pro Asn Gly
           595                 600                 605
   gcc gtt acc ctg tcg aca ggt ctc tgt gcc agt gta tcc ttt gct gca      1872
   Ala Val Thr Leu Ser Thr Gly Leu Cys Ala Ser Val Ser Phe Ala Ala
       610                 615                 620
   gtg ctg acg gcc ttc gcc ctg ttt gct tga                              1902
   Val Leu Thr Ala Phe Ala Leu Phe Ala
   625                 630
<210> 8
   <211> 633
   <212> PRT
   <213> Ustilago maydis
<400> 8
   Met Ala Ala Ser Ser Met Ala Ala Thr Pro Gly Gly Ser Glu Ile Val
   1               5                   10                  15
   Gly Ser Ser Ala Val Ser Gly Met Met Leu Phe Asn Ser Ala Pro Gly
   20 25 30
   Lys Val Ile Ile Leu Asp Lys Thr Glu Gly Asn Ala Ala Arg Ile Asn
   35 40 45
   Gly His Pro Ala Trp Gly Glu Glu Trp Asp Thr Glu Ala Arg Thr Ser
   50 55 60
   Arg Leu Met Asn Val Val Thr Asn Thr Phe Cys Ala Gly Gly Met Ser
   65 70 75 80
   Leu Gly Asn Gly Thr Trp Ala Val Phe Gly Gly Asn Glu Asn Val Gly
   85 90 95
   Pro Gly Gly Asn Ser Thr Thr Pro Arg Phe Ser Thr Thr Ala Pro Tyr
   100 105 110
   Tyr Asp Gly Asp Gly Gly Ala Ala Ala Arg Phe Tyr Thr Pro Asn Ser
   115 120 125
   Gln Gly Thr Ser Asp Trp Asp Asp Gly Asn His Tyr Met Gln Arg Arg
   130 135 140
   Arg Trp Tyr Pro Thr Val Glu Ala Leu Gly Asp Gly Thr Leu Trp Ile
   145 150 155 160
   Gly Gly Gly Glu Asp Tyr Gly Gly Tyr Val Ala Asp Glu Gly Gln Asn
   165 170 175
   Gln Pro Asn Phe Glu Tyr Trp Pro Pro Arg Gly Ala Ala Ile Asn Met
   180 185 190
   Asp Phe Leu Thr Gln Thr Leu Pro Met Asn Leu Tyr Pro Leu Ala Trp
   195 200 205
   Leu Met Ala Ser Gly Arg Leu Phe Val Gln Ala Gly Gln Asp Ala Ile
   210 215 220
   Leu Tyr Asp Leu Glu Ser Asn Ser Val Ala Lys Gly Leu Pro Ser Thr
   225 230 235 240
   Thr Gly Pro Met Lys Val Tyr Pro Ala Ser Ala Gly Val Ala Met Leu
   245 250 255
   Pro Leu Thr Pro Ala Asn Asn Tyr Ser Gln Glu Val Leu Phe Cys Gly
   260 265 270
   Gly Val Gln Arg Pro Leu Asn Glu Trp Gly Asn Gly Ala Gly Pro Leu
   275 280 285
   Tyr Asn Pro Leu Pro Phe Ala Ala Ser Lys Val Cys Glu Arg Ile Thr
   290 295 300
   Pro Glu Ala Asp Asn Pro Thr Trp Glu Gln Asp Asp Asp Leu Ile Asn
   305 310 315 320
   Gly Arg Ser Met Gly Thr Phe Val Tyr Leu Pro Asp Gly Lys Leu Trp
   325 330 335
   Phe Gly Gln Gly Val Arg Met Gly Thr Gly Gly Tyr Ser Gly Gln Pro
   340 345 350
   Tyr Asn Lys Asn Ile Gly Ile Ser Leu Gly Asp Gln Pro Asp Phe Gln
   355 360 365
   Pro Met Leu Tyr Asp Pro Ser Ala Ala Lys Gly Ser Arg Phe Ser Thr
   370 375 380
   Thr Gly Leu Ala Gln Met Gln Val Gln Arg Met Tyr His Ser Thr Ala
   385 390 395 400
   Ile Leu Leu Glu Asp Gly Ser Val Leu Thr Ser Gly Ser Asn Pro Asn
   405 410 415
   Ala Asp Val Ser Leu Ser Asn Ala Ala Asn Tyr Thr Asn Thr Glu Tyr
   420 425 430
   Arg Leu Glu Gln Trp Tyr Pro Leu Trp Tyr Asn Glu Pro Arg Pro Thr
   435 440 445
   Gln Pro Asn Val Thr Gln Ile Ala Tyr Gly Gly Gly Ser Phe Asp Val
   450 455 460
   Pro Leu Ser Glu Ser Asp Leu Ser Asn Asn Ile Thr Asn Ile Lys Thr
   465 470 475 480
   Ala Lys Met Val Ile Ile Arg Ser Gly Phe Ala Thr His Gly Val Asn
   485 490 495
   Phe Gly Gln Arg Tyr Leu Glu Leu Asn Ser Thr Tyr Thr Ala Phe Gln
   500 505 510
   Asn Gly Ser Val Gly Gly Thr Leu His Val Ser Asn Met Pro Pro Asn
   515 520 525
   Ala Asn Leu Phe Gln Pro Gly Pro Ala Met Ala Phe Leu Val Ile Asn
       530                 535                 540
   Gly Val Pro Ser His Gly Gln His Val Met Ile Gly Thr Gly Gln Leu
   545                 550                 555                 560
   Gly Asp Gln Asn Val Met Ala Ser Thr Val Leu Pro Ala Ser Gln Asp
                   565                 570                 575
   Pro Pro Ala Pro Arg Thr Gly Ser Ser Gly Ser Gly Ser Lys Gly Ser
               580                 585                 590
   Asn Gly Ser Asn Gly Ser Asn Gly Thr Leu Lys Asp Ser Pro Asn Gly
           595                 600                 605
   Ala Val Thr Leu Ser Thr Gly Leu Cys Ala Ser Val Ser Phe Ala Ala
       610                 615                 620
   Val Leu Thr Ala Phe Ala Leu Phe Ala
   625                 630
<210> 9
   <211> 1970
   <212> DNA
   <213> Botrytis cinerea
<220>
   <221> CDS
   <222> (1)..(1968)
<400> 9
   atg cta att ttt acc gtt ttt agt tat tgt gga tct aca act gat cac       48
   Met Leu Ile Phe Thr Val Phe Ser Tyr Cys Gly Ser Thr Thr Asp His
   1               5                   10                  15
   tgt ttg gct tcc aat ggt tgc cag aat gga tgc aca ggc tca caa tct       96
   Cys Leu Ala Ser Asn Gly Cys Gln Asn Gly Cys Thr Gly Ser Gln Ser
               20                  25                  30
   tca tca gcc gcc aag act act acc aca gct gca gca ggc agc gca ccc      144
   Ser Ser Ala Ala Lys Thr Thr Thr Thr Ala Ala Ala Gly Ser Ala Pro
           35                  40                  45
   tct tca tct aca act caa gaa cca gtg att gcc cca gtt agt tct aca      192
   Ser Ser Ser Thr Thr Gln Glu Pro Val Ile Ala Pro Val Ser Ser Thr
       50                  55                  60
   ctt acg cct gcc gca gct agc agt gca cca gta act act gat gga tca      240
   Leu Thr Pro Ala Ala Ala Ser Ser Ala Pro Val Thr Thr Asp Gly Ser
   65                  70                  75                  80
   tgt ggt act gcc aat gga ggt acc gtt tgt ggc aat tgg gta aat gga      288
   Cys Gly Thr Ala Asn Gly Gly Thr Val Cys Gly Asn Trp Val Asn Gly
                   85                  90                  95
   aat tgt tgt tcc atg tac ggt ttt tgt ggc agt acc aat gcg cat tgc      336
   Asn Cys Cys Ser Met Tyr Gly Phe Cys Gly Ser Thr Asn Ala His Cys
               100                 105                 110
   ggt gcc gga tgc caa tca gga gat tgt ttg aat gcg cct gcg gtt gca      384
   Gly Ala Gly Cys Gln Ser Gly Asp Cys Leu Asn Ala Pro Ala Val Ala
           115                 120                 125
   gct cct ggt gca agc cct gcc cca gct gcc cca gta gga ggt gcc ttt      432
   Ala Pro Gly Ala Ser Pro Ala Pro Ala Ala Pro Val Gly Gly Ala Phe
       130                 135                 140
   aat atc gtc ggg tcg tct gga gtt cct gct atg cat gct gca ctt atg      480
   Asn Ile Val Gly Ser Ser Gly Val Pro Ala Met His Ala Ala Leu Met
   145                 150                 155                 160
   cca aac ggt cga gtt atg ttc ctc gac aaa tta gag aac tac acc caa      528
   Pro Asn Gly Arg Val Met Phe Leu Asp Lys Leu Glu Asn Tyr Thr Gln
                   165                 170                 175
   ttg aaa ttg cca aat gga tac tac gcc atg tct tca gaa tac gac cca      576
   Leu Lys Leu Pro Asn Gly Tyr Tyr Ala Met Ser Ser Glu Tyr Asp Pro
               180                 185                 190
   gcc act aac gca gtc gcc act cct tta gct tac aaa aca aat gcg ttt      624
   Ala Thr Asn Ala Val Ala Thr Pro Leu Ala Tyr Lys Thr Asn Ala Phe
           195                 200                 205
   tgt tcc gga ggc act ttc ctt gct gat gga cgt gtt gtt tct ctt gga      672
   Cys Ser Gly Gly Thr Phe Leu Ala Asp Gly Arg Val Val Ser Leu Gly
       210                 215                 220
   ggc aac gcg cct tta gat tgg ctc gat cca aac att ggg gat gga ttt      720
   Gly Asn Ala Pro Leu Asp Trp Leu Asp Pro Asn Ile Gly Asp Gly Phe
   225                 230                 235                 240
   gac gcc att aga tat ctt gaa cga tca tct acc gat gct agc ctc aat      768
   Asp Ala Ile Arg Tyr Leu Glu Arg Ser Ser Thr Asp Ala Ser Leu Asn
                   245                 250                 255
   gga aaa gac tgg agt gaa cca ggt aac aag ctc gcg agt gct cgt tgg      816
   Gly Lys Asp Trp Ser Glu Pro Gly Asn Lys Leu Ala Ser Ala Arg Trp
               260                 265                 270
   tat gct act gct caa act atg ggt gat gga acc att ttg gtc gct ttt      864
   Tyr Ala Thr Ala Gln Thr Met Gly Asp Gly Thr Ile Leu Val Ala Phe
           275                 280                 285
   gga agt ttg aac ggc ctc gat ccg act gtc aaa acg aac aac aat cct      912
   Gly Ser Leu Asn Gly Leu Asp Pro Thr Val Lys Thr Asn Asn Asn Pro
       290                 295                 300
   aca tac gag att ttc agt gct acc gct gtg tcg caa ggt aag aac att      960
   Thr Tyr Glu Ile Phe Ser Ala Thr Ala Val Ser Gln Gly Lys Asn Ile
   305                 310                 315                 320
   gac atg gaa att ttg gag aaa aat cag cca tat tat atg tat cct ttt     1008
   Asp Met Glu Ile Leu Glu Lys Asn Gln Pro Tyr Tyr Met Tyr Pro Phe
                   325                 330                 335
   gtt cat ctc ctc aat ggt gga aat ttg ttc gtc ttc gtt tcc aag tct      1056
   Val His Leu Leu Asn Gly Gly Asn Leu Phe Val Phe Val Ser Lys Ser
               340                 345                 350
   tcc caa gta ctc aat gtc ggt acc aac act atc gtc aag gaa tta cct      1104
   Ser Gln Val Leu Asn Val Gly Thr Asn Thr Ile Val Lys Glu Leu Pro
           355                 360                 365
   gaa ctt gct gga gac tat cgc aca tat ccc aac act ggt gga agt gtt      1152
   Glu Leu Ala Gly Asp Tyr Arg Thr Tyr Pro Asn Thr Gly Gly Ser Val
       370                 375                 380
   tta ctc cct ttg tca agc gca aac aaa tgg aac cct gat atc atc atc      1200
   Leu Leu Pro Leu Ser Ser Ala Asn Lys Trp Asn Pro Asp Ile Ile Ile
   385                 390                 395                 400
   tgc ggg gga ggt gca tat caa gat att acc agt cca aca gag cca agt      1248
   Cys Gly Gly Gly Ala Tyr Gln Asp Ile Thr Ser Pro Thr Glu Pro Ser
                   405                 410                 415
   tgt gga aga atc cag cca ttg agt gca aac ccc aca tgg gag ttg gac      1296
   Cys Gly Arg Ile Gln Pro Leu Ser Ala Asn Pro Thr Trp Glu Leu Asp
               420                 425                 430
   gct atg cct gaa ggc cgt ggt atg gtt gaa gga acc tta ctt cca gat      1344
   Ala Met Pro Glu Gly Arg Gly Met Val Glu Gly Thr Leu Leu Pro Asp
           435                 440                 445
   gga aca gtt gtc tgg ctt aat gga ggg aac ttg ggt gct caa gga ttt      1392
   Gly Thr Val Val Trp Leu Asn Gly Gly Asn Leu Gly Ala Gln Gly Phe
       450                 455                 460
   gga ctt gca aaa gac cca aca ttg gaa gct ctt ctt tac gat cct acg      1440
   Gly Leu Ala Lys Asp Pro Thr Leu Glu Ala Leu Leu Tyr Asp Pro Thr
   465                 470                 475                 480
   aaa gct aag ggt caa aga ttc tca act ctt gca aca tca act atc cca      1488
   Lys Ala Lys Gly Gln Arg Phe Ser Thr Leu Ala Thr Ser Thr Ile Pro
                   485                 490                 495
   cgt ctc tac cat tct gtc tct ctc ctc ctt ctt gac ggt aca cta atg      1536
   Arg Leu Tyr His Ser Val Ser Leu Leu Leu Leu Asp Gly Thr Leu Met
               500                 505                 510
   gtc gct ggc tca aac cct gtc gag atg cca aag ctt caa cca gat gca      1584
   Val Ala Gly Ser Asn Pro Val Glu Met Pro Lys Leu Gln Pro Asp Ala
           515                 520                 525
   gcc gat cca tat gtt acg gag ttc cga gtt gag aac tat gtt cct ccc      1632
   Ala Asp Pro Tyr Val Thr Glu Phe Arg Val Glu Asn Tyr Val Pro Pro
       530                 535                 540
   tat ctc tca ggc gat aat gca aag aag cgt cct act aat gta aaa ttg      1680
   Tyr Leu Ser Gly Asp Asn Ala Lys Lys Arg Pro Thr Asn Val Lys Leu
   545                 550                 555                 560
   tca tca ggt agc ttc aaa gca gat ggt agc aca ctt gat gtc aca ttt      1728
   Ser Ser Gly Ser Phe Lys Ala Asp Gly Ser Thr Leu Asp Val Thr Phe
                   565                 570                 575
   gat tgt cca gct ggc gcg aaa gca gtt act gta act ttg tac cac ggt      1776
   Asp Cys Pro Ala Gly Ala Lys Ala Val Thr Val Thr Leu Tyr His Gly
               580                 585                 590
   gga ttc gtc act cac tct gta cat atg ggt cat cgc atg ctg cac ttg      1824
   Gly Phe Val Thr His Ser Val His Met Gly His Arg Met Leu His Leu
           595                 600                 605
   gat aac aca ggc ttc ggc gct ggt gcc aca cag cag aag ttg act gtt      1872
   Asp Asn Thr Gly Phe Gly Ala Gly Ala Thr Gln Gln Lys Leu Thr Val
       610                 615                 620
   act cga cca cca aac aac aat gtt gca cct cca ggt cca tat gtt gtt      1920
   Thr Arg Pro Pro Asn Asn Asn Val Ala Pro Pro Gly Pro Tyr Val Val
   625                 630                 635                 640
   tac att ctt gta gac ggc att cct gcc atg gga cag ttt gtt acg gtt      1968
   Tyr Ile Leu Val Asp Gly Ile Pro Ala Met Gly Gln Phe Val Thr Val
                   645                 650                 655
   tg                                                                   1970
<210> 10
   <211> 656
   <212> PRT
   <213> Botrytis cinerea
<400> 10
   Met Leu Ile Phe Thr Val Phe Ser Tyr Cys Gly Ser Thr Thr Asp His
   1               5                   10                  15
   Cys Leu Ala Ser Asn Gly Cys Gln Asn Gly Cys Thr Gly Ser Gln Ser
               20                  25                  30
   Ser Ser Ala Ala Lys Thr Thr Thr Thr Ala Ala Ala Gly Ser Ala Pro
           35                  40                  45
   Ser Ser Ser Thr Thr Gln Glu Pro Val Ile Ala Pro Val Ser Ser Thr
       50                  55                  60
   Leu Thr Pro Ala Ala Ala Ser Ser Ala Pro Val Thr Thr Asp Gly Ser
   65                  70                  75                  80
   Cys Gly Thr Ala Asn Gly Gly Thr Val Cys Gly Asn Trp Val Asn Gly
                   85                  90                  95
   Asn Cys Cys Ser Met Tyr Gly Phe Cys Gly Ser Thr Asn Ala His Cys
               100                 105                 110
   Gly Ala Gly Cys Gln Ser Gly Asp Cys Leu Asn Ala Pro Ala Val Ala
           115                 120                 125
   Ala Pro Gly Ala Ser Pro Ala Pro Ala Ala Pro Val Gly Gly Ala Phe
       130                 135                 140
   Asn Ile Val Gly Ser Ser Gly Val Pro Ala Met His Ala Ala Leu Met
   145                 150                 155                 160
   Pro Asn Gly Arg Val Met Phe Leu Asp Lys Leu Glu Asn Tyr Thr Gln
                   165                 170                 175
   Leu Lys Leu Pro Asn Gly Tyr Tyr Ala Met Ser Ser Glu Tyr Asp Pro
               180                 185                 190
   Ala Thr Asn Ala Val Ala Thr Pro Leu Ala Tyr Lys Thr Asn Ala Phe
           195                 200                 205
   Cys Ser Gly Gly Thr Phe Leu Ala Asp Gly Arg Val Val Ser Leu Gly
       210                 215                 220
   Gly Asn Ala Pro Leu Asp Trp Leu Asp Pro Asn Ile Gly Asp Gly Phe
   225                 230                 235                 240
   Asp Ala Ile Arg Tyr Leu Glu Arg Ser Ser Thr Asp Ala Ser Leu Asn
                   245                 250                 255
   Gly Lys Asp Trp Ser Glu Pro Gly Asn Lys Leu Ala Ser Ala Arg Trp
               260                 265                 270
   Tyr Ala Thr Ala Gln Thr Met Gly Asp Gly Thr Ile Leu Val Ala Phe
           275                 280                 285
   Gly Ser Leu Asn Gly Leu Asp Pro Thr Val Lys Thr Asn Asn Asn Pro
       290                 295                 300
   Thr Tyr Glu Ile Phe Ser Ala Thr Ala Val Ser Gln Gly Lys Asn Ile
   305                 310                 315                 320
   Asp Met Glu Ile Leu Glu Lys Asn Gln Pro Tyr Tyr Met Tyr Pro Phe
                   325                 330                 335
   Val His Leu Leu Asn Gly Gly Asn Leu Phe Val Phe Val Ser Lys Ser
               340                 345                 350
   Ser Gln Val Leu Asn Val Gly Thr Asn Thr Ile Val Lys Glu Leu Pro
           355                 360                 365
   Glu Leu Ala Gly Asp Tyr Arg Thr Tyr Pro Asn Thr Gly Gly Ser Val
       370                 375                 380
   Leu Leu Pro Leu Ser Ser Ala Asn Lys Trp Asn Pro Asp Ile Ile Ile
   385                 390                 395                 400
   Cys Gly Gly Gly Ala Tyr Gln Asp Ile Thr Ser Pro Thr Glu Pro Ser
                   405                 410                 415
   Cys Gly Arg Ile Gln Pro Leu Ser Ala Asn Pro Thr Trp Glu Leu Asp
               420                 425                 430
   Ala Met Pro Glu Gly Arg Gly Met Val Glu Gly Thr Leu Leu Pro Asp
           435                 440                 445
   Gly Thr Val Val Trp Leu Asn Gly Gly Asn Leu Gly Ala Gln Gly Phe
       450                 455                 460
   Gly Leu Ala Lys Asp Pro Thr Leu Glu Ala Leu Leu Tyr Asp Pro Thr
   465                 470                 475                 480
   Lys Ala Lys Gly Gln Arg Phe Ser Thr Leu Ala Thr Ser Thr Ile Pro
                   485                 490                 495
   Arg Leu Tyr His Ser Val Ser Leu Leu Leu Leu Asp Gly Thr Leu Met
               500                 505                 510
   Val Ala Gly Ser Asn Pro Val Glu Met Pro Lys Leu Gln Pro Asp Ala
           515                 520                 525
   Ala Asp Pro Tyr Val Thr Glu Phe Arg Val Glu Asn Tyr Val Pro Pro
       530                 535                 540
   Tyr Leu Ser Gly Asp Asn Ala Lys Lys Arg Pro Thr Asn Val Lys Leu
   545                 550                 555                 560
   Ser Ser Gly Ser Phe Lys Ala Asp Gly Ser Thr Leu Asp Val Thr Phe
                   565                 570                 575
   Asp Cys Pro Ala Gly Ala Lys Ala Val Thr Val Thr Leu Tyr His Gly
               580                 585                 590
   Gly Phe Val Thr His Ser Val His Met Gly His Arg Met Leu His Leu
           595                 600                 605
   Asp Asn Thr Gly Phe Gly Ala Gly Ala Thr Gln Gln Lys Leu Thr Val
       610                 615                 620
   Thr Arg Pro Pro Asn Asn Asn Val Ala Pro Pro Gly Pro Tyr Val Val
   625                 630                 635                 640
   Tyr Ile Leu Val Asp Gly Ile Pro Ala Met Gly Gln Phe Val Thr Val
                   645                 650                 655
<210> 11
   <211> 2024
   <212> DNA
   <213> Botrytis cinerea
<220>
   <221> CDS
   <222> (1)..(315)
<220>
   <221> CDS
   <222> (370)..(2022)
<400> 11
   atg cta att ttt acc gtt ttt agt tat tgt gga tct aca act gat cac       48
   Met Leu Ile Phe Thr Val Phe Ser Tyr Cys Gly Ser Thr Thr Asp His
   1               5                   10                  15
   tgt ttg gct tcc aat ggt tgc cag aat gga tgc aca ggc tca caa tct       96
   Cys Leu Ala Ser Asn Gly Cys Gln Asn Gly Cys Thr Gly Ser Gln Ser
               20                  25                  30
   tca tca gcc gcc aag act act acc aca gct gca gca ggc agc gca ccc      144
   Ser Ser Ala Ala Lys Thr Thr Thr Thr Ala Ala Ala Gly Ser Ala Pro
           35                  40                  45
   tct tca tct aca act caa gaa cca gtg att gcc cca gtt agt tct aca      192
   Ser Ser Ser Thr Thr Gln Glu Pro Val Ile Ala Pro Val Ser Ser Thr
       50                  55                  60
   ctt acg cct gcc gca gct agc agt gca cca gta act act gat gga tca      240
   Leu Thr Pro Ala Ala Ala Ser Ser Ala Pro Val Thr Thr Asp Gly Ser
   65                  70                  75                  80
   tgt ggt act gcc aat gga ggt acc gtt tgt ggc aat tgg gta aat gga      288
   Cys Gly Thr Ala Asn Gly Gly Thr Val Cys Gly Asn Trp Val Asn Gly
                   85                  90                  95
   aat tgt tgt tcc atg tac ggt ttt tg g taagtgcaat cattcactca           335
   Asn Cys Cys Ser Met Tyr Gly Phe Cys
               100                 105
   cccgcgaatc ttcgataatc taacacaatg tag t ggc agt acc aat gcg cat tgc   390
                                          Gly Ser Thr Asn Ala His Cys
                                                              110
   ggt gcc gga tgc caa tca gga gat tgt ttg aat gcg cct gcg gtt gca      438
   Gly Ala Gly Cys Gln Ser Gly Asp Cys Leu Asn Ala Pro Ala Val Ala
           115                 120                 125
   gct cct ggt gca agc cct gcc cca gct gcc cca gta gga ggt gcc ttt      486
   Ala Pro Gly Ala Ser Pro Ala Pro Ala Ala Pro Val Gly Gly Ala Phe
       130                 135                 140
   aat atc gtc ggg tcg tct gga gtt cct gct atg cat gct gca ctt atg      534
   Asn Ile Val Gly Ser Ser Gly Val Pro Ala Met His Ala Ala Leu Met
   145                 150                 155                 160
   cca aac ggt cga gtt atg ttc ctc gac aaa tta gag aac tac acc caa      582
   Pro Asn Gly Arg Val Met Phe Leu Asp Lys Leu Glu Asn Tyr Thr Gln
                   165                 170                 175
   ttg aaa ttg cca aat gga tac tac gcc atg tct tca gaa tac gac cca      630
   Leu Lys Leu Pro Asn Gly Tyr Tyr Ala Met Ser Ser Glu Tyr Asp Pro
               180                 185                 190
   gcc act aac gca gtc gcc act cct tta gct tac aaa aca aat gcg ttt      678
   Ala Thr Asn Ala Val Ala Thr Pro Leu Ala Tyr Lys Thr Asn Ala Phe
           195                 200                 205
   tgt tcc gga ggc act ttc ctt gct gat gga cgt gtt gtt tct ctt gga      726
   Cys Ser Gly Gly Thr Phe Leu Ala Asp Gly Arg Val Val Ser Leu Gly
       210                 215                 220
   ggc aac gcg cct tta gat tgg ctc gat cca aac att ggg gat gga ttt      774
   Gly Asn Ala Pro Leu Asp Trp Leu Asp Pro Asn Ile Gly Asp Gly Phe
   225                 230                 235                 240
   gac gcc att aga tat ctt gaa cga tca tct acc gat gct agc ctc aat      822
   Asp Ala Ile Arg Tyr Leu Glu Arg Ser Ser Thr Asp Ala Ser Leu Asn
                   245                 250                 255
   gga aaa gac tgg agt gaa cca ggt aac aag ctc gcg agt gct cgt tgg      870
   Gly Lys Asp Trp Ser Glu Pro Gly Asn Lys Leu Ala Ser Ala Arg Trp
               260                 265                 270
   tat gct act gct caa act atg ggt gat gga acc att ttg gtc gct ttt      918
   Tyr Ala Thr Ala Gln Thr Met Gly Asp Gly Thr Ile Leu Val Ala Phe
           275                 280                 285
   gga agt ttg aac ggc ctc gat ccg act gtc aaa acg aac aac aat cct      966
   Gly Ser Leu Asn Gly Leu Asp Pro Thr Val Lys Thr Asn Asn Asn Pro
       290                 295                 300
   aca tac gag att ttc agt gct acc gct gtg tcg caa ggt aag aac att     1014
   Thr Tyr Glu Ile Phe Ser Ala Thr Ala Val Ser Gln Gly Lys Asn Ile
   305                 310                 315                 320
   gac atg gaa att ttg gag aaa aat cag cca tat tat atg tat cct ttt     1062
   Asp Met Glu Ile Leu Glu Lys Asn Gln Pro Tyr Tyr Met Tyr Pro Phe
                   325                 330                 335
   gtt cat ctc ctc aat ggt gga aat ttg ttc gtc ttc gtt tcc aag tct     1110
   Val His Leu Leu Asn Gly Gly Asn Leu Phe Val Phe Val Ser Lys Ser
               340                 345                 350
   tcc caa gta ctc aat gtc ggt acc aac act atc gtc aag gaa tta cct     1158
   Ser Gln Val Leu Asn Val Gly Thr Asn Thr Ile Val Lys Glu Leu Pro
           355                 360                 365
   gaa ctt gct gga gac tat cgc aca tat ccc aac act ggt gga agt gtt      1206
   Glu Leu Ala Gly Asp Tyr Arg Thr Tyr Pro Asn Thr Gly Gly Ser Val
       370                 375                 380
   tta ctc cct ttg tca agc gca aac aaa tgg aac cct gat atc atc atc      1254
   Leu Leu Pro Leu Ser Ser Ala Asn Lys Trp Asn Pro Asp Ile Ile Ile
   385                 390                 395                 400
   tgc ggg gga ggt gca tat caa gat att acc agt cca aca gag cca agt      1302
   Cys Gly Gly Gly Ala Tyr Gln Asp Ile Thr Ser Pro Thr Glu Pro Ser
                   405                 410                 415
   tgt gga aga atc cag cca ttg agt gca aac ccc aca tgg gag ttg gac      1350
   Cys Gly Arg Ile Gln Pro Leu Ser Ala Asn Pro Thr Trp Glu Leu Asp
               420                 425                 430
   gct atg cct gaa ggc cgt ggt atg gtt gaa gga acc tta ctt cca gat      1398
   Ala Met Pro Glu Gly Arg Gly Met Val Glu Gly Thr Leu Leu Pro Asp
           435                 440                 445
   gga aca gtt gtc tgg ctt aat gga ggg aac ttg ggt gct caa gga ttt      1446
   Gly Thr Val Val Trp Leu Asn Gly Gly Asn Leu Gly Ala Gln Gly Phe
       450                 455                 460
   gga ctt gca aaa gac cca aca ttg gaa gct ctt ctt tac gat cct acg      1494
   Gly Leu Ala Lys Asp Pro Thr Leu Glu Ala Leu Leu Tyr Asp Pro Thr
   465                 470                 475                 480
   aaa gct aag ggt caa aga ttc tca act ctt gca aca tca act atc cca      1542
   Lys Ala Lys Gly Gln Arg Phe Ser Thr Leu Ala Thr Ser Thr Ile Pro
                   485                 490                 495
   cgt ctc tac cat tct gtc tct ctc ctc ctt ctt gac ggt aca cta atg      1590
   Arg Leu Tyr His Ser Val Ser Leu Leu Leu Leu Asp Gly Thr Leu Met
               500                 505                 510
   gtc gct ggc tca aac cct gtc gag atg cca aag ctt caa cca gat gca      1638
   Val Ala Gly Ser Asn Pro Val Glu Met Pro Lys Leu Gln Pro Asp Ala
           515                 520                 525
   gcc gat cca tat gtt acg gag ttc cga gtt gag aac tat gtt cct ccc      1686
   Ala Asp Pro Tyr Val Thr Glu Phe Arg Val Glu Asn Tyr Val Pro Pro
       530                 535                 540
   tat ctc tca ggc gat aat gca aag aag cgt cct act aat gta aaa ttg      1734
   Tyr Leu Ser Gly Asp Asn Ala Lys Lys Arg Pro Thr Asn Val Lys Leu
   545                 550                 555                 560
   tca tca ggt agc ttc aaa gca gat ggt agc aca ctt gat gtc aca ttt      1782
   Ser Ser Gly Ser Phe Lys Ala Asp Gly Ser Thr Leu Asp Val Thr Phe
                   565                 570                 575
   gat tgt cca gct ggc gcg aaa gca gtt act gta act ttg tac cac ggt      1830
   Asp Cys Pro Ala Gly Ala Lys Ala Val Thr Val Thr Leu Tyr His Gly
               580                 585                 590
   gga ttc gtc act cac tct gta cat atg ggt cat cgc atg ctg cac ttg      1878
   Gly Phe Val Thr His Ser Val His Met Gly His Arg Met Leu His Leu
           595                 600                 605
   gat aac aca ggc ttc ggc gct ggt gcc aca cag cag aag ttg act gtt      1926
   Asp Asn Thr Gly Phe Gly Ala Gly Ala Thr Gln Gln Lys Leu Thr Val
       610                 615                 620
   act cga cca cca aac aac aat gtt gca cct cca ggt cca tat gtt gtt     1974
   Thr Arg Pro Pro Asn Asn Asn Val Ala Pro Pro Gly Pro Tyr Val Val
   625                 630                 635                 640
   tac att ctt gta gac ggc att cct gcc atg gga cag ttt gtt acg gtt     2022
   Tyr Ile Leu Val Asp Gly Ile Pro Ala Met Gly Gln Phe Val Thr Val
                   645                 650                 655
   tg                                                                   2024
<210> 12
   <211> 656
   <212> PRT
   <213> Botrytis cinerea
<400> 12
   Met Leu Ile Phe Thr Val Phe Ser Tyr Cys Gly Ser Thr Thr Asp His
   1               5                   10                  15
   Cys Leu Ala Ser Asn Gly Cys Gln Asn Gly Cys Thr Gly Ser Gln Ser
               20                  25                  30
   Ser Ser Ala Ala Lys Thr Thr Thr Thr Ala Ala Ala Gly Ser Ala Pro
           35                  40                  45
   Ser Ser Ser Thr Thr Gln Glu Pro Val Ile Ala Pro Val Ser Ser Thr
       50                  55                  60
   Leu Thr Pro Ala Ala Ala Ser Ser Ala Pro Val Thr Thr Asp Gly Ser
   65                  70                  75                  80
   Cys Gly Thr Ala Asn Gly Gly Thr Val Cys Gly Asn Trp Val Asn Gly
                   85                  90                  95
   Asn Cys Cys Ser Met Tyr Gly Phe Cys Gly Ser Thr Asn Ala His Cys
               100                 105                 110
   Gly Ala Gly Cys Gln Ser Gly Asp Cys Leu Asn Ala Pro Ala Val Ala
           115                 120                 125
   Ala Pro Gly Ala Ser Pro Ala Pro Ala Ala Pro Val Gly Gly Ala Phe
       130                 135                 140
   Asn Ile Val Gly Ser Ser Gly Val Pro Ala Met His Ala Ala Leu Met
   145 150 155 160
   Pro Asn Gly Arg Val Met Phe Leu Asp Lys Leu Glu Asn Tyr Thr Gln
   165 170 175
   Leu Lys Leu Pro Asn Gly Tyr Tyr Ala Met Ser Ser Glu Tyr Asp Pro
   180 185 190
   Ala Thr Asn Ala Val Ala Thr Pro Leu Ala Tyr Lys Thr Asn Ala Phe
   195 200 205
   Cys Ser Gly Gly Thr Phe Leu Ala Asp Gly Arg Val Val Ser Leu Gly
   210 215 220
   Gly Asn Ala Pro Leu Asp Trp Leu Asp Pro Asn Ile Gly Asp Gly Phe
   225 230 235 240
   Asp Ala Ile Arg Tyr Leu Glu Arg Ser Ser Thr Asp Ala Ser Leu Asn
   245 250 255
   Gly Lys Asp Trp Ser Glu Pro Gly Asn Lys Leu Ala Ser Ala Arg Trp
   260 265 270
   Tyr Ala Thr Ala Gln Thr Met Gly Asp Gly Thr Ile Leu Val Ala Phe
   275 280 285
   Gly Ser Leu Asn Gly Leu Asp Pro Thr Val Lys Thr Asn Asn Asn Pro
   290 295 300
   Thr Tyr Glu Ile Phe Ser Ala Thr Ala Val Ser Gln Gly Lys Asn Ile
   305 310 315 320
   Asp Met Glu Ile Leu Glu Lys Asn Gln Pro Tyr Tyr Met Tyr Pro Phe
   325 330 335
   Val His Leu Leu Asn Gly Gly Asn Leu Phe Val Phe Val Ser Lys Ser
   340 345 350
   Ser Gln Val Leu Asn Val Gly Thr Asn Thr Ile Val Lys Glu Leu Pro
   355 360 365
   Glu Leu Ala Gly Asp Tyr Arg Thr Tyr Pro Asn Thr Gly Gly Ser Val
   370 375 380
   Leu Leu Pro Leu Ser Ser Ala Asn Lys Trp Asn Pro Asp Ile Ile Ile
   385 390 395 400
   Cys Gly Gly Gly Ala Tyr Gln Asp Ile Thr Ser Pro Thr Glu Pro Ser
   405 410 415
   Cys Gly Arg Ile Gln Pro Leu Ser Ala Asn Pro Thr Trp Glu Leu Asp
   420 425 430
   Ala Met Pro Glu Gly Arg Gly Met Val Glu Gly Thr Leu Leu Pro Asp
   435 440 445
   Gly Thr Val Val Trp Leu Asn Gly Gly Asn Leu Gly Ala Gln Gly Phe
   450 455 460
   Gly Leu Ala Lys Asp Pro Thr Leu Glu Ala Leu Leu Tyr Asp Pro Thr
   465 470 475 480
   Lys Ala Lys Gly Gln Arg Phe Ser Thr Leu Ala Thr Ser Thr Ile Pro
   485 490 495
   Arg Leu Tyr His Ser Val Ser Leu Leu Leu Leu Asp Gly Thr Leu Met
   500 505 510
   Val Ala Gly Ser Asn Pro Val Glu Met Pro Lys Leu Gln Pro Asp Ala
   515 520 525
   Ala Asp Pro Tyr Val Thr Glu Phe Arg Val Glu Asn Tyr Val Pro Pro
   530 535 540
   Tyr Leu Ser Gly Asp Asn Ala Lys Lys Arg Pro Thr Asn Val Lys Leu
   545 550 555 560
   Ser Ser Gly Ser Phe Lys Ala Asp Gly Ser Thr Leu Asp Val Thr Phe
   565 570 575
   Asp Cys Pro Ala Gly Ala Lys Ala Val Thr Val Thr Leu Tyr His Gly
   580 585 590
   Gly Phe Val Thr His Ser Val His Met Gly His Arg Met Leu His Leu
   595 600 605
   Asp Asn Thr Gly Phe Gly Ala Gly Ala Thr Gln Gln Lys Leu Thr Val
   610 615 620
   Thr Arg Pro Pro Asn Asn Asn Val Ala Pro Pro Gly Pro Tyr Val Val
   625 630 635 640
   Tyr Ile Leu Val Asp Gly Ile Pro Ala Met Gly Gln Phe Val Thr Val
   645 650 655

### SEQUENCE LISTING

<110> Bayer AG
<120> Glyoxaloxidasen aus Pilzen
<130> Le A 35 261
<160> 12
<170> Patent In version 3.0
<210> 1
   <211> 2589
   <212> DNA
   <213> Ustilago maydis
<220>
   <221> CDS
   <222> (1) .. (2589)
<400> 1
   atg acg agg cac ctc tcc tca tcc tcg agg cgc tcc tcg ctc gcc aaa       48
   Met Thr Arg His Leu Ser Ser Ser Ser Arg Arg Ser Ser Leu Ala Lys
   1               5                   10                  15
   agc gcc atg acc ctc gca acc ctt tct ctc gcc cta acc tcg tgc gca       96
   Ser Ala Met Thr Leu Ala Thr Leu Ser Leu Ala Leu Thr Ser Cys Ala
               20                  25                  30
   tcg gcc gcc agc aag gcc ggc tca tac gag gtt gtc aac acc aac tca      144
   Ser Ala Ala Ser Lys Ala Gly Ser Tyr Glu Val Val Asn Thr Asn Ser
           35                  40                  45
   ctc gcc tcg gcc atg atg ctc ggt tta atg gac gag gac aac gtc ttt      192
   Leu Ala Ser Ala Met Met Leu Gly Leu Met Asp Glu Asp Asn Val Phe
       50                  55                  60
   att ctc gac aaa gct gaa aac aac tcg gct cgt ctc gcc gat ggc cgt      240
   Ile Leu Asp Lys Ala Glu Asn Asn Ser Ala Arg Leu Ala Asp Gly Arg
   65                  70                  75                  80
   cat gtc tgg ggt tct ttc tac aag ctt tcc gac aat tcg gtc acc ggc      288
   His Val Trp Gly Ser Phe Tyr Lys Leu Ser Asp Asn Ser Val Thr Gly
                   85                  90                  95
   acc gcc gtc cag acc aac act ttc tgt gcc tct ggt gcc acc ttg gga      336
   Thr Ala Val Gln Thr Asn Thr Phe Cys Ala Ser Gly Ala Thr Leu Gly
               100                 105                 110
   aat ggt tct tgg ctt gta gct ggc ggc aac cag gcc gta ggt tac ggt      384
   Asn Gly Ser Trp Leu Val Ala Gly Gly Asn Gln Ala Val Gly Tyr Gly
           115                 120                 125
   ggc gct gca cag gcc cag gag atc aac ccc tac tcg gac ttc gac gga      432
   Gly Ala Ala Gln Ala Gln Glu Ile Asn Pro Tyr Ser Asp Phe Asp Gly
       130                 135                 140
   act agg gcg att cgt ctg ctc gaa ccc aac tcg cag acg tgg atc gac      480
   Thr Arg Ala Ile Arg Leu Leu Glu Pro Asn Ser Gln Thr Trp Ile Asp
   145                 150                 155                 160
   tcg ccc agt aca act gtc gca cag gtc aac atg ctc cag caa ccc cgt      528
   Ser Pro Ser Thr Thr Val Ala Gln Val Asn Met Leu Gln Gln Pro Arg
                   165                 170                 175
   tgg tac ccc ggt atc gag gtt ctt gaa gac ggt agc gtt atc ttt atc      576
   Trp Tyr Pro Gly Ile Glu Val Leu Glu Asp Gly Ser Val Ile Phe Ile
               180                 185                 190
   gga ggt gcc gtc tcg ggc ggc tac att aat cgc aac acg cct acc act      624
   Gly Gly Ala Val Ser Gly Gly Tyr Ile Asn Arg Asn Thr Pro Thr Thr
           195                 200                 205
   gat cct ctt tac cag aat gga ggc gct aac ccc acc tac gaa tac ttt      672
   Asp Pro Leu Tyr Gln Asn Gly Gly Ala Asn Pro Thr Tyr Glu Tyr Phe
       210                 215                 220
   ccc tcc aag acc acc gga aac cta ccc atc tgt aac ttt atg gct cag      720
   Pro Ser Lys Thr Thr Gly Asn Leu Pro Ile Cys Asn Phe Met Ala Gln
   225                 230                 235                 240
   act aac ggt ctc aac atg tac ccg cac acc tac ctc atg ccc tct ggc      768
   Thr Asn Gly Leu Asn Met Tyr Pro His Thr Tyr Leu Met Pro Ser Gly
                   245                 250                 255
   aag atc ttc atg cag gcc aac gtc agt acc atc ctc tgg gac cac gtc      816
   Lys Ile Phe Met Gln Ala Asn Val Ser Thr Ile Leu Trp Asp His Val
               260                 265                 270
   aac aac act cag atc gac ctt ccc gac atg cct ggc gga gtc gtg cgc      864
   Asn Asn Thr Gln Ile Asp Leu Pro Asp Met Pro Gly Gly Val Val Arg
           275                 280                 285
   gtc tac ccc gcc tcg gct gcc act gcc atg ctg cca ctc act cct cag      912
   Val Tyr Pro Ala Ser Ala Ala Thr Ala Met Leu Pro Leu Thr Pro Gln
       290                 295                 300
   aat cag tac aca cct acc atc ctg ttt tgc ggt ggt agt gtc atg agc      960
   Asn Gln Tyr Thr Pro Thr Ile Leu Phe Cys Gly Gly Ser Val Met Ser
   305                 310                 315                 320
   gac cag atg tgg ggc aac tac agt ggt ccc ggt ggc aac att ctc ggt     1008
   Asp Gln Met Trp Gly Asn Tyr Ser Gly Pro Gly Gly Asn Ile Leu Gly
                   325                 330                 335
   ctc caa gcc tct gat gac tgc tcg tcc atc aac ccc gag gac aat cag     1056
   Leu Gln Ala Ser Asp Asp Cys Ser Ser Ile Asn Pro Glu Asp Asn Gln
               340                 345                 350
   ggc aac cag atc act gac gct cag tac gtc cag gag ggg cgg ctt ccc     1104
   Gly Asn Gln Ile Thr Asp Ala Gln Tyr Val Gln Glu Gly Arg Leu Pro
           355                 360                 365
   gaa ggt cgt tcc atg gga cag ttc atc cac ctc cct gac ggt acc atg      1152
   Glu Gly Arg Ser Met Gly Gln Phe Ile His Leu Pro Asp Gly Thr Met
       370                 375                 380
   gtc gtc ctc aac ggc gcc aac aag gga act gcc ggc tat tcg aac cag      1200
   Val Val Leu Asn Gly Ala Asn Lys Gly Thr Ala Gly Tyr Ser Asn Gln
   385                 390                 395                 400
   aca tgg aac acc atc cag tac aac ggt cgc acc gtc gtc acc gaa ggt      1248
   Thr Trp Asn Thr Ile Gln Tyr Asn Gly Arg Thr Val Val Thr Glu Gly
                   405                 410                 415
   ctt tcg cag gat ccc act tac gtt ccc gtc atc tat gac ccg tcc aag      1296
   Leu Ser Gln Asp Pro Thr Tyr Val Pro Val Ile Tyr Asp Pro Ser Lys
               420                 425                 430
   ccc aga ggt cag cgt ctc tcc aat gct aat ctc aag cct tcc acc att      1344
   Pro Arg Gly Gln Arg Leu Ser Asn Ala Asn Leu Lys Pro Ser Thr Ile
           435                 440                 445
   gct cgt ctc tac cac tcg agc gct att ttg ctc ccc gat ggt tcc gtc      1392
   Ala Arg Leu Tyr His Ser Ser Ala Ile Leu Leu Pro Asp Gly Ser Val
       450                 455                 460
   atg gtt gca ggt tcc aac ccg cat cag gat gtt gcg ctc gac atg ccc      1440
   Met Val Ala Gly Ser Asn Pro His Gln Asp Val Ala Leu Asp Met Pro
   465                 470                 475                 480
   acc ggc acc acg cct cag gct ttc aac acc acc tac gag gtt gaa aag      1488
   Thr Gly Thr Thr Pro Gln Ala Phe Asn Thr Thr Tyr Glu Val Glu Lys
                   485                 490                 495
   tgg tac cct cct tac tgg gac tcg cca cgc cct tac cca cag ggc gtg      1536
   Trp Tyr Pro Pro Tyr Trp Asp Ser Pro Arg Pro Tyr Pro Gln Gly Val
               500                 505                 510
   ccc aat tcg gtg ctg tac ggc ggc agt cct ttc aac att acc gtc aac      1584
   Pro Asn Ser Val Leu Tyr Gly Gly Ser Pro Phe Asn Ile Thr Val Asn
           515                 520                 525
   ggt acc ttt atg ggt gac tcg gcc aac gcc aag gca gcc aac acc aag      1632
   Gly Thr Phe Met Gly Asp Ser Ala Asn Ala Lys Ala Ala Asn Thr Lys
       530                 535                 540
   ttt gcc atc att cgt acc ggt ttc tcc acc cac gcc atg aac atg ggg      1680
   Phe Ala Ile Ile Arg Thr Gly Phe Ser Thr His Ala Met Asn Met Gly
   545                 550                 555                 560
   cag cgc gcc gtc tac ctc gac tac acc tac acc gtt aac gat gac gcc      1728
   Gln Arg Ala Val Tyr Leu Asp Tyr Thr Tyr Thr Val Asn Asp Asp Ala
                   565                 570                 575
   tcg gtc acc tac atg gtc aac cct ttg ccc aac act aag gct atg aac      1776
   Ser Val Thr Tyr Met Val Asn Pro Leu Pro Asn Thr Lys Ala Met Asn
               580                 585                 590
   cgc ctc ttt gtg cct ggc ccg gcc ttc ttc tac gtc acc gtc ggt ggc      1824
   Arg Leu Phe Val Pro Gly Pro Ala Phe Phe Tyr Val Thr Val Gly Gly
           595                 600                 605
   gtg cca agc cat ggc aag ctg atc atg gtg gga act tcc ccc act ggc      1872
   Val Pro Ser His Gly Lys Leu Ile Met Val Gly Thr Ser Pro Thr Gly
       610                 615                 620
   act ggc aac gtc ccc ttc act cct cag ctc ggg tct gca ctc gtc gcg      1920
   Thr Gly Asn Val Pro Phe Thr Pro Gln Leu Gly Ser Ala Leu Val Ala
   625                 630                 635                 640
   ctt ccc cct gct gtc aac agc acc aaa ttc aca gcc tcc ctc ccc aag      1968
   Leu Pro Pro Ala Val Asn Ser Thr Lys Phe Thr Ala Ser Leu Pro Lys
                   645                 650                 655
   gct ggc agc agc tct tcc tcc gag ttt ggc ctc ggc aag atc att ggt      2016
   Ala Gly Ser Ser Ser Ser Ser Glu Phe Gly Leu Gly Lys Ile Ile Gly
               660                 665                 670
   atc gct gtt gct ggc gcc gca gtt ttg gcc ctc att gct ctc ggc tgt      2064
   Ile Ala Val Ala Gly Ala Ala Val Leu Ala Leu Ile Ala Leu Gly Cys
           675                 680                 685
   tgt ctg tgg agg cgc aag ggc agg agc cat agc gac aag gct gcc tcg      2112
   Cys Leu Trp Arg Arg Lys Gly Arg Ser His Ser Asp Lys Ala Ala Ser
       690                 695                 700
   cgc cag tcg gct gcc cct tgg acc agc cgc gac ctt ggc tcg ggt ccc      2160
   Arg Gln Ser Ala Ala Pro Trp Thr Ser Arg Asp Leu Gly Ser Gly Pro
   705                 710                 715                 720
   gag tac aag cgt gtc gac act cct gtc gga tcc atc agc ggt ggt cgc      2208
   Glu Tyr Lys Arg Val Asp Thr Pro Val Gly Ser Ile Ser Gly Gly Arg
                   725                 730                 735
   ttt ggg gcc gcc agg atg gac agc tcg aat acg ttt gag agc tat cgg      2256
   Phe Gly Ala Ala Arg Met Asp Ser Ser Asn Thr Phe Glu Ser Tyr Arg
               740                 745                 750
   ttg cac gac cag gtc agc acg agc gaa agc aag gag gcg att ggc agc      2304
   Leu His Asp Gln Val Ser Thr Ser Glu Ser Lys Glu Ala Ile Gly Ser
           755                 760                 765
   tac tac gac caa cct cgc agc ggc agc cgt ggc ggc tac gct cct agc      2352
   Tyr Tyr Asp Gln Pro Arg Ser Gly Ser Arg Gly Gly Tyr Ala Pro Ser
       770                 775                 780
   ccg ctc gcc tac gac caa cac gga cgt ggc gcc tcg caa ggc cag tac      2400
   Pro Leu Ala Tyr Asp Gln His Gly Arg Gly Ala Ser Gln Gly Gln Tyr
   785                 790                 795                 800
   cac cag caa ggc tgg ggc gaa tac cac gct ggc gat gct ggt gca tac      2448
   His Gln Gln Gly Trp Gly Glu Tyr His Ala Gly Asp Ala Gly Ala Tyr
                   805                 810                 815
   tac gag gac aac act agc agg tac ggc agc ggt ggc ggt gga cac agc      2496
   Tyr Glu Asp Asn Thr Ser Arg Tyr Gly Ser Gly Gly Gly Gly His Ser
               820                 825                 830
   tac gat gat tac tcg cac cag caa tac caa cag cag cat tac tat gac      2544
   Tyr Asp Asp Tyr Ser His Gln Gln Tyr Gln Gln Gln His Tyr Tyr Asp
           835                 840                 845
   agc cca ggt cat cag cac caa gga agc tac tct agt cga cgc taa          2589
   Ser Pro Gly His Gln His Gln Gly Ser Tyr Ser Ser Arg Arg
       850                 855                 860
<210> 2
   <211> 862
   <212> PRT
   <213> Ustilago maydis
<400> 2
   Met Thr Arg His Leu Ser Ser Ser Ser Arg Arg Ser Ser Leu Ala Lys
   1               5                   10                  15
   Ser Ala Met Thr Leu Ala Thr Leu Ser Leu Ala Leu Thr Ser Cys Ala
               20                  25                  30
   Ser Ala Ala Ser Lys Ala Gly Ser Tyr Glu Val Val Asn Thr Asn Ser
           35                  40                  45
   Leu Ala Ser Ala Met Met Leu Gly Leu Met Asp Glu Asp Asn Val Phe
       50                  55                  60
   Ile Leu Asp Lys Ala Glu Asn Asn Ser Ala Arg Leu Ala Asp Gly Arg
   65                  70                  75                  80
   His Val Trp Gly Ser Phe Tyr Lys Leu Ser Asp Asn Ser Val Thr Gly
                   85                  90                  95
   Thr Ala Val Gln Thr Asn Thr Phe Cys Ala Ser Gly Ala Thr Leu Gly
               100                 105                 110
   Asn Gly Ser Trp Leu Val Ala Gly Gly Asn Gln Ala Val Gly Tyr Gly
           115                 120                 125
   Gly Ala Ala Gln Ala Gln Glu Ile Asn Pro Tyr Ser Asp Phe Asp Gly
       130                 135                 140
   Thr Arg Ala Ile Arg Leu Leu Glu Pro Asn Ser Gln Thr Trp Ile Asp
   145                 150                 155                 160
   Ser Pro Ser Thr Thr Val Ala Gln Val Asn Met Leu Gln Gln Pro Arg
                   165                 170                 175
   Trp Tyr Pro Gly Ile Glu Val Leu Glu Asp Gly Ser Val Ile Phe Ile
               180                 185                 190
   Gly Gly Ala Val Ser Gly Gly Tyr Ile Asn Arg Asn Thr Pro Thr Thr
           195                 200                 205
   Asp Pro Leu Tyr Gln Asn Gly Gly Ala Asn Pro Thr Tyr Glu Tyr Phe
       210                 215                 220
   Pro Ser Lys Thr Thr Gly Asn Leu Pro Ile Cys Asn Phe Met Ala Gln
   225                 230                 235                 240
   Thr Asn Gly Leu Asn Met Tyr Pro His Thr Tyr Leu Met Pro Ser Gly
                   245                 250                 255
   Lys Ile Phe Met Gln Ala Asn Val Ser Thr Ile Leu Trp Asp His Val
               260                 265                 270
   Asn Asn Thr Gln Ile Asp Leu Pro Asp Met Pro Gly Gly Val Val Arg
           275                 280                 285
   Val Tyr Pro Ala Ser Ala Ala Thr Ala Met Leu Pro Leu Thr Pro Gln
       290                 295                 300
   Asn Gln Tyr Thr Pro Thr Ile Leu Phe Cys Gly Gly Ser Val Met Ser
   305                 310                 315                 320
   Asp Gln Met Trp Gly Asn Tyr Ser Gly Pro Gly Gly Asn Ile Leu Gly
                   325                 330                 335
   Leu Gln Ala Ser Asp Asp Cys Ser Ser Ile Asn Pro Glu Asp Asn Gln
               340                 345                 350
   Gly Asn Gln Ile Thr Asp Ala Gln Tyr Val Gln Glu Gly Arg Leu Pro
           355                 360                 365
   Glu Gly Arg Ser Met Gly Gln Phe Ile His Leu Pro Asp Gly Thr Met
       370                 375                 380
   Val Val Leu Asn Gly Ala Asn Lys Gly Thr Ala Gly Tyr Ser Asn Gln
   385                 390                 395                 400
   Thr Trp Asn Thr Ile Gln Tyr Asn Gly Arg Thr Val Val Thr Glu Gly
                   405                 410                 415
   Leu Ser Gln Asp Pro Thr Tyr Val Pro Val Ile Tyr Asp Pro Ser Lys
               420                 425                 430
   Pro Arg Gly Gln Arg Leu Ser Asn Ala Asn Leu Lys Pro Ser Thr Ile
           435                 440                 445
   Ala Arg Leu Tyr His Ser Ser Ala Ile Leu Leu Pro Asp Gly Ser Val
   450 455 460
   Met Val Ala Gly Ser Asn Pro His Gln Asp Val Ala Leu Asp Met Pro
   465 470 475 480
   Thr Gly Thr Thr Pro Gln Ala Phe Asn Thr Thr Tyr Glu Val Glu Lys
   485 490 495
   Trp Tyr Pro Pro Tyr Trp Asp Ser Pro Arg Pro Tyr Pro Gln Gly Val
   500 505 510
   Pro Asn Ser Val Leu Tyr Gly Gly Ser Pro Phe Asn Ile Thr Val Asn
   515 520 525
   Gly Thr Phe Met Gly Asp Ser Ala Asn Ala Lys Ala Ala Asn Thr Lys
   530 535 540
   Phe Ala Ile Ile Arg Thr Gly Phe Ser Thr His Ala Met Asn Met Gly
   545 550 555 560
   Gln Arg Ala Val Tyr Leu Asp Tyr Thr Tyr Thr Val Asn Asp Asp Ala
   565 570 575
   Ser Val Thr Tyr Met Val Asn Pro Leu Pro Asn Thr Lys Ala Met Asn
   580 585 590
   Arg Leu Phe Val Pro Gly Pro Ala Phe Phe Tyr Val Thr Val Gly Gly
   595 600 605
   Val Pro Ser His Gly Lys Leu Ile Met Val Gly Thr Ser Pro Thr Gly
   610 615 620
   Thr Gly Asn Val Pro Phe Thr Pro Gln Leu Gly Ser Ala Leu Val Ala
   625 630 635 640
   Leu Pro Pro Ala Val Asn Ser Thr Lys Phe Thr Ala Ser Leu Pro Lys
   645 650 655
   Ala Gly Ser Ser Ser Ser Ser Glu Phe Gly Leu Gly Lys Ile Ile Gly
   660 665 670
   Ile Ala Val Ala Gly Ala Ala Val Leu Ala Leu Ile Ala Leu Gly Cys
   675 680 685
   Cys Leu Trp Arg Arg Lys Gly Arg Ser His Ser Asp Lys Ala Ala Ser
   690 695 700
   Arg Gln Ser Ala Ala Pro Trp Thr Ser Arg Asp Leu Gly Ser Gly Pro
   705 710 715 720
   Glu Tyr Lys Arg Val Asp Thr Pro Val Gly Ser Ile Ser Gly Gly Arg
   725 730 735
   Phe Gly Ala Ala Arg Met Asp Ser Ser Asn Thr Phe Glu Ser Tyr Arg
   740 745 750
   Leu His Asp Gln Val Ser Thr Ser Glu Ser Lys Glu Ala Ile Gly Ser
   755 760 765
   Tyr Tyr Asp Gln Pro Arg Ser Gly Ser Arg Gly Gly Tyr Ala Pro Ser
   770 775 780
   Pro Leu Ala Tyr Asp Gln His Gly Arg Gly Ala Ser Gln Gly Gln Tyr
   785 790 795 800
   His Gln Gln Gly Trp Gly Glu Tyr His Ala Gly Asp Ala Gly Ala Tyr
   805 810 815
   Tyr Glu Asp Asn Thr Ser Arg Tyr Gly Ser Gly Gly Gly Gly His Ser
   820 825 830
   Tyr Asp Asp Tyr Ser His Gln Gln Tyr Gln Gln Gln His Tyr Tyr Asp
   835 840 845
   Ser Pro Gly His Gln His Gln Gly Ser Tyr Ser Ser Arg Arg
   850 855 860
<210> 3
   <211> 2923
   <212> DNA
   <213> Ustilago maydis
<220>
   <221> CDS
   <222> (238)..(2823)
<400> 3
   acgttccttc tcccttttcc tcgctttcac cactgcctcg acgttccttc tttggcttct 60
   gcagttctga ctgttgccac tttttcgtcc cctccgtctc gcctttgatt tatcaccacc 120
   gcgcactcat tggctgcggc gaattaccac gctttgggct cacgccatcc atcgctcagc 180
   cacatttcca ttcaatatca ctgagctctg tcttccagaa aggatcgttt acacacc 237
   atg acg agg cac ctc tcc tca tcc tcg agg cgc tcc tcg ctc gcc aaa      285
   Met Thr Arg His Leu Ser Ser Ser Ser Arg Arg Ser Ser Leu Ala Lys
   1               5                   10                  15
   agc gcc atg acc ctc gca acc ctt tct ctc gcc cta acc tcg tgc gca      333
   Ser Ala Met Thr Leu Ala Thr Leu Ser Leu Ala Leu Thr Ser Cys Ala
               20                  25                  30
   tcg gcc gcc agc aag gcc ggc tca tac gag gtt gtc aac acc aac tca      381
   Ser Ala Ala Ser Lys Ala Gly Ser Tyr Glu Val Val Asn Thr Asn Ser
           35                  40                  45
   ctc gcc tcg gcc atg atg ctc ggt tta atg gac gag gac aac gtc ttt      429
   Leu Ala Ser Ala Met Met Leu Gly Leu Met Asp Glu Asp Asn Val Phe
       50                  55                  60
   att ctc gac aaa gct gaa aac aac tcg gct cgt ctc gcc gat ggc cgt      477
   Ile Leu Asp Lys Ala Glu Asn Asn Ser Ala Arg Leu Ala Asp Gly Arg
   65                  70                  75                  80
   cat gtc tgg ggt tct ttc tac aag ctt tcc gac aat tcg gtc acc ggc      525
   His Val Trp Gly Ser Phe Tyr Lys Leu Ser Asp Asn Ser Val Thr Gly
                   85                  90                  95
   acc gcc gtc cag acc aac act ttc tgt gcc tct ggt gcc acc ttg gga      573
   Thr Ala Val Gln Thr Asn Thr Phe Cys Ala Ser Gly Ala Thr Leu Gly
               100                 105                 110
   aat ggt tct tgg ctt gta gct ggc ggc aac cag gcc gta ggt tac ggt      621
   Asn Gly Ser Trp Leu Val Ala Gly Gly Asn Gln Ala Val Gly Tyr Gly
           115                 120                 125
   ggc gct gca cag gcc cag gag atc aac ccc tac tcg gac ttc gac gga      669
   Gly Ala Ala Gln Ala Gln Glu Ile Asn Pro Tyr Ser Asp Phe Asp Gly
       130                 135                 140
   act agg gcg att cgt ctg ctc gaa ccc aac tcg cag acg tgg atc gac      717
   Thr Arg Ala Ile Arg Leu Leu Glu Pro Asn Ser Gln Thr Trp Ile Asp
   145                 150                 155                 160
   tcg ccc agt aca act gtc gca cag gtc aac atg ctc cag caa ccc cgt      765
   Ser Pro Ser Thr Thr Val Ala Gln Val Asn Met Leu Gln Gln Pro Arg
                   165                 170                 175
   tgg tac ccc ggt atc gag gtt ctt gaa gac ggt agc gtt atc ttt atc      813
   Trp Tyr Pro Gly Ile Glu Val Leu Glu Asp Gly Ser Val Ile Phe Ile
               180                 185                 190
   gga ggt gcc gtc tcg ggc ggc tac att aat cgc aac acg cct acc act      861
   Gly Gly Ala Val Ser Gly Gly Tyr Ile Asn Arg Asn Thr Pro Thr Thr
           195                 200                 205
   gat cct ctt tac cag aat gga ggc gct aac ccc acc tac gaa tac ttt      909
   Asp Pro Leu Tyr Gln Asn Gly Gly Ala Asn Pro Thr Tyr Glu Tyr Phe
       210                 215                 220
   ccc tcc aag acc acc gga aac cta ccc atc tgt aac ttt atg gct cag      957
   Pro Ser Lys Thr Thr Gly Asn Leu Pro Ile Cys Asn Phe Met Ala Gln
   225                 230                 235                 240
   act aac ggt ctc aac atg tac ccg cac acc tac ctc atg ccc tct ggc     1005
   Thr Asn Gly Leu Asn Met Tyr Pro His Thr Tyr Leu Met Pro Ser Gly
                   245                 250                 255
   aag atc ttc atg cag gcc aac gtc agt acc atc ctc tgg gac cac gtc      1053
   Lys Ile Phe Met Gln Ala Asn Val Ser Thr Ile Leu Trp Asp His Val
               260                 265                 270
   aac aac act cag atc gac ctt ccc gac atg cct ggc gga gtc gtg cgc      1101
   Asn Asn Thr Gln Ile Asp Leu Pro Asp Met Pro Gly Gly Val Val Arg
           275                 280                 285
   gtc tac ccc gcc tcg gct gcc act gcc atg ctg cca ctc act cct cag      1149
   Val Tyr Pro Ala Ser Ala Ala Thr Ala Met Leu Pro Leu Thr Pro Gln
       290                 295                 300
   aat cag tac aca cct acc atc ctg ttt tgc ggt ggt agt gtc atg agc      1197
   Asn Gln Tyr Thr Pro Thr Ile Leu Phe Cys Gly Gly Ser Val Met Ser
   305                 310                 315                 320
   gac cag atg tgg ggc aac tac agt ggt ccc ggt ggc aac att ctc ggt      1245
   Asp Gln Met Trp Gly Asn Tyr Ser Gly Pro Gly Gly Asn Ile Leu Gly
                   325                 330                 335
   ctc caa gcc tct gat gac tgc tcg tcc atc aac ccc gag gac aat cag      1293
   Leu Gln Ala Ser Asp Asp Cys Ser Ser Ile Asn Pro Glu Asp Asn Gln
               340                 345                 350
   ggc aac cag atc act gac gct cag tac gtc cag gag ggg cgg ctt ccc      1341
   Gly Asn Gln Ile Thr Asp Ala Gln Tyr Val Gln Glu Gly Arg Leu Pro
           355                 360                 365
   gaa ggt cgt tcc atg gga cag ttc atc cac ctc cct gac ggt acc atg      1389
   Glu Gly Arg Ser Met Gly Gln Phe Ile His Leu Pro Asp Gly Thr Met
       370                 375                 380
   gtc gtc ctc aac ggc gcc aac aag gga act gcc ggc tat tcg aac cag      1437
   Val Val Leu Asn Gly Ala Asn Lys Gly Thr Ala Gly Tyr Ser Asn Gln
   385                 390                 395                 400
   aca tgg aac acc atc cag tac aac ggt cgc acc gtc gtc acc gaa ggt      1485
   Thr Trp Asn Thr Ile Gln Tyr Asn Gly Arg Thr Val Val Thr Glu Gly
                   405                 410                 415
   ctt tcg cag gat ccc act tac gtt ccc gtc atc tat gac ccg tcc aag      1533
   Leu Ser Gln Asp Pro Thr Tyr Val Pro Val Ile Tyr Asp Pro Ser Lys
               420                 425                 430
   ccc aga ggt cag cgt ctc tcc aat gct aat ctc aag cct tcc acc att      1581
   Pro Arg Gly Gln Arg Leu Ser Asn Ala Asn Leu Lys Pro Ser Thr Ile
           435                 440                 445
   gct cgt ctc tac cac tcg agc gct att ttg ctc ccc gat ggt tcc gtc      1629
   Ala Arg Leu Tyr His Ser Ser Ala Ile Leu Leu Pro Asp Gly Ser Val
       450                 455                 460
   atg gtt gca ggt tcc aac ccg cat cag gat gtt gcg ctc gac atg ccc      1677
   Met Val Ala Gly Ser Asn Pro His Gln Asp Val Ala Leu Asp Met Pro
   465                 470                 475                 480
   acc ggc acc acg cct cag gct ttc aac acc acc tac gag gtt gaa aag      1725
   Thr Gly Thr Thr Pro Gln Ala Phe Asn Thr Thr Tyr Glu Val Glu Lys
                   485                 490                 495
   tgg tac cct cct tac tgg gac tcg cca cgc cct tac cca cag ggc gtg      1773
   Trp Tyr Pro Pro Tyr Trp Asp Ser Pro Arg Pro Tyr Pro Gln Gly Val
               500                 505                 510
   ccc aat tcg gtg ctg tac ggc ggc agt cct ttc aac att acc gtc aac      1821
   Pro Asn Ser Val Leu Tyr Gly Gly Ser Pro Phe Asn Ile Thr Val Asn
           515                 520                 525
   ggt acc ttt atg ggt gac tcg gcc aac gcc aag gca gcc aac acc aag      1869
   Gly Thr Phe Met Gly Asp Ser Ala Asn Ala Lys Ala Ala Asn Thr Lys
       530                 535                 540
   ttt gcc atc att cgt acc ggt ttc tcc acc cac gcc atg aac atg ggg      1917
   Phe Ala Ile Ile Arg Thr Gly Phe Ser Thr His Ala Met Asn Met Gly
   545                 550                 555                 560
   cag cgc gcc gtc tac ctc gac tac acc tac acc gtt aac gat gac gcc      1965
   Gln Arg Ala Val Tyr Leu Asp Tyr Thr Tyr Thr Val Asn Asp Asp Ala
                   565                 570                 575
   tcg gtc acc tac atg gtc aac cct ttg ccc aac act aag gct atg aac      2013
   Ser Val Thr Tyr Met Val Asn Pro Leu Pro Asn Thr Lys Ala Met Asn
               580                 585                 590
   cgc ctc ttt gtg cct ggc ccg gcc ttc ttc tac gtc acc gtc ggt ggc      2061
   Arg Leu Phe Val Pro Gly Pro Ala Phe Phe Tyr Val Thr Val Gly Gly
           595                 600                 605
   gtg cca agc cat ggc aag ctg atc atg gtg gga act tcc ccc act ggc      2109
   Val Pro Ser His Gly Lys Leu Ile Met Val Gly Thr Ser Pro Thr Gly
       610                 615                 620
   act ggc aac gtc ccc ttc act cct cag ctc ggg tct gca ctc gtc gcg      2157
   Thr Gly Asn Val Pro Phe Thr Pro Gln Leu Gly Ser Ala Leu Val Ala
   625                 630                 635                 640
   ctt ccc cct gct gtc aac agc acc aaa ttc aca gcc tcc ctc ccc aag      2205
   Leu Pro Pro Ala Val Asn Ser Thr Lys Phe Thr Ala Ser Leu Pro Lys
                   645                 650                 655
   gct ggc agc agc tct tcc tcc gag ttt ggc ctc ggc aag atc att ggt      2253
   Ala Gly Ser Ser Ser Ser Ser Glu Phe Gly Leu Gly Lys Ile Ile Gly
               660                 665                 670
   atc gct gtt gct ggc gcc gca gtt ttg gcc ctc att gct ctc ggc tgt      2301
   Ile Ala Val Ala Gly Ala Ala Val Leu Ala Leu Ile Ala Leu Gly Cys
           675                 680                 685
   tgt ctg tgg agg cgc aag ggc agg agc cat agc gac aag gct gcc tcg      2349
   Cys Leu Trp Arg Arg Lys Gly Arg Ser His Ser Asp Lys Ala Ala Ser
       690                 695                 700
   cgc cag tcg gct gcc cct tgg acc agc cgc gac ctt ggc tcg ggt ccc      2397
   Arg Gln Ser Ala Ala Pro Trp Thr Ser Arg Asp Leu Gly Ser Gly Pro
   705                 710                 715                 720
   gag tac aag cgt gtc gac act cct gtc gga tcc atc agc ggt ggt cgc      2445
   Glu Tyr Lys Arg Val Asp Thr Pro Val Gly Ser Ile Ser Gly Gly Arg
                   725                 730                 735
   ttt ggg gcc gcc agg atg gac agc tcg aat acg ttt gag agc tat cgg      2493
   Phe Gly Ala Ala Arg Met Asp Ser Ser Asn Thr Phe Glu Ser Tyr Arg
               740                 745                 750
   ttg cac gac cag gtc agc acg agc gaa agc aag gag gcg att ggc agc      2541
   Leu His Asp Gln Val Ser Thr Ser Glu Ser Lys Glu Ala Ile Gly Ser
           755                 760                 765
   tac tac gac caa cct cgc agc ggc agc cgt ggc ggc tac gct cct agc      2589
   Tyr Tyr Asp Gln Pro Arg Ser Gly Ser Arg Gly Gly Tyr Ala Pro Ser
       770                 775                 780
   ccg ctc gcc tac gac caa cac gga cgt ggc gcc tcg caa ggc cag tac      2637
   Pro Leu Ala Tyr Asp Gln His Gly Arg Gly Ala Ser Gln Gly Gln Tyr
   785                 790                 795                 800
   cac cag caa ggc tgg ggc gaa tac cac gct ggc gat gct ggt gca tac      2685
   His Gln Gln Gly Trp Gly Glu Tyr His Ala Gly Asp Ala Gly Ala Tyr
                   805                 810                 815
   tac gag gac aac act agc agg tac ggc agc ggt ggc ggt gga cac agc      2733
   Tyr Glu Asp Asn Thr Ser Arg Tyr Gly Ser Gly Gly Gly Gly His Ser
               820                 825                 830
   tac gat gat tac tcg cac cag caa tac caa cag cag cat tac tat gac      2781
   Tyr Asp Asp Tyr Ser His Gln Gln Tyr Gln Gln Gln His Tyr Tyr Asp
           835                 840                 845
   agc cca ggt cat cag cac caa gga agc tac tct agt cga cgc              2823
   Ser Pro Gly His Gln His Gln Gly Ser Tyr Ser Ser Arg Arg
       850                 855                 860
   taagccccga aaaacgctgc tggtgctttg tcagtcagtg catgggggat cctctagagt    2883
   cgacctgcag gcatgcaagc ttggcactgg ccgtcgtttt                          2923
<210> 4
   <211> 862
   <212> PRT
   <213> Ustilago maydis
<400> 4
   Met Thr Arg His Leu Ser Ser Ser Ser Arg Arg Ser Ser Leu Ala Lys
   1               5                   10                  15
   Ser Ala Met Thr Leu Ala Thr Leu Ser Leu Ala Leu Thr Ser Cys Ala
               20                  25                  30
   Ser Ala Ala Ser Lys Ala Gly Ser Tyr Glu Val Val Asn Thr Asn Ser
           35                  40                  45
   Leu Ala Ser Ala Met Met Leu Gly Leu Met Asp Glu Asp Asn Val Phe
       50                  55                  60
   Ile Leu Asp Lys Ala Glu Asn Asn Ser Ala Arg Leu Ala Asp Gly Arg
   65                  70                  75                  80
   His Val Trp Gly Ser Phe Tyr Lys Leu Ser Asp Asn Ser Val Thr Gly
                   85                  90                  95
   Thr Ala Val Gln Thr Asn Thr Phe Cys Ala Ser Gly Ala Thr Leu Gly
               100                 105                 110
   Asn Gly Ser Trp Leu Val Ala Gly Gly Asn Gln Ala Val Gly Tyr Gly
           115                 120                 125
   Gly Ala Ala Gln Ala Gln Glu Ile Asn Pro Tyr Ser Asp Phe Asp Gly
       130                 135                 140
   Thr Arg Ala Ile Arg Leu Leu Glu Pro Asn Ser Gln Thr Trp Ile Asp
   145                 150                 155                 160
   Ser Pro Ser Thr Thr Val Ala Gln Val Asn Met Leu Gln Gln Pro Arg
                   165                 170                 175
   Trp Tyr Pro Gly Ile Glu Val Leu Glu Asp Gly Ser Val Ile Phe Ile
               180                 185                 190
   Gly Gly Ala Val Ser Gly Gly Tyr Ile Asn Arg Asn Thr Pro Thr Thr
           195                 200                 205
   Asp Pro Leu Tyr Gln Asn Gly Gly Ala Asn Pro Thr Tyr Glu Tyr Phe
       210                 215                 220
   Pro Ser Lys Thr Thr Gly Asn Leu Pro Ile Cys Asn Phe Met Ala Gln
   225                 230                 235                 240
   Thr Asn Gly Leu Asn Met Tyr Pro His Thr Tyr Leu Met Pro Ser Gly
                   245                 250                 255
   Lys Ile Phe Met Gln Ala Asn Val Ser Thr Ile Leu Trp Asp His Val
               260                 265                 270
   Asn Asn Thr Gln Ile Asp Leu Pro Asp Met Pro Gly Gly Val Val Arg
           275                 280                 285
   Val Tyr Pro Ala Ser Ala Ala Thr Ala Met Leu Pro Leu Thr Pro Gln
       290                 295                 300
   Asn Gln Tyr Thr Pro Thr Ile Leu Phe Cys Gly Gly Ser Val Met Ser
   305                 310                 315                 320
   Asp Gln Met Trp Gly Asn Tyr Ser Gly Pro Gly Gly Asn Ile Leu Gly
                   325                 330                 335
   Leu Gln Ala Ser Asp Asp Cys Ser Ser Ile Asn Pro Glu Asp Asn Gln
               340                 345                 350
   Gly Asn Gln Ile Thr Asp Ala Gln Tyr Val Gln Glu Gly Arg Leu Pro
           355                 360                 365
   Glu Gly Arg Ser Met Gly Gln Phe Ile His Leu Pro Asp Gly Thr Met
       370                 375                 380
   Val Val Leu Asn Gly Ala Asn Lys Gly Thr Ala Gly Tyr Ser Asn Gln
   385                 390                 395                 400
   Thr Trp Asn Thr Ile Gln Tyr Asn Gly Arg Thr Val Val Thr Glu Gly
                   405                 410                 415
   Leu Ser Gln Asp Pro Thr Tyr Val Pro Val Ile Tyr Asp Pro Ser Lys
               420                 425                 430
   Pro Arg Gly Gln Arg Leu Ser Asn Ala Asn Leu Lys Pro Ser Thr Ile
           435                 440                 445
   Ala Arg Leu Tyr His Ser Ser Ala Ile Leu Leu Pro Asp Gly Ser Val
       450                 455                 460
   Met Val Ala Gly Ser Asn Pro His Gln Asp Val Ala Leu Asp Met Pro
   465                 470                 475                 480
   Thr Gly Thr Thr Pro Gln Ala Phe Asn Thr Thr Tyr Glu Val Glu Lys
                   485                 490                 495
   Trp Tyr Pro Pro Tyr Trp Asp Ser Pro Arg Pro Tyr Pro Gln Gly Val
               500                 505                 510
   Pro Asn Ser Val Leu Tyr Gly Gly Ser Pro Phe Asn Ile Thr Val Asn
           515                 520                 525
   Gly Thr Phe Met Gly Asp Ser Ala Asn Ala Lys Ala Ala Asn Thr Lys
       530                 535                 540
   Phe Ala Ile Ile Arg Thr Gly Phe Ser Thr His Ala Met Asn Met Gly
   545                 550                 555                 560
   Gln Arg Ala Val Tyr Leu Asp Tyr Thr Tyr Thr Val Asn Asp Asp Ala
                   565                 570                 575
   Ser Val Thr Tyr Met Val Asn Pro Leu Pro Asn Thr Lys Ala Met Asn
   580 585 590
   Arg Leu Phe Val Pro Gly Pro Ala Phe Phe Tyr Val Thr Val Gly Gly
   595 600 605
   Val Pro Ser His Gly Lys Leu Ile Met Val Gly Thr Ser Pro Thr Gly
   610 615 620
   Thr Gly Asn Val Pro Phe Thr Pro Gln Leu Gly Ser Ala Leu Val Ala
   625 630 635 640
   Leu Pro Pro Ala Val Asn Ser Thr Lys Phe Thr Ala Ser Leu Pro Lys
   645 650 655
   Ala Gly Ser Ser Ser Ser Ser Glu Phe Gly Leu Gly Lys Ile Ile Gly
   660 665 670
   Ile Ala Val Ala Gly Ala Ala Val Leu Ala Leu Ile Ala Leu Gly Cys
   675 680 685
   Cys Leu Trp Arg Arg Lys Gly Arg Ser His Ser Asp Lys Ala Ala Ser
   690 695 700
   Arg Gln Ser Ala Ala Pro Trp Thr Ser Arg Asp Leu Gly Ser Gly Pro
   705 710 715 720
   Glu Tyr Lys Arg Val Asp Thr Pro Val Gly Ser Ile Ser Gly Gly Arg
   725 730 735
   Phe Gly Ala Ala Arg Met Asp Ser Ser Asn Thr Phe Glu Ser Tyr Arg
   740 745 750
   Leu His Asp Gln Val Ser Thr Ser Glu Ser Lys Glu Ala Ile Gly Ser
   755 760 765
   Tyr Tyr Asp Gln Pro Arg Ser Gly Ser Arg Gly Gly Tyr Ala Pro Ser
   770 775 780
   Pro Leu Ala Tyr Asp Gln His Gly Arg Gly Ala Ser Gln Gly Gln Tyr
   785 790 795 800
   His Gln Gln Gly Trp Gly Glu Tyr His Ala Gly Asp Ala Gly Ala Tyr
   805 810 815
   Tyr Glu Asp Asn Thr Ser Arg Tyr Gly Ser Gly Gly Gly Gly His Ser
   820 825 830
   Tyr Asp Asp Tyr Ser His Gln Gln Tyr Gln Gln Gln His Tyr Tyr Asp
           835                 840                 845
   Ser Pro Gly His Gln His Gln Gly Ser Tyr Ser Ser Arg Arg
       850                 855                 860
<210> 5
   <211> 1614
   <212> DNA
   <213> Ustilago maydis
<220>
   <221> CDS
   <222> (1)..(1614)
<400> 5
   atg gag gtg cgt tcc aac acg ttc tgt gcc ggc ggt atg acg ctg ggc      48
   Met Glu Val Arg Ser Asn Thr Phe Cys Ala Gly Gly Met Thr Leu Gly
   1               5                   10                  15
   gac ggc agt tgg ctc gtc acg ggc gga aac aag gcg gtt acc acg aat      96
   Asp Gly Ser Trp Leu Val Thr Gly Gly Asn Lys Ala Val Thr Thr Asn
               20                  25                  30
   ggc gcg act gct aag gca ggt gct gga tac ggc gct tac aat ggc ggt     144
   Gly Ala Thr Ala Lys Ala Gly Ala Gly Tyr Gly Ala Tyr Asn Gly Gly
           35                  40                  45
   aag gca ctg cga ttc ctt agc cct tgc gac aac atg caa tgt cag tgg     192
   Lys Ala Leu Arg Phe Leu Ser Pro Cys Asp Asn Met Gln Cys Gln Trp
       50                  55                  60
   aac gac caa aac agc aat cag ctc aac atg gag agg tgg tat cct acc     240
   Asn Asp Gln Asn Ser Asn Gln Leu Asn Met Glu Arg Trp Tyr Pro Thr
   65                  70                  75                  80
   gta gag cct cta gcc gat gga tcc aat atc atc ctt gga ggc atg cgc     288
   Val Glu Pro Leu Ala Asp Gly Ser Asn Ile Ile Leu Gly Gly Met Arg
                   85                  90                  95
   gac ggt ggc ttt gtt cca agc cag ggc tct aat gtt cct act tac gag     336
   Asp Gly Gly Phe Val Pro Ser Gln Gly Ser Asn Val Pro Thr Tyr Glu
               100                 105                 110
   ttc tac cct cct aag agt ggc gga gct agt att aat ttg cca atc ctg     384
   Phe Tyr Pro Pro Lys Ser Gly Gly Ala Ser Ile Asn Leu Pro Ile Leu
           115                 120                 125
   caa cgt act gta ccc ctc tca ctc tac ccg atc gcg tat ctc atg tcg     432
   Gln Arg Thr Val Pro Leu Ser Leu Tyr Pro Ile Ala Tyr Leu Met Ser
       130                 135                 140
   tcc ggt gag gtg ttt atc caa gcc gga agg gag gcg atc ctt tgg aat     480
   Ser Gly Glu Val Phe Ile Gln Ala Gly Arg Glu Ala Ile Leu Trp Asn
   145                 150                 155                 160
   tac gac cag cag agc gag cgc gca ttt gcc aag att cca ggt gct cct       528
   Tyr Asp Gln Gln Ser Glu Arg Ala Phe Ala Lys Ile Pro Gly Ala Pro
                   165                 170                 175
   cgt gtc tat cct gcc tct ggt ggc tcg gct atg ctt cct cta act ccg       576
   Arg Val Tyr Pro Ala Ser Gly Gly Ser Ala Met Leu Pro Leu Thr Pro
               180                 185                 190
   gca gac gat tac aag gag acc atc ctc ttc tgc ggt ggt acg agc ttg       624
   Ala Asp Asp Tyr Lys Glu Thr Ile Leu Phe Cys Gly Gly Thr Ser Leu
           195                 200                 205
   ggc aag gtc tcg aac tgg ggt aac gag ggt gga ccc tcg atc ccc ata       672
   Gly Lys Val Ser Asn Trp Gly Asn Glu Gly Gly Pro Ser Ile Pro Ile
       210                 215                 220
   tct cag gtt ccc gca tcg acg tcg tgc gag cag atc agc cca ttc cag       720
   Ser Gln Val Pro Ala Ser Thr Ser Cys Glu Gln Ile Ser Pro Phe Gln
   225                 230                 235                 240
   ggt gga aac tgg gaa tcg gtc gac gat ttg ccc gag cgt cgt tcc atg       768
   Gly Gly Asn Trp Glu Ser Val Asp Asp Leu Pro Glu Arg Arg Ser Met
                   245                 250                 255
   ggt caa ttt atc aac ctg ccc gac ggc acc ctg tgg ttc ggc aac ggt       816
   Gly Gln Phe Ile Asn Leu Pro Asp Gly Thr Leu Trp Phe Gly Asn Gly
               260                 265                 270
   gtc acc act ggc gtt gct ggt tac agc acc gac ccc aac tct gtc ggc       864
   Val Thr Thr Gly Val Ala Gly Tyr Ser Thr Asp Pro Asn Ser Val Gly
           275                 280                 285
   aaa ccg gtg ggc gag tcg tat ggc gac aac ccg tcg tac cag cct ctc       912
   Lys Pro Val Gly Glu Ser Tyr Gly Asp Asn Pro Ser Tyr Gln Pro Leu
       290                 295                 300
   gta tac gac ccc aag gca agc cga ggc aac cga tgg aag cgc gtc gga       960
   Val Tyr Asp Pro Lys Ala Ser Arg Gly Asn Arg Trp Lys Arg Val Gly
   305                 310                 315                 320
   agc acc aac att ggt cga ctc tat cat tcg tct gct acg ctg ctt ccg      1008
   Ser Thr Asn Ile Gly Arg Leu Tyr His Ser Ser Ala Thr Leu Leu Pro
                   325                 330                 335
   gat tcg tct atc ctc gtt gct ggt tcc aac cct aat gct gac gtc aac      1056
   Asp Ser Ser Ile Leu Val Ala Gly Ser Asn Pro Asn Ala Asp Val Asn
               340                 345                 350
   cac cat gtc aag tgg aag acg gaa tac cgc att gaa cga tgg tac cca      1104
   His His Val Lys Trp Lys Thr Glu Tyr Arg Ile Glu Arg Trp Tyr Pro
           355                 360                 365
   gac ttc tac gat cag cct cgg ccc tcg aac gac ggt ctc cct agc tct      1152
   Asp Phe Tyr Asp Gln Pro Arg Pro Ser Asn Asp Gly Leu Pro Ser Ser
       370                 375                 380
   ttc tcg tac ggc ggt caa ggc ttt acc atc agg ctc agt tct gca gca      1200
   Phe Ser Tyr Gly Gly Gln Gly Phe Thr Ile Arg Leu Ser Ser Ala Ala
   385                 390                 395                 400
   cag gcg cag aag gcc aag gtg gtc ctg att cga act gga ttt tcc acg      1248
   Gln Ala Gln Lys Ala Lys Val Val Leu Ile Arg Thr Gly Phe Ser Thr
                   405                 410                 415
   cat ggc atg aat atg ggt caa cgc atg atc gag ctc aag tcg aca cat      1296
   His Gly Met Asn Met Gly Gln Arg Met Ile Glu Leu Lys Ser Thr His
               420                 425                 430
   cgg ggc agc aag ctc tac gta gcg cag ctt cca ccc aat ccg aac ctg      1344
   Arg Gly Ser Lys Leu Tyr Val Ala Gln Leu Pro Pro Asn Pro Asn Leu
           435                 440                 445
   ttt gct ccc ggt cct gcg ctc gcg ttc gtt gta gtc gat ggc gtt ccg      1392
   Phe Ala Pro Gly Pro Ala Leu Ala Phe Val Val Val Asp Gly Val Pro
       450                 455                 460
   agt caa gga aag atg gtc atg gtg ggc aac gga aag atc ggc gag cag      1440
   Ser Gln Gly Lys Met Val Met Val Gly Asn Gly Lys Ile Gly Glu Gln
   465                 470                 475                 480
   cct gtc gat gca gag agc gtg ctg ccc ggc tcg acc gcc ccg atg aac      1488
   Pro Val Asp Ala Glu Ser Val Leu Pro Gly Ser Thr Ala Pro Met Asn
                   485                 490                 495
   gac atg ttt caa aga cga cag aat gcg tcc cag acc gaa cgc gat gtg      1536
   Asp Met Phe Gln Arg Arg Gln Asn Ala Ser Gln Thr Glu Arg Asp Val
               500                 505                 510
   gct tcc agt cac aac caa gtg ctc cac cga agc ggc ttg cat gcc cgt      1584
   Ala Ser Ser His Asn Gln Val Leu His Arg Ser Gly Leu His Ala Arg
           515                 520                 525
   cat caa aag ggt ggc gtc gat cgt tat tga                              1614
   His Gln Lys Gly Gly Val Asp Arg Tyr
       530                 535
<210> 6
   <211> 537
   <212> PRT
   <213> Ustilago maydis
<400> 6
   Met Glu Val Arg Ser Asn Thr Phe Cys Ala Gly Gly Met Thr Leu Gly
   1               5                   10                  15
   Asp Gly Ser Trp Leu Val Thr Gly Gly Asn Lys Ala Val Thr Thr Asn
               20                  25                  30
   Gly Ala Thr Ala Lys Ala Gly Ala Gly Tyr Gly Ala Tyr Asn Gly Gly
           35                  40                  45
   Lys Ala Leu Arg Phe Leu Ser Pro Cys Asp Asn Met Gln Cys Gln Trp
       50                  55                  60
   Asn Asp Gln Asn Ser Asn Gln Leu Asn Met Glu Arg Trp Tyr Pro Thr
   65                  70                  75                  80
   Val Glu Pro Leu Ala Asp Gly Ser Asn Ile Ile Leu Gly Gly Met Arg
                   85                  90                  95
   Asp Gly Gly Phe Val Pro Ser Gln Gly Ser Asn Val Pro Thr Tyr Glu
               100                 105                 110
   Phe Tyr Pro Pro Lys Ser Gly Gly Ala Ser Ile Asn Leu Pro Ile Leu
           115                 120                 125
   Gln Arg Thr Val Pro Leu Ser Leu Tyr Pro Ile Ala Tyr Leu Met Ser
       130                 135                 140
   Ser Gly Glu Val Phe Ile Gln Ala Gly Arg Glu Ala Ile Leu Trp Asn
   145                 150                 155                 160
   Tyr Asp Gln Gln Ser Glu Arg Ala Phe Ala Lys Ile Pro Gly Ala Pro
                   165                 170                 175
   Arg Val Tyr Pro Ala Ser Gly Gly Ser Ala Met Leu Pro Leu Thr Pro
               180                 185                 190
   Ala Asp Asp Tyr Lys Glu Thr Ile Leu Phe Cys Gly Gly Thr Ser Leu
           195                 200                 205
   Gly Lys Val Ser Asn Trp Gly Asn Glu Gly Gly Pro Ser Ile Pro Ile
       210                 215                 220
   Ser Gln Val Pro Ala Ser Thr Ser Cys Glu Gln Ile Ser Pro Phe Gln
   225                 230                 235                 240
   Gly Gly Asn Trp Glu Ser Val Asp Asp Leu Pro Glu Arg Arg Ser Met
                   245                 250                 255
   Gly Gln Phe Ile Asn Leu Pro Asp Gly Thr Leu Trp Phe Gly Asn Gly
               260                 265                 270
   Val Thr Thr Gly Val Ala Gly Tyr Ser Thr Asp Pro Asn Ser Val Gly
           275                 280                 285
   Lys Pro Val Gly Glu Ser Tyr Gly Asp Asn Pro Ser Tyr Gln Pro Leu
       290                 295                 300
   Val Tyr Asp Pro Lys Ala Ser Arg Gly Asn Arg Trp Lys Arg Val Gly
   305                 310                 315                 320
   Ser Thr Asn Ile Gly Arg Leu Tyr His Ser Ser Ala Thr Leu Leu Pro
                   325                 330                 335
   Asp Ser Ser Ile Leu Val Ala Gly Ser Asn Pro Asn Ala Asp Val Asn
               340                 345                 350
   His His Val Lys Trp Lys Thr Glu Tyr Arg Ile Glu Arg Trp Tyr Pro
           355                 360                 365
   Asp Phe Tyr Asp Gln Pro Arg Pro Ser Asn Asp Gly Leu Pro Ser Ser
       370                 375                 380
   Phe Ser Tyr Gly Gly Gln Gly Phe Thr Ile Arg Leu Ser Ser Ala Ala
   385                 390                 395                 400
   Gln Ala Gln Lys Ala Lys Val Val Leu Ile Arg Thr Gly Phe Ser Thr
                   405                 410                 415
   His Gly Met Asn Met Gly Gln Arg Met Ile Glu Leu Lys Ser Thr His
               420                 425                 430
   Arg Gly Ser Lys Leu Tyr Val Ala Gln Leu Pro Pro Asn Pro Asn Leu
           435                 440                 445
   Phe Ala Pro Gly Pro Ala Leu Ala Phe Val Val Val Asp Gly Val Pro
       450                 455                 460
   Ser Gln Gly Lys Met Val Met Val Gly Asn Gly Lys Ile Gly Glu Gln
   465                 470                 475                 480
   Pro Val Asp Ala Glu Ser Val Leu Pro Gly Ser Thr Ala Pro Met Asn
                   485                 490                 495
   Asp Met Phe Gln Arg Arg Gln Asn Ala Ser Gln Thr Glu Arg Asp Val
               500                 505                 510
   Ala Ser Ser His Asn Gln Val Leu His Arg Ser Gly Leu His Ala Arg
           515                 520                 525
   His Gln Lys Gly Gly Val Asp Arg Tyr
       530                 535
<210> 7
   <211> 1902
   <212> DNA
   <213> Ustilago maydis
<220>
   <221> CDS
   <222> (1)..(1902)
<400> 7
   atg gct gca tcg tcc atg gcg gct aca cca gga gga agc gag atc gtc      48
   Met Ala Ala Ser Ser Met Ala Ala Thr Pro Gly Gly Ser Glu Ile Val
   1               5                   10                  15
   ggc tcg tcc gcc gtc tca ggc atg atg ctc ttc aac agc gcc cca ggc      96
   Gly Ser Ser Ala Val Ser Gly Met Met Leu Phe Asn Ser Ala Pro Gly
               20                  25                  30
   aaa gtc atc atc ctc gac aag acc gaa ggc aat gca gcc cgc atc aac      144
   Lys Val Ile Ile Leu Asp Lys Thr Glu Gly Asn Ala Ala Arg Ile Asn
           35                  40                  45
   ggc cat cct gct tgg gga gag gag tgg gac acc gag gct cgc acc agt      192
   Gly His Pro Ala Trp Gly Glu Glu Trp Asp Thr Glu Ala Arg Thr Ser
       50                  55                  60
   cgt ctg atg aac gtc gtc acc aac acg ttt tgt gca ggc ggt atg tcg      240
   Arg Leu Met Asn Val Val Thr Asn Thr Phe Cys Ala Gly Gly Met Ser
   65                  70                  75                  80
   ctc ggc aac ggc acc tgg gct gtc ttt gga ggc aat gag aac gtc ggg      288
   Leu Gly Asn Gly Thr Trp Ala Val Phe Gly Gly Asn Glu Asn Val Gly
                   85                  90                  95
   ccc gga ggc aac tcg acc acc cca cgt ttc agc acc aca gcg cct tac      336
   Pro Gly Gly Asn Ser Thr Thr Pro Arg Phe Ser Thr Thr Ala Pro Tyr
               100                 105                 110
   tat gat ggc gat gga ggc gct gct gct cgt ttc tac act ccc aat tct      384
   Tyr Asp Gly Asp Gly Gly Ala Ala Ala Arg Phe Tyr Thr Pro Asn Ser
           115                 120                 125
   cag ggc acc tcc gat tgg gat gat ggt aac cac tac atg cag agg cgc      432
   Gln Gly Thr Ser Asp Trp Asp Asp Gly Asn His Tyr Met Gln Arg Arg
       130                 135                 140
   aga tgg tat cca act gtc gaa gct ctc ggt gat ggc acg ctc tgg ata      480
   Arg Trp Tyr Pro Thr Val Glu Ala Leu Gly Asp Gly Thr Leu Trp Ile
   145                 150                 155                 160
   gga ggc ggt gaa gac tat gga ggt tac gtt gca gac gaa gga cag aac      528
   Gly Gly Gly Glu Asp Tyr Gly Gly Tyr Val Ala Asp Glu Gly Gln Asn
                   165                 170                 175
   caa ccc aac ttt gag tac tgg ccg cca aga ggc gcc gcc atc aac atg      576
   Gln Pro Asn Phe Glu Tyr Trp Pro Pro Arg Gly Ala Ala Ile Asn Met
               180                 185                 190
   gac ttt ctt acc cag act ttg cca atg aac ctg tat cct ttg gcg tgg      624
   Asp Phe Leu Thr Gln Thr Leu Pro Met Asn Leu Tyr Pro Leu Ala Trp
           195                 200                 205
   ctc atg gca tcc ggt cgc ttg ttt gtc cag gca ggg cag gat gcg atc      672
   Leu Met Ala Ser Gly Arg Leu Phe Val Gln Ala Gly Gln Asp Ala Ile
       210                 215                 220
   ctg tac gac ttg gag agc aat tcg gtt gcc aaa ggt ctt ccg tcc acc      720
   Leu Tyr Asp Leu Glu Ser Asn Ser Val Ala Lys Gly Leu Pro Ser Thr
   225                 230                 235                 240
   acg gga ccc atg aaa gtt tac ccg gct tca gcg ggc gta gct atg ttg      768
   Thr Gly Pro Met Lys Val Tyr Pro Ala Ser Ala Gly Val Ala Met Leu
                   245                 250                 255
   cca ctg aca ccc gcg aac aac tat tcg caa gag gtg ctc ttc tgt ggc      816
   Pro Leu Thr Pro Ala Asn Asn Tyr Ser Gln Glu Val Leu Phe Cys Gly
               260                 265                 270
   ggc gtg cag cga ccg ctt aac gaa tgg ggt aac ggt gcg ggt cct ctg      864
   Gly Val Gln Arg Pro Leu Asn Glu Trp Gly Asn Gly Ala Gly Pro Leu
           275                 280                 285
   tac aac cca ctt ccg ttt gcg gca agc aag gtg tgc gag cgc atc acg      912
   Tyr Asn Pro Leu Pro Phe Ala Ala Ser Lys Val Cys Glu Arg Ile Thr
       290                 295                 300
   ccc gag gcc gac aat ccg acg tgg gag cag gac gac gat ctg atc aat      960
   Pro Glu Ala Asp Asn Pro Thr Trp Glu Gln Asp Asp Asp Leu Ile Asn
   305                 310                 315                 320
   ggt cga tct atg ggc act ttt gtc tat ctg ccc gac gga aag ctg tgg     1008
   Gly Arg Ser Met Gly Thr Phe Val Tyr Leu Pro Asp Gly Lys Leu Trp
                   325                 330                 335
   ttt gga caa ggg gtg cgt atg ggt acc ggg ggc tat tca ggt cag cct     1056
   Phe Gly Gln Gly Val Arg Met Gly Thr Gly Gly Tyr Ser Gly Gln Pro
               340                 345                 350
   tac aac aag aac att ggt att tcg ttg ggc gac caa ccg gac ttc cag     1104
   Tyr Asn Lys Asn Ile Gly Ile Ser Leu Gly Asp Gln Pro Asp Phe Gln
           355                 360                 365
   ccg atg ctc tac gat cct tca gcg gcg aag ggc tcg cgt ttt tcg aca     1152
   Pro Met Leu Tyr Asp Pro Ser Ala Ala Lys Gly Ser Arg Phe Ser Thr
       370                 375                 380
   act ggc cta gcg cag atg cag gtg caa agg atg tac cat tcg acc gcc     1200
   Thr Gly Leu Ala Gln Met Gln Val Gln Arg Met Tyr His Ser Thr Ala
   385                 390                 395                 400
   atc ttg ctc gag gac ggc tcc gtg ctc act tcc ggc tcc aac cct aac     1248
   Ile Leu Leu Glu Asp Gly Ser Val Leu Thr Ser Gly Ser Asn Pro Asn
                   405                 410                 415
   gcc gac gtt tcg ctt agt aac gca gcc aac tac acc aac acc gag tac     1296
   Ala Asp Val Ser Leu Ser Asn Ala Ala Asn Tyr Thr Asn Thr Glu Tyr
               420                 425                 430
   cgt ctg gag cag tgg tac ccg ttg tgg tac aac gag ccc agg cct acg     1344
   Arg Leu Glu Gln Trp Tyr Pro Leu Trp Tyr Asn Glu Pro Arg Pro Thr
           435                 440                 445
   cag ccc aac gtc act cag att gct tac ggt ggt ggt tcc ttt gac gtg     1392
   Gln Pro Asn Val Thr Gln Ile Ala Tyr Gly Gly Gly Ser Phe Asp Val
       450                 455                 460
   ccg ctc tct gaa tcg gac ctc tcg aac aac att acc aac atc aag aca      1440
   Pro Leu Ser Glu Ser Asp Leu Ser Asn Asn Ile Thr Asn Ile Lys Thr
   465                 470                 475                 480
   gcc aag atg gtt att att cgg tcc gga ttc gcg aca cac ggt gtc aac      1488
   Ala Lys Met Val Ile Ile Arg Ser Gly Phe Ala Thr His Gly Val Asn
                   485                 490                 495
   ttt gga cag cgc tac ctc gag ctc aat tcg acc tac act gcc ttt cag      1536
   Phe Gly Gln Arg Tyr Leu Glu Leu Asn Ser Thr Tyr Thr Ala Phe Gln
               500                 505                 510
   aat ggc agc gtt gga ggc acg ctg cac gtg tcc aac atg ccg cct aac      1584
   Asn Gly Ser Val Gly Gly Thr Leu His Val Ser Asn Met Pro Pro Asn
           515                 520                 525
   gct aac ctt ttc cag cct ggg ccg gcc atg gca ttt ttg gta atc aac      1632
   Ala Asn Leu Phe Gln Pro Gly Pro Ala Met Ala Phe Leu Val Ile Asn
       530                 535                 540
   ggt gtg cct tcc cac ggt cag cac gta atg atc ggc act ggc cag ctg      1680
   Gly Val Pro Ser His Gly Gln His Val Met Ile Gly Thr Gly Gln Leu
   545                 550                 555                 560
   ggc gac cag aat gtg atg gct tcg acg gtg ctt cct gcc tca cag gat      1728
   Gly Asp Gln Asn Val Met Ala Ser Thr Val Leu Pro Ala Ser Gln Asp
                   565                 570                 575
   cca cca gca ccg aga acg ggt agt agt gga tct ggc tcg aaa gga tcc      1776
   Pro Pro Ala Pro Arg Thr Gly Ser Ser Gly Ser Gly Ser Lys Gly Ser
               580                 585                 590
   aac ggc tcg aat gga tcc aac ggt act ctg aag gac tcg ccc aat ggt      1824
   Asn Gly Ser Asn Gly Ser Asn Gly Thr Leu Lys Asp Ser Pro Asn Gly
           595                 600                 605
   gcc gtt acc ctg tcg aca ggt ctc tgt gcc agt gta tcc ttt gct gca      1872
   Ala Val Thr Leu Ser Thr Gly Leu Cys Ala Ser Val Ser Phe Ala Ala
       610                 615                 620
   gtg ctg acg gcc ttc gcc ctg ttt gct tga                              1902
   Val Leu Thr Ala Phe Ala Leu Phe Ala
   625                 630
<210> 8
   <211> 633
   <212> PRT
   <213> Ustilago maydis
<400> 8
   Met Ala Ala Ser Ser Met Ala Ala Thr Pro Gly Gly Ser Glu Ile Val
   1               5                   10                  15
   Gly Ser Ser Ala Val Ser Gly Met Met Leu Phe Asn Ser Ala Pro Gly
   20 25 30
   Lys Val Ile Ile Leu Asp Lys Thr Glu Gly Asn Ala Ala Arg Ile Asn
   35 40 45
   Gly His Pro Ala Trp Gly Glu Glu Trp Asp Thr Glu Ala Arg Thr Ser
   50 55 60
   Arg Leu Met Asn Val Val Thr Asn Thr Phe Cys Ala Gly Gly Met Ser
   65 70 75 80
   Leu Gly Asn Gly Thr Trp Ala Val Phe Gly Gly Asn Glu Asn Val Gly
   85 90 95
   Pro Gly Gly Asn Ser Thr Thr Pro Arg Phe Ser Thr Thr Ala Pro Tyr
   100 105 110
   Tyr Asp Gly Asp Gly Gly Ala Ala Ala Arg Phe Tyr Thr Pro Asn Ser
   115 120 125
   Gln Gly Thr Ser Asp Trp Asp Asp Gly Asn His Tyr Met Gln Arg Arg
   130 135 140
   Arg Trp Tyr Pro Thr Val Glu Ala Leu Gly Asp Gly Thr Leu Trp Ile
   145 150 155 160
   Gly Gly Gly Glu Asp Tyr Gly Gly Tyr Val Ala Asp Glu Gly Gln Asn
   165 170 175
   Gln Pro Asn Phe Glu Tyr Trp Pro Pro Arg Gly Ala Ala Ile Asn Met
   180 185 190
   Asp Phe Leu Thr Gln Thr Leu Pro Met Asn Leu Tyr Pro Leu Ala Trp
   195 200 205
   Leu Met Ala Ser Gly Arg Leu Phe Val Gln Ala Gly Gln Asp Ala Ile
   210 215 220
   Leu Tyr Asp Leu Glu Ser Asn Ser Val Ala Lys Gly Leu Pro Ser Thr
   225 230 235 240
   Thr Gly Pro Met Lys Val Tyr Pro Ala Ser Ala Gly Val Ala Met Leu
   245 250 255
   Pro Leu Thr Pro Ala Asn Asn Tyr Ser Gln Glu Val Leu Phe Cys Gly
   260 265 270
   Gly Val Gln Arg Pro Leu Asn Glu Trp Gly Asn Gly Ala Gly Pro Leu
   275 280 285
   Tyr Asn Pro Leu Pro Phe Ala Ala Ser Lys Val Cys Glu Arg Ile Thr
   290 295 300
   Pro Glu Ala Asp Asn Pro Thr Trp Glu Gln Asp Asp Asp Leu Ile Asn
   305 310 315 320
   Gly Arg Ser Met Gly Thr Phe Val Tyr Leu Pro Asp Gly Lys Leu Trp
   325 330 335
   Phe Gly Gln Gly Val Arg Met Gly Thr Gly Gly Tyr Ser Gly Gln Pro
   340 345 350
   Tyr Asn Lys Asn Ile Gly Ile Ser Leu Gly Asp Gln Pro Asp Phe Gln
   355 360 365
   Pro Met Leu Tyr Asp Pro Ser Ala Ala Lys Gly Ser Arg Phe Ser Thr
   370 375 380
   Thr Gly Leu Ala Gln Met Gln Val Gln Arg Met Tyr His Ser Thr Ala
   385 390 395 400
   Ile Leu Leu Glu Asp Gly Ser Val Leu Thr Ser Gly Ser Asn Pro Asn
   405 410 415
   Ala Asp Val Ser Leu Ser Asn Ala Ala Asn Tyr Thr Asn Thr Glu Tyr
   420 425 430
   Arg Leu Glu Gln Trp Tyr Pro Leu Trp Tyr Asn Glu Pro Arg Pro Thr
   435 440 445
   Gln Pro Asn Val Thr Gln Ile Ala Tyr Gly Gly Gly Ser Phe Asp Val
   450 455 460
   Pro Leu Ser Glu Ser Asp Leu Ser Asn Asn Ile Thr Asn Ile Lys Thr
   465 470 475 480
   Ala Lys Met Val Ile Ile Arg Ser Gly Phe Ala Thr His Gly Val Asn
   485 490 495
   Phe Gly Gln Arg Tyr Leu Glu Leu Asn Ser Thr Tyr Thr Ala Phe Gln
   500 505 510
   Asn Gly Ser Val Gly Gly Thr Leu His Val Ser Asn Met Pro Pro Asn
   515 520 525
   Ala Asn Leu Phe Gln Pro Gly Pro Ala Met Ala Phe Leu Val Ile Asn
       530                 535                 540
   Gly Val Pro Ser His Gly Gln His Val Met Ile Gly Thr Gly Gln Leu
   545                 550                 555                 560
   Gly Asp Gln Asn Val Met Ala Ser Thr Val Leu Pro Ala Ser Gln Asp
                   565                 570                 575
   Pro Pro Ala Pro Arg Thr Gly Ser Ser Gly Ser Gly Ser Lys Gly Ser
               580                 585                 590
   Asn Gly Ser Asn Gly Ser Asn Gly Thr Leu Lys Asp Ser Pro Asn Gly
           595                 600                 605
   Ala Val Thr Leu Ser Thr Gly Leu Cys Ala Ser Val Ser Phe Ala Ala
       610                 615                 620
   Val Leu Thr Ala Phe Ala Leu Phe Ala
   625                 630
<210> 9
   <211> 1970
   <212> DNA
   <213> Botrytis cinerea
<220>
   <221> CDS
   <222> (1)..(1968)
<400> 9
   atg cta att ttt acc gtt ttt agt tat tgt gga tct aca act gat cac       48
   Met Leu Ile Phe Thr Val Phe Ser Tyr Cys Gly Ser Thr Thr Asp His
   1               5                   10                  15
   tgt ttg gct tcc aat ggt tgc cag aat gga tgc aca ggc tca caa tct       96
   Cys Leu Ala Ser Asn Gly Cys Gln Asn Gly Cys Thr Gly Ser Gln Ser
               20                  25                  30
   tca tca gcc gcc aag act act acc aca gct gca gca ggc agc gca ccc      144
   Ser Ser Ala Ala Lys Thr Thr Thr Thr Ala Ala Ala Gly Ser Ala Pro
           35                  40                  45
   tct tca tct aca act caa gaa cca gtg att gcc cca gtt agt tct aca      192
   Ser Ser Ser Thr Thr Gln Glu Pro Val Ile Ala Pro Val Ser Ser Thr
       50                  55                  60
   ctt acg cct gcc gca gct agc agt gca cca gta act act gat gga tca      240
   Leu Thr Pro Ala Ala Ala Ser Ser Ala Pro Val Thr Thr Asp Gly Ser
   65                  70                  75                  80
   tgt ggt act gcc aat gga ggt acc gtt tgt ggc aat tgg gta aat gga       288
   Cys Gly Thr Ala Asn Gly Gly Thr Val Cys Gly Asn Trp Val Asn Gly
                   85                  90                  95
   aat tgt tgt tcc atg tac ggt ttt tgt ggc agt acc aat gcg cat tgc       336
   Asn Cys Cys Ser Met Tyr Gly Phe Cys Gly Ser Thr Asn Ala His Cys
               100                 105                 110
   ggt gcc gga tgc caa tca gga gat tgt ttg aat gcg cct gcg gtt gca       384
   Gly Ala Gly Cys Gln Ser Gly Asp Cys Leu Asn Ala Pro Ala Val Ala
           115                 120                 125
   gct cct ggt gca agc cct gcc cca gct gcc cca gta gga ggt gcc ttt       432
   Ala Pro Gly Ala Ser Pro Ala Pro Ala Ala Pro Val Gly Gly Ala Phe
       130                 135                 140
   aat atc gtc ggg tcg tct gga gtt cct gct atg cat gct gca ctt atg       480
   Asn Ile Val Gly Ser Ser Gly Val Pro Ala Met His Ala Ala Leu Met
   145                 150                 155                 160
   cca aac ggt cga gtt atg ttc ctc gac aaa tta gag aac tac acc caa       528
   Pro Asn Gly Arg Val Met Phe Leu Asp Lys Leu Glu Asn Tyr Thr Gln
                   165                 170                 175
   ttg aaa ttg cca aat gga tac tac gcc atg tct tca gaa tac gac cca       576
   Leu Lys Leu Pro Asn Gly Tyr Tyr Ala Met Ser Ser Glu Tyr Asp Pro
               180                 185                 190
   gcc act aac gca gtc gcc act cct tta gct tac aaa aca aat gcg ttt       624
   Ala Thr Asn Ala Val Ala Thr Pro Leu Ala Tyr Lys Thr Asn Ala Phe
           195                 200                 205
   tgt tcc gga ggc act ttc ctt gct gat gga cgt gtt gtt tct ctt gga       672
   Cys Ser Gly Gly Thr Phe Leu Ala Asp Gly Arg Val Val Ser Leu Gly
       210                 215                 220
   ggc aac gcg cct tta gat tgg ctc gat cca aac att ggg gat gga ttt       720
   Gly Asn Ala Pro Leu Asp Trp Leu Asp Pro Asn Ile Gly Asp Gly Phe
   225                 230                 235                 240
   gac gcc att aga tat ctt gaa cga tca tct acc gat gct agc ctc aat       768
   Asp Ala Ile Arg Tyr Leu Glu Arg Ser Ser Thr Asp Ala Ser Leu Asn
                   245                 250                 255
   gga aaa gac tgg agt gaa cca ggt aac aag ctc gcg agt gct cgt tgg       816
   Gly Lys Asp Trp Ser Glu Pro Gly Asn Lys Leu Ala Ser Ala Arg Trp
               260                 265                 270
   tat gct act gct caa act atg ggt gat gga acc att ttg gtc gct ttt       864
   Tyr Ala Thr Ala Gln Thr Met Gly Asp Gly Thr Ile Leu Val Ala Phe
           275                 280                 285
   gga agt ttg aac ggc ctc gat ccg act gtc aaa acg aac aac aat cct       912
   Gly Ser Leu Asn Gly Leu Asp Pro Thr Val Lys Thr Asn Asn Asn Pro
       290                 295                 300
   aca tac gag att ttc agt gct acc gct gtg tcg caa ggt aag aac att       960
   Thr Tyr Glu Ile Phe Ser Ala Thr Ala Val Ser Gln Gly Lys Asn Ile
   305                 310                 315                 320
   gac atg gaa att ttg gag aaa aat cag cca tat tat atg tat cct ttt      1008
   Asp Met Glu Ile Leu Glu Lys Asn Gln Pro Tyr Tyr Met Tyr Pro Phe
                   325                 330                 335
   gtt cat ctc ctc aat ggt gga aat ttg ttc gtc ttc gtt tcc aag tct     1056
   Val His Leu Leu Asn Gly Gly Asn Leu Phe Val Phe Val Ser Lys Ser
               340                 345                 350
   tcc caa gta ctc aat gtc ggt acc aac act atc gtc aag gaa tta cct     1104
   Ser Gln Val Leu Asn Val Gly Thr Asn Thr Ile Val Lys Glu Leu Pro
           355                 360                 365
   gaa ctt gct gga gac tat cgc aca tat ccc aac act ggt gga agt gtt     1152
   Glu Leu Ala Gly Asp Tyr Arg Thr Tyr Pro Asn Thr Gly Gly Ser Val
       370                 375                 380
   tta ctc cct ttg tca agc gca aac aaa tgg aac cct gat atc atc atc     1200
   Leu Leu Pro Leu Ser Ser Ala Asn Lys Trp Asn Pro Asp Ile Ile Ile
   385                 390                 395                 400
   tgc ggg gga ggt gca tat caa gat att acc agt cca aca gag cca agt     1248
   Cys Gly Gly Gly Ala Tyr Gln Asp Ile Thr Ser Pro Thr Glu Pro Ser
                   405                 410                 415
   tgt gga aga atc cag cca ttg agt gca aac ccc aca tgg gag ttg gac     1296
   Cys Gly Arg Ile Gln Pro Leu Ser Ala Asn Pro Thr Trp Glu Leu Asp
               420                 425                 430
   gct atg cct gaa ggc cgt ggt atg gtt gaa gga acc tta ctt cca gat     1344
   Ala Met Pro Glu Gly Arg Gly Met Val Glu Gly Thr Leu Leu Pro Asp
           435                 440                 445
   gga aca gtt gtc tgg ctt aat gga ggg aac ttg ggt gct caa gga ttt     1392
   Gly Thr Val Val Trp Leu Asn Gly Gly Asn Leu Gly Ala Gln Gly Phe
       450                 455                 460
   gga ctt gca aaa gac cca aca ttg gaa gct ctt ctt tac gat cct acg     1440
   Gly Leu Ala Lys Asp Pro Thr Leu Glu Ala Leu Leu Tyr Asp Pro Thr
   465                 470                 475                 480
   aaa gct aag ggt caa aga ttc tca act ctt gca aca tca act atc cca     1488
   Lys Ala Lys Gly Gln Arg Phe Ser Thr Leu Ala Thr Ser Thr Ile Pro
                   485                 490                 495
   cgt ctc tac cat tct gtc tct ctc ctc ctt ctt gac ggt aca cta atg     1536
   Arg Leu Tyr His Ser Val Ser Leu Leu Leu Leu Asp Gly Thr Leu Met
               500                 505                 510
   gtc gct ggc tca aac cct gtc gag atg cca aag ctt caa cca gat gca     1584
   Val Ala Gly Ser Asn Pro Val Glu Met Pro Lys Leu Gln Pro Asp Ala
           515                 520                 525
   gcc gat cca tat gtt acg gag ttc cga gtt gag aac tat gtt cct ccc     1632
   Ala Asp Pro Tyr Val Thr Glu Phe Arg Val Glu Asn Tyr Val Pro Pro
       530                 535                 540
   tat ctc tca ggc gat aat gca aag aag cgt cct act aat gta aaa ttg     1680
   Tyr Leu Ser Gly Asp Asn Ala Lys Lys Arg Pro Thr Asn Val Lys Leu
   545                 550                 555                 560
   tca tca ggt agc ttc aaa gca gat ggt agc aca ctt gat gtc aca ttt     1728
   Ser Ser Gly Ser Phe Lys Ala Asp Gly Ser Thr Leu Asp Val Thr Phe
                   565                 570                 575
   gat tgt cca gct ggc gcg aaa gca gtt act gta act ttg tac cac ggt      1776
   Asp Cys Pro Ala Gly Ala Lys Ala Val Thr Val Thr Leu Tyr His Gly
               580                 585                 590
   gga ttc gtc act cac tct gta cat atg ggt cat cgc atg ctg cac ttg      1824
   Gly Phe Val Thr His Ser Val His Met Gly His Arg Met Leu His Leu
           595                 600                 605
   gat aac aca ggc ttc ggc gct ggt gcc aca cag cag aag ttg act gtt      1872
   Asp Asn Thr Gly Phe Gly Ala Gly Ala Thr Gln Gln Lys Leu Thr Val
       610                 615                 620
   act cga cca cca aac aac aat gtt gca cct cca ggt cca tat gtt gtt      1920
   Thr Arg Pro Pro Asn Asn Asn Val Ala Pro Pro Gly Pro Tyr Val Val
   625                 630                 635                 640
   tac att ctt gta gac ggc att cct gcc atg gga cag ttt gtt acg gtt      1968
   Tyr Ile Leu Val Asp Gly Ile Pro Ala Met Gly Gln Phe Val Thr Val
                   645                 650                 655
   tg                                                                   1970
<210> 10
   <211> 656
   <212> PRT
   <213> Botrytis cinerea
<400> 10
   Met Leu Ile Phe Thr Val Phe Ser Tyr Cys Gly Ser Thr Thr Asp His
   1               5                   10                  15
   Cys Leu Ala Ser Asn Gly Cys Gln Asn Gly Cys Thr Gly Ser Gln Ser
               20                  25                  30
   Ser Ser Ala Ala Lys Thr Thr Thr Thr Ala Ala Ala Gly Ser Ala Pro
           35                  40                  45
   Ser Ser Ser Thr Thr Gln Glu Pro Val Ile Ala Pro Val Ser Ser Thr
       50                  55                  60
   Leu Thr Pro Ala Ala Ala Ser Ser Ala Pro Val Thr Thr Asp Gly Ser
   65                  70                  75                  80
   Cys Gly Thr Ala Asn Gly Gly Thr Val Cys Gly Asn Trp Val Asn Gly
                   85                  90                  95
   Asn Cys Cys Ser Met Tyr Gly Phe Cys Gly Ser Thr Asn Ala His Cys
               100                 105                 110
   Gly Ala Gly Cys Gln Ser Gly Asp Cys Leu Asn Ala Pro Ala Val Ala
   115 120 125
   Ala Pro Gly Ala Ser Pro Ala Pro Ala Ala Pro Val Gly Gly Ala Phe
   130 135 140
   Asn Ile Val Gly Ser Ser Gly Val Pro Ala Met His Ala Ala Leu Met
   145 150 155 160
   Pro Asn Gly Arg Val Met Phe Leu Asp Lys Leu Glu Asn Tyr Thr Gln
   165 170 175
   Leu Lys Leu Pro Asn Gly Tyr Tyr Ala Met Ser Ser Glu Tyr Asp Pro
   180 185 190
   Ala Thr Asn Ala Val Ala Thr Pro Leu Ala Tyr Lys Thr Asn Ala Phe
   195 200 205
   Cys Ser Gly Gly Thr Phe Leu Ala Asp Gly Arg Val Val Ser Leu Gly
   210 215 220
   Gly Asn Ala Pro Leu Asp Trp Leu Asp Pro Asn Ile Gly Asp Gly Phe
   225 230 235 240
   Asp Ala Ile Arg Tyr Leu Glu Arg Ser Ser Thr Asp Ala Ser Leu Asn
   245 250 255
   Gly Lys Asp Trp Ser Glu Pro Gly Asn Lys Leu Ala Ser Ala Arg Trp
   260 265 270
   Tyr Ala Thr Ala Gln Thr Met Gly Asp Gly Thr Ile Leu Val Ala Phe
   275 280 285
   Gly Ser Leu Asn Gly Leu Asp Pro Thr Val Lys Thr Asn Asn Asn Pro
   290 295 300
   Thr Tyr Glu Ile Phe Ser Ala Thr Ala Val Ser Gln Gly Lys Asn Ile
   305 310 315 320
   Asp Met Glu Ile Leu Glu Lys Asn Gln Pro Tyr Tyr Met Tyr Pro Phe
   325 330 335
   Val His Leu Leu Asn Gly Gly Asn Leu Phe Val Phe Val Ser Lys Ser
   340 345 350
   Ser Gln Val Leu Asn Val Gly Thr Asn Thr Ile Val Lys Glu Leu Pro
   355 360 365
   Glu Leu Ala Gly Asp Tyr Arg Thr Tyr Pro Asn Thr Gly Gly Ser Val
   370 375 380
   Leu Leu Pro Leu Ser Ser Ala Asn Lys Trp Asn Pro Asp Ile Ile Ile
   385 390 395 400
   Cys Gly Gly Gly Ala Tyr Gln Asp Ile Thr Ser Pro Thr Glu Pro Ser
   405 410 415
   Cys Gly Arg Ile Gln Pro Leu Ser Ala Asn Pro Thr Trp Glu Leu Asp
   420 425 430
   Ala Met Pro Glu Gly Arg Gly Met Val Glu Gly Thr Leu Leu Pro Asp
   435 440 445
   Gly Thr Val Val Trp Leu Asn Gly Gly Asn Leu Gly Ala Gln Gly Phe
   450 455 460
   Gly Leu Ala Lys Asp Pro Thr Leu Glu Ala Leu Leu Tyr Asp Pro Thr
   465 470 475 480
   Lys Ala Lys Gly Gln Arg Phe Ser Thr Leu Ala Thr Ser Thr Ile Pro
   485 490 495
   Arg Leu Tyr His Ser Val Ser Leu Leu Leu Leu Asp Gly Thr Leu Met
   500 505 510
   Val Ala Gly Ser Asn Pro Val Glu Met Pro Lys Leu Gln Pro Asp Ala
   515 520 525
   Ala Asp Pro Tyr Val Thr Glu Phe Arg Val Glu Asn Tyr Val Pro Pro
   530 535 540
   Tyr Leu Ser Gly Asp Asn Ala Lys Lys Arg Pro Thr Asn Val Lys Leu
   545 550 555 560
   Ser Ser Gly Ser Phe Lys Ala Asp Gly Ser Thr Leu Asp Val Thr Phe
   565 570 575
   Asp Cys Pro Ala Gly Ala Lys Ala Val Thr Val Thr Leu Tyr His Gly
   580 585 590
   Gly Phe Val Thr His Ser Val His Met Gly His Arg Met Leu His Leu
   595 600 605
   Asp Asn Thr Gly Phe Gly Ala Gly Ala Thr Gln Gln Lys Leu Thr Val
   610 615 620
   Thr Arg Pro Pro Asn Asn Asn Val Ala Pro Pro Gly Pro Tyr Val Val
   625                 630                 635                 640
   Tyr Ile Leu Val Asp Gly Ile Pro Ala Met Gly Gln Phe Val Thr Val
                   645                 650                 655
<210> 11
   <211> 2024
   <212> DNA
   <213> Botrytis cinerea
<220>
   <221> CDS
   <222> (1)..(315)
<220>
   <221> CDS
   <222> (370)..(2022)
<400> 11
   atg cta att ttt acc gtt ttt agt tat tgt gga tct aca act gat cac       48
   Met Leu Ile Phe Thr Val Phe Ser Tyr Cys Gly Ser Thr Thr Asp His
   1               5                   10                  15
   tgt ttg gct tcc aat ggt tgc cag aat gga tgc aca ggc tca caa tct       96
   Cys Leu Ala Ser Asn Gly Cys Gln Asn Gly Cys Thr Gly Ser Gln Ser
               20                  25                  30
   tca tca gcc gcc aag act act acc aca gct gca gca ggc agc gca ccc      144
   Ser Ser Ala Ala Lys Thr Thr Thr Thr Ala Ala Ala Gly Ser Ala Pro
           35                  40                  45
   tct tca tct aca act caa gaa cca gtg att gcc cca gtt agt tct aca      192
   Ser Ser Ser Thr Thr Gln Glu Pro Val Ile Ala Pro Val Ser Ser Thr
       50                  55                  60
   ctt acg cct gcc gca gct agc agt gca cca gta act act gat gga tca      240
   Leu Thr Pro Ala Ala Ala Ser Ser Ala Pro Val Thr Thr Asp Gly Ser
   65                  70                  75                  80
   tgt ggt act gcc aat gga ggt acc gtt tgt ggc aat tgg gta aat gga      288
   Cys Gly Thr Ala Asn Gly Gly Thr Val Cys Gly Asn Trp Val Asn Gly
                   85                  90                  95
   aat tgt tgt tcc atg tac ggt ttt tg g taagtgcaat cattcactca           335
   Asn Cys Cys Ser Met Tyr Gly Phe Cys
               100                 105
   cccgcgaatc ttcgataatc taacacaatg tag t ggc agt acc aat gcg cat tgc   390
                                          Gly Ser Thr Asn Ala His Cys
                                                          110
   ggt gcc gga tgc caa tca gga gat tgt ttg aat gcg cct gcg gtt gca        438
   Gly Ala Gly Cys Gln Ser Gly Asp Cys Leu Asn Ala Pro Ala Val Ala
           115                 120                 125
   gct cct ggt gca agc cct gcc cca gct gcc cca gta gga ggt gcc ttt        486
   Ala Pro Gly Ala Ser Pro Ala Pro Ala Ala Pro Val Gly Gly Ala Phe
       130                 135                 140
   aat atc gtc ggg tcg tct gga gtt cct gct atg cat gct gca ctt atg        534
   Asn Ile Val Gly Ser Ser Gly Val Pro Ala Met His Ala Ala Leu Met
   145                 150                 155                 160
   cca aac ggt cga gtt atg ttc ctc gac aaa tta gag aac tac acc caa        582
   Pro Asn Gly Arg Val Met Phe Leu Asp Lys Leu Glu Asn Tyr Thr Gln
                   165                 170                 175
   ttg aaa ttg cca aat gga tac tac gcc atg tct tca gaa tac gac cca        630
   Leu Lys Leu Pro Asn Gly Tyr Tyr Ala Met Ser Ser Glu Tyr Asp Pro
               180                 185                 190
   gcc act aac gca gtc gcc act cct tta gct tac aaa aca aat gcg ttt        678
   Ala Thr Asn Ala Val Ala Thr Pro Leu Ala Tyr Lys Thr Asn Ala Phe
           195                 200                 205
   tgt tcc gga ggc act ttc ctt gct gat gga cgt gtt gtt tct ctt gga        726
   Cys Ser Gly Gly Thr Phe Leu Ala Asp Gly Arg Val Val Ser Leu Gly
       210                 215                 220
   ggc aac gcg cct tta gat tgg ctc gat cca aac att ggg gat gga ttt        774
   Gly Asn Ala Pro Leu Asp Trp Leu Asp Pro Asn Ile Gly Asp Gly Phe
   225                 230                 235                 240
   gac gcc att aga tat ctt gaa cga tca tct acc gat gct agc ctc aat        822
   Asp Ala Ile Arg Tyr Leu Glu Arg Ser Ser Thr Asp Ala Ser Leu Asn
                   245                 250                 255
   gga aaa gac tgg agt gaa cca ggt aac aag ctc gcg agt gct cgt tgg        870
   Gly Lys Asp Trp Ser Glu Pro Gly Asn Lys Leu Ala Ser Ala Arg Trp
               260                 265                 270
   tat gct act gct caa act atg ggt gat gga acc att ttg gtc gct ttt        918
   Tyr Ala Thr Ala Gln Thr Met Gly Asp Gly Thr Ile Leu Val Ala Phe
           275                 280                 285
   gga agt ttg aac ggc ctc gat ccg act gtc aaa acg aac aac aat cct        966
   Gly Ser Leu Asn Gly Leu Asp Pro Thr Val Lys Thr Asn Asn Asn Pro
       290                 295                 300
   aca tac gag att ttc agt gct acc gct gtg tcg caa ggt aag aac att       1014
   Thr Tyr Glu Ile Phe Ser Ala Thr Ala Val Ser Gln Gly Lys Asn Ile
   305                 310                 315                 320
   gac atg gaa att ttg gag aaa aat cag cca tat tat atg tat cct ttt       1062
   Asp Met Glu Ile Leu Glu Lys Asn Gln Pro Tyr Tyr Met Tyr Pro Phe
                   325                 330                 335
   gtt cat ctc ctc aat ggt gga aat ttg ttc gtc ttc gtt tcc aag tct       1110
   Val His Leu Leu Asn Gly Gly Asn Leu Phe Val Phe Val Ser Lys Ser
               340                 345                 350
   tcc caa gta ctc aat gtc ggt acc aac act atc gtc aag gaa tta cct       1158
   Ser Gln Val Leu Asn Val Gly Thr Asn Thr Ile Val Lys Glu Leu Pro
           355                 360                 365
   gaa ctt gct gga gac tat cgc aca tat ccc aac act ggt gga agt gtt      1206
   Glu Leu Ala Gly Asp Tyr Arg Thr Tyr Pro Asn Thr Gly Gly Ser Val
       370                 375                 380
   tta ctc cct ttg tca agc gca aac aaa tgg aac cct gat atc atc atc      1254
   Leu Leu Pro Leu Ser Ser Ala Asn Lys Trp Asn Pro Asp Ile Ile Ile
   385                 390                 395                 400
   tgc ggg gga ggt gca tat caa gat att acc agt cca aca gag cca agt      1302
   Cys Gly Gly Gly Ala Tyr Gln Asp Ile Thr Ser Pro Thr Glu Pro Ser
                   405                 410                 415
   tgt gga aga atc cag cca ttg agt gca aac ccc aca tgg gag ttg gac      1350
   Cys Gly Arg Ile Gln Pro Leu Ser Ala Asn Pro Thr Trp Glu Leu Asp
               420                 425                 430
   gct atg cct gaa ggc cgt ggt atg gtt gaa gga acc tta ctt cca gat      1398
   Ala Met Pro Glu Gly Arg Gly Met Val Glu Gly Thr Leu Leu Pro Asp
           435                 440                 445
   gga aca gtt gtc tgg ctt aat gga ggg aac ttg ggt gct caa gga ttt      1446
   Gly Thr Val Val Trp Leu Asn Gly Gly Asn Leu Gly Ala Gln Gly Phe
       450                 455                 460
   gga ctt gca aaa gac cca aca ttg gaa gct ctt ctt tac gat cct acg      1494
   Gly Leu Ala Lys Asp Pro Thr Leu Glu Ala Leu Leu Tyr Asp Pro Thr
   465                 470                 475                 480
   aaa gct aag ggt caa aga ttc tca act ctt gca aca tca act atc cca      1542
   Lys Ala Lys Gly Gln Arg Phe Ser Thr Leu Ala Thr Ser Thr Ile Pro
                   485                 490                 495
   cgt ctc tac cat tct gtc tct ctc ctc ctt ctt gac ggt aca cta atg      1590
   Arg Leu Tyr His Ser Val Ser Leu Leu Leu Leu Asp Gly Thr Leu Met
               500                 505                 510
   gtc gct ggc tca aac cct gtc gag atg cca aag ctt caa cca gat gca      1638
   Val Ala Gly Ser Asn Pro Val Glu Met Pro Lys Leu Gln Pro Asp Ala
           515                 520                 525
   gcc gat cca tat gtt acg gag ttc cga gtt gag aac tat gtt cct ccc      1686
   Ala Asp Pro Tyr Val Thr Glu Phe Arg Val Glu Asn Tyr Val Pro Pro
       530                 535                 540
   tat ctc tca ggc gat aat gca aag aag cgt cct act aat gta aaa ttg      1734
   Tyr Leu Ser Gly Asp Asn Ala Lys Lys Arg Pro Thr Asn Val Lys Leu
   545                 550                 555                 560
   tca tca ggt agc ttc aaa gca gat ggt agc aca ctt gat gtc aca ttt      1782
   Ser Ser Gly Ser Phe Lys Ala Asp Gly Ser Thr Leu Asp Val Thr Phe
                   565                 570                 575
   gat tgt cca gct ggc gcg aaa gca gtt act gta act ttg tac cac ggt      1830
   Asp Cys Pro Ala Gly Ala Lys Ala Val Thr Val Thr Leu Tyr His Gly
               580                 585                 590
   gga ttc gtc act cac tct gta cat atg ggt cat cgc atg ctg cac ttg      1878
   Gly Phe Val Thr His Ser Val His Met Gly His Arg Met Leu His Leu
           595                 600                 605
   gat aac aca ggc ttc ggc gct ggt gcc aca cag cag aag ttg act gtt      1926
   Asp Asn Thr Gly Phe Gly Ala Gly Ala Thr Gln Gln Lys Leu Thr Val
       610                 615                 620
   act cga cca cca aac aac aat gtt gca cct cca ggt cca tat gtt gtt      1974
   Thr Arg Pro Pro Asn Asn Asn Val Ala Pro Pro Gly Pro Tyr Val Val
   625                 630                 635                 640
   tac att ctt gta gac ggc att cct gcc atg gga cag ttt gtt acg gtt      2022
   Tyr Ile Leu Val Asp Gly Ile Pro Ala Met Gly Gln Phe Val Thr Val
                   645                 650                 655
   tg                                                                   2024
<210> 12
   <211> 656
   <212> PRT
   <213> Botrytis cinerea
<400> 12
   Met Leu Ile Phe Thr Val Phe Ser Tyr Cys Gly Ser Thr Thr Asp His
   1               5                   10                  15
   Cys Leu Ala Ser Asn Gly Cys Gln Asn Gly Cys Thr Gly Ser Gln Ser
               20                  25                  30
   Ser Ser Ala Ala Lys Thr Thr Thr Thr Ala Ala Ala Gly Ser Ala Pro
           35                  40                  45
   Ser Ser Ser Thr Thr Gln Glu Pro Val Ile Ala Pro Val Ser Ser Thr
       50                  55                  60
   Leu Thr Pro Ala Ala Ala Ser Ser Ala Pro Val Thr Thr Asp Gly Ser
   65                  70                  75                  80
   Cys Gly Thr Ala Asn Gly Gly Thr Val Cys Gly Asn Trp Val Asn Gly
                   85                  90                  95
   Asn Cys Cys Ser Met Tyr Gly Phe Cys Gly Ser Thr Asn Ala His Cys
               100                 105                 110
   Gly Ala Gly Cys Gln Ser Gly Asp Cys Leu Asn Ala Pro Ala Val Ala
           115                 120                 125
   Ala Pro Gly Ala Ser Pro Ala Pro Ala Ala Pro Val Gly Gly Ala Phe
       130                 135                 140
   Asn Ile Val Gly Ser Ser Gly Val Pro Ala Met His Ala Ala Leu Met
   145 150 155 160
   Pro Asn Gly Arg Val Met Phe Leu Asp Lys Leu Glu Asn Tyr Thr Gln
   165 170 175
   Leu Lys Leu Pro Asn Gly Tyr Tyr Ala Met Ser Ser Glu Tyr Asp Pro
   180 185 190
   Ala Thr Asn Ala Val Ala Thr Pro Leu Ala Tyr Lys Thr Asn Ala Phe
   195 200 205
   Cys Ser Gly Gly Thr Phe Leu Ala Asp Gly Arg Val Val Ser Leu Gly
   210 215 220
   Gly Asn Ala Pro Leu Asp Trp Leu Asp Pro Asn Ile Gly Asp Gly Phe
   225 230 235 240
   Asp Ala Ile Arg Tyr Leu Glu Arg Ser Ser Thr Asp Ala Ser Leu Asn
   245 250 255
   Gly Lys Asp Trp Ser Glu Pro Gly Asn Lys Leu Ala Ser Ala Arg Trp
   260 265 270
   Tyr Ala Thr Ala Gln Thr Met Gly Asp Gly Thr Ile Leu Val Ala Phe
   275 280 285
   Gly Ser Leu Asn Gly Leu Asp Pro Thr Val Lys Thr Asn Asn Asn Pro
   290 295 300
   Thr Tyr Glu Ile Phe Ser Ala Thr Ala Val Ser Gln Gly Lys Asn Ile
   305 310 315 320
   Asp Met Glu Ile Leu Glu Lys Asn Gln Pro Tyr Tyr Met Tyr Pro Phe
   325 330 335
   Val His Leu Leu Asn Gly Gly Asn Leu Phe Val Phe Val Ser Lys Ser
   340 345 350
   Ser Gln Val Leu Asn Val Gly Thr Asn Thr Ile Val Lys Glu Leu Pro
   355 360 365
   Glu Leu Ala Gly Asp Tyr Arg Thr Tyr Pro Asn Thr Gly Gly Ser Val
   370 375 380
   Leu Leu Pro Leu Ser Ser Ala Asn Lys Trp Asn Pro Asp Ile Ile Ile
   385 390 395 400
   Cys Gly Gly Gly Ala Tyr Gln Asp Ile Thr Ser Pro Thr Glu Pro Ser
                   405                 410                 415
   Cys Gly Arg Ile Gln Pro Leu Ser Ala Asn Pro Thr Trp Glu Leu Asp
               420                 425                 430
   Ala Met Pro Glu Gly Arg Gly Met Val Glu Gly Thr Leu Leu Pro Asp
           435                 440                 445
   Gly Thr Val Val Trp Leu Asn Gly Gly Asn Leu Gly Ala Gln Gly Phe
       450                 455                 460
   Gly Leu Ala Lys Asp Pro Thr Leu Glu Ala Leu Leu Tyr Asp Pro Thr
   465                 470                 475                 480
   Lys Ala Lys Gly Gln Arg Phe Ser Thr Leu Ala Thr Ser Thr Ile Pro
                   485                 490                 495
   Arg Leu Tyr His Ser Val Ser Leu Leu Leu Leu Asp Gly Thr Leu Met
               500                 505                 510
   Val Ala Gly Ser Asn Pro Val Glu Met Pro Lys Leu Gln Pro Asp Ala
           515                 520                 525
   Ala Asp Pro Tyr Val Thr Glu Phe Arg Val Glu Asn Tyr Val Pro Pro
       530                 535                 540
   Tyr Leu Ser Gly Asp Asn Ala Lys Lys Arg Pro Thr Asn Val Lys Leu
   545                 550                 555                 560
   Ser Ser Gly Ser Phe Lys Ala Asp Gly Ser Thr Leu Asp Val Thr Phe
                   565                 570                 575
   Asp Cys Pro Ala Gly Ala Lys Ala Val Thr Val Thr Leu Tyr His Gly
               580                 585                 590
   Gly Phe Val Thr His Ser Val His Met Gly His Arg Met Leu His Leu
           595                 600                 605
   Asp Asn Thr Gly Phe Gly Ala Gly Ala Thr Gln Gln Lys Leu Thr Val
       610                 615                 620
   Thr Arg Pro Pro Asn Asn Asn Val Ala Pro Pro Gly Pro Tyr Val Val
   625                 630                 635                 640
   Tyr Ile Leu Val Asp Gly Ile Pro Ala Met Gly Gln Phe Val Thr Val
                   645                 650                 655

## Beschreibung

Unerwünschtes Pilzwachstum, dass z.B. in der Landwirtschaft jedes Jahr zu beträchtlichen Schäden führt, kann durch die Verwendung von Fungiziden kontrolliert werden. Die Ansprüche an Fungizide sind dabei hinsichtlich ihrer Wirksamkeit, Kosten und vor allem ihrer Umweltverträglichkeit stetig angestiegen. Es existiert deshalb ein Bedarf an neuen Substanzen bzw. Substanzklassen, die zu leistungsfähigen und umweltverträglichen neuen Fungiziden entwickelt werden können. Im Allgemeinen ist es üblich, in Gewächshaustests nach solchen neuen Leitstrukturen zu suchen. Solche Tests sind jedoch arbeitsintensiv und teuer. Die Anzahl der Substanzen, die im Gewächshaus getestet werden können, ist entsprechend begrenzt. Eine Alternative zu solchen Tests ist die Verwendung von so genannten Hochdurchsatzverfahren (HTS = high throughput screening). Dabei werden in einem automatisierten Verfahren eine große Anzahl von Einzelsubstanzen hinsichtlich ihrer Wirkung auf Zellen, individuelle Genprodukte oder Gene getestet. Wird für bestimmte Substanzen eine Wirkung nachgewiesen, so können diese in herkömmlichen Screeningverfahren untersucht und gegebenenfalls weiter entwickelt werden.

Vorteilhafte Angriffspunkte für Fungizide werden oft in essentiellen Biosynthesewegen gesucht. Ideale Fungizide sind weiterhin solche Stoffe, die Genprodukte hemmen, die eine entscheidende Bedeutung bei der Ausprägung der Pathogenität eines Pilzes haben. Ein Beispiel für ein solches Fungizid ist z.B: der Wirkstoff Carpropamid, der die Melaninbiosynthese des Pilzes hemmt und so die Ausbildung intakter Appressorien (Haftorgane) verhindert. Es gibt jedoch nur sehr wenige bekannte Genprodukte, die für Pilze eine solche Rolle spielen. Darüber hinaus sind Fungizide bekannt, die durch die Hemmung entsprechender Biosynthesewege zur Auxotrophie der Zielzellen und in der Folge zum Verlust der Pathogenität führen. So führt z.B. die Inhibition der Adenosin Deaminase bei Zugabe von Ethirimol in *Blumeria graminis* zu einer signifikanten Verminderung der Pathogenität (Hollomon, D.W. 1979).

Der Pilz *Phanerochaete chrysosporium,* der zu den Basidiomyceten zählt, kann unter Mangelbedingungen das Lignin des Holzes abbauen. Dieser Abbau erfolgt enzymatisch durch die Mangan-abhängigen Ligninperoxidasen (MnPs) und Ligninperoxidasen (LiPs). Als Substrat für diese Enzyme dient Wasserstoffperoxid (H₂O₂) (Kersten et al., 1990). Dieses wird von einer Glyoxaloxidase bereitgestellt, die die folgende Reaktion katalysiert:

RCHO + O₂ + H₂O → RCO₂H + H₂O₂

Dabei wird eine Aldehydfunktion unter Reduktion von elementarem Sauerstoff zu Wasserstoffperoxid zur Carbonsäure oxidiert. Das Enzym weist eine große Substratbreite auf, so dass eine Reihe von einfachen Aldehyden, α-Dicarbonyl- und verschiedene α-Hydroxycarbonyl-Verbindungen, wie z.B. HCHO, CH₃CHO, CH₂OHCHO, CHOCHO, CHOCOOH, CH₂OHCOCH₂OH, CHOCHOHCH₂OH oder auch CH₃COCHO als Substrat akzeptiert werden. Zusätzlich werden auch andere Produkte der Umsetzung von Lignin-Modellsubstanzen durch die Lignin-Peroxidase durch die Glyoxaloxidase (Kersten et al., 1995), insbesondere jedoch Glyoxal und Methylglyoxal als Intermediärmetabolite beim Wachstum auf den Hauptkomponenten der Ligninocellulose, umgesetzt (Kersten et al., 1993). Abgesehen von der Fähigkeit des Pilzes *Phanerochaete chrysosporium* mittels der Glyoxaloxidase Lignin abzubauen, ist keine andere Bedeutung des Enzyms für den Pilz bekannt geworden.

Bei der Glyoxaloxidase von *Phanerochaete chrysosporium* handelt es sich um ein Kupfer-Metalloenzym, das eine essentielle Komponente des Lignin-Abbauweges darstellt (Whittaker et al., (1996). Das Enzym wird sekretiert. Die Glyoxaloxidase stellt einerseits Wasserstoffperoxid für Peroxidasen zur Verfügung und setzt andererseits Methylglyoxal und Glyoxal, die im Medium von lignolytischen Kulturen als Sekundärmetaboliten gefunden werden, als Hauptsubstrate um (Kersten et al., 1987).

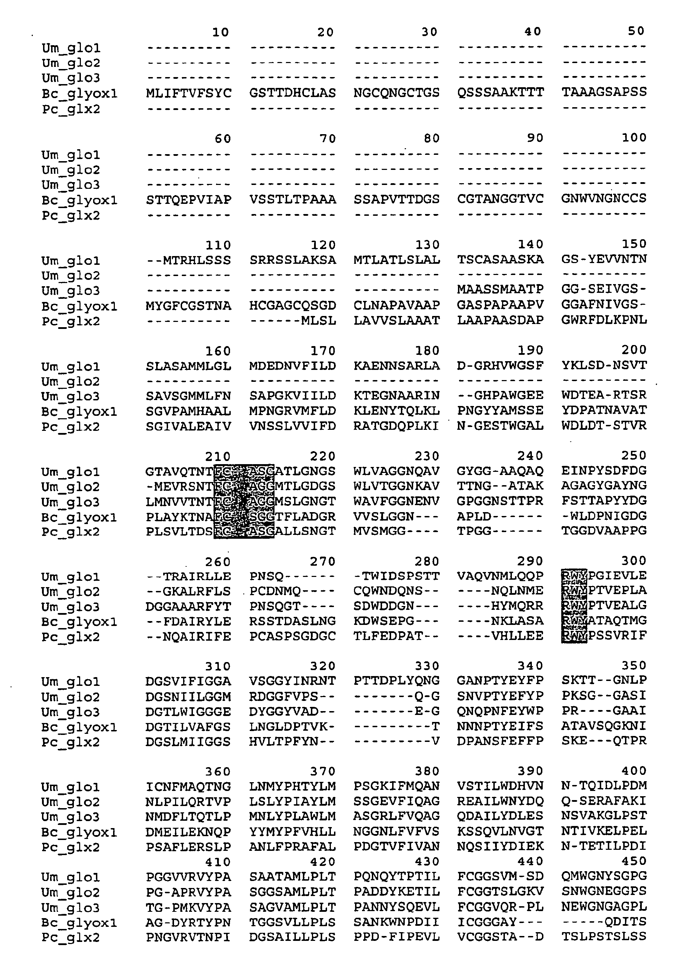

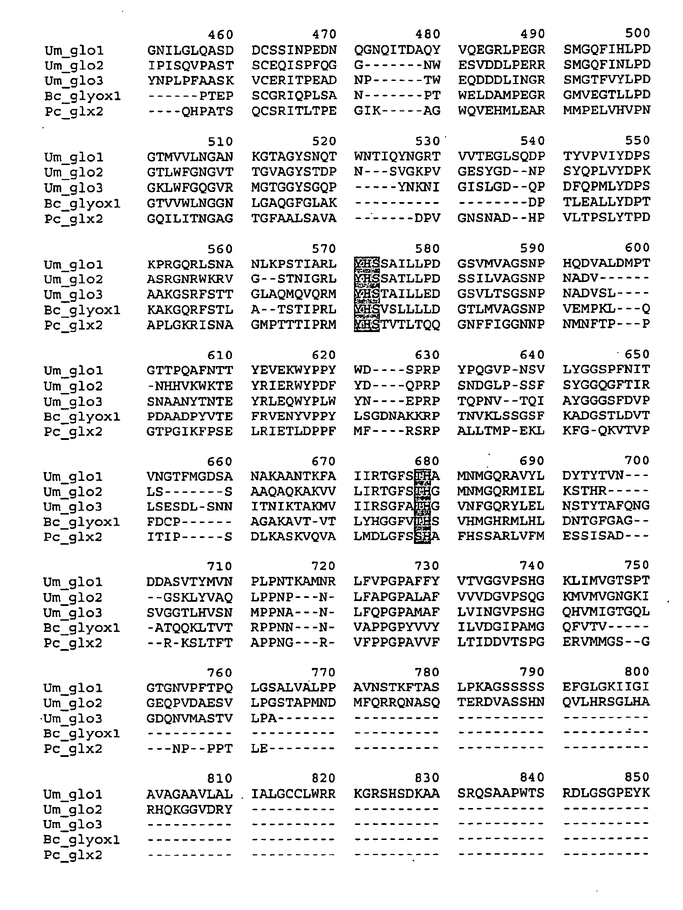

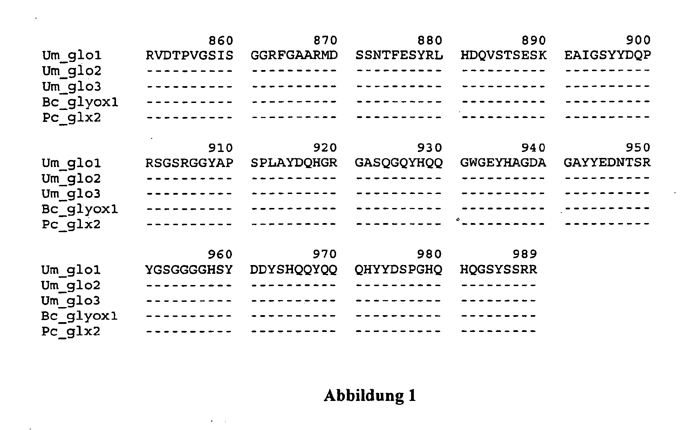

Durch spektroskopische Untersuchungen konnte gezeigt werden, dass in Übereinstimmung mit dem pilzlichen Metalloenzym Galactose-Oxidase im aktiven Zentrum ein ungewöhnliches freies Radikal, das an das Kupfer-Ion gebunden ist, vorliegt. Ein Homologievergleich zwischen der Glyoxaloxidase aus *Phanerochaete chrysosporium* und der erfindungsgemäßen Glyoxaloxidase 1 (Glo 1) aus *U. maydis* (siehe Abbildung 1) bzw. der Glyoxaloxidase aus *B. cinerea* erlaubt eine Zuordnung des *U. maydis* Enzyms zu dieser Enzymklasse der so genannten Radikal-Kupfer-Oxidasen. In dieser Enzymklasse wird das katalytische Motiv von einer Aminosäure-Seitenkette, die das Radikal trägt und mit dem Kupferion verbunden ist, gebildet (Formel I).

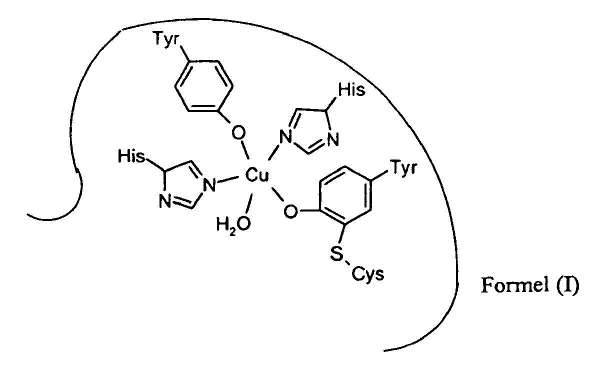

Durch Sequenzvergleich zwischen der Galactoseoxidase und der Glyoxaloxidase aus *Phanerochaete* und anschließende site-directed Mutagenese (Whittaker et al., 1999) konnten schließlich auch die anderen katalytisch wichtigen Aminosäuren zugeordnet werden. Durch EPRspektroskopische Untersuchungen wurden zwei Stickstoff-Liganden in einem Kupfer (II)-Komplex und durch Absorptions- und Raman-Spektroskopie der Tyrosin- und Tyrosin-Cystein-Dimer-Ligand im aktiven Zentrum identifiziert. Bei diesen Aminosäuren handelt es sich um die folgenden Aminosäuren bzw. Positionen:
**Tyrosin-Ligand 1:** Tyr 178 (*U. maydis*) bzw. Tyr 273 (*B. cinerea*),
**Tyrosin-Ligand 2:** Tyr 452 (*U. maydis*) bzw. Tyr 499 (*B. cinerea*),
**Histidin-Ligand 1**: His 453 (*U. maydis*) bzw. His 500 (*B. cinerea*),
**Histidin-Ligand 2:** His 555 (*U. maydis*) bzw. His 597 (*B. cinerea*),
**Cystein-Rest:** Cys 105 (*U. maydis*) bzw. Cys 209 (*B. cinerea*).

Diese für die Cu²⁺-Ionenbindung charakteristischen und in allen erfindungsgemäßen Polypeptiden vorhandenen konservierten Aminosäuren sind damit ein strukturell charakteristisches Merkmal dieser Enzyme. Im Gegensatz zu anderen Radikalenzymen, die Prozesse unter Übertragung eines Elektrons katalysieren, werden durch dieses katalytische Zentrum zwei Elektronen übertragen. Das am besten untersuchte Enzym der Klasse der Radikal-Kupfer-Oxidasen ist die Galactoseoxidase, deren Kristallstruktur auch aufgeklärt ist.

Glyoxaloxidasen aus anderen pilzlichen Organismen als *Phanerochaete chrysosporium* sind bisher noch nicht bekannt geworden.

Im Rahmen der vorliegenden Erfindung wurden nun vollständige cDNA Klone, und die entsprechenden Gene (genomische bzw. cDNA Sequenzen) aus *Ustilago maydis* und aus *Botrytis cinerea* kodierend für Glyoxaloxidase isoliert.

Der Brandpilz *Ustilago maydis,* ein Basidiomycet, befällt Maispflanzen. Die Krankheit kommt in allen Maisanbaugebieten vor, erreicht jedoch nur in trockenen Jahren eine größere Bedeutung. Typische Symptome sind die beulenartigen, faustgroßen Anschwellungen (Brandbeulen), die an allen oberirdischen Pflanzenteilen gebildet werden. Die Beulen sind zuerst von einer weiß-grauen, derben Haut überzogen. Beim Aufreissen der Haut wird eine schwarze, zunächst schmierige, später pulvrige Brandsporenmasse frei. Weitere Arten der Gattung *Ustilago* sind z.B. *U. nuda* (verursacht Gersten- und Weizenflugbrand), *U. nigra* (verursacht Gerstenschwarzbrand), *U. hordei* (verursacht Gerstenhartbrand) und *U. avenae* (verursacht Haferflugbrand).

Der Pilz *Botrytis cinerea,* ein Ascomycet, verursacht die sogenannte "Graufäule". Es handelt sich hier um die Krankheit, die konstant sehr große Schäden in der Landwirtschaft verursacht und deshalb auch massiv bekämpft wird. Sie kann alle Teile der Pflanze befallen, wirkt aber vor allem schädigend auf reifende Beeren. Der weltweit vorhandene Pilz ist omnivor und überdauert saprophytisch auf Holz und Pflanzenresten sowohl als Mycel als auch als Sklerotien. Er dringt durch Wunden ein, kann aber auch nach der Blüte über Blütenreste die Pflanze infizieren. In grünen Beeren bleibt er latent und erst nach Beginn der Reifung kommt es zur explosionsartigen Entwicklung.

Mit Hilfe der oben erwähnten genomischen DNA bzw. Fragmenten davon wurden nun sowohl in *U. maydis* als auch in *B. cinerea* Knock-out Mutanten hergestellt, die überraschenderweise in beiden Fällen, d.h. bei einem Basidiomyceten als auch bei einem Ascomyceten, die beide pflanzenpathogen sind, zur Apathogenität der Pilze führten. Dabei ist zu beobachten, dass in *Ustilago maydis* drei verschiedene Gene, *glo1, glo2* und *glo3,* identifiziert werden können, die alle für eine Glyoxaloxidase kodieren. Im Rahmen der vorliegenden Erfindung wurde festgestellt, dass im Falle des Gens *glo1* (vgl. SEQ ID NO:1 bzw. 3) der vorstehend beschriebene Effekt erzielt wird. Ein Knock-out von *glo2* zeigt dagegen keinen Einfluss auf die Pathogenität des Pilzes. *glo3* wurde wie *glo1* als Mutante in einem Screen auf Apathogenität als Pathogenitätsdeterminante identifiziert. Der Grund für diese unterschiedlichen Phänotypen kann im Expressionsmuster der verschiedenen Enzyme, in deren zellulärer Lokalisation oder auch in der spezifischen Aktivität der Enzyme gesucht werden. Offensichtlich spielt jedoch gerade Glo1 eine entscheidende Rolle für die Pathogenität des Pilzes.

In einem REMI-Mutageneseansatz (restriction enzyme mediated integration, siehe z.B. Kahmann und Basse 1999) konnten bereits morphologisch auffällige Mutanten des Stammes CL13 isoliert werden (M. Bölker und R. Kahmann, nicht publiziert). Die REMI-Mutante #5662 zeichnet sich durch einen flockigen, verfilzten Phänotyp aus. Die Mutante zeigt darüber hinaus eine auffallende Melanisierung.

Im Pathogenitätstest konnte nunmehr keine Infektion von Maispflanzen nachgewiesen werden, d.h. die Mutante ist apathogen. Zur Gewinnung der für die Glyoxaloxidase kodierenden Nukleinsäuren wurden so genannte "plasmid rescue" Experimente durchgeführt.

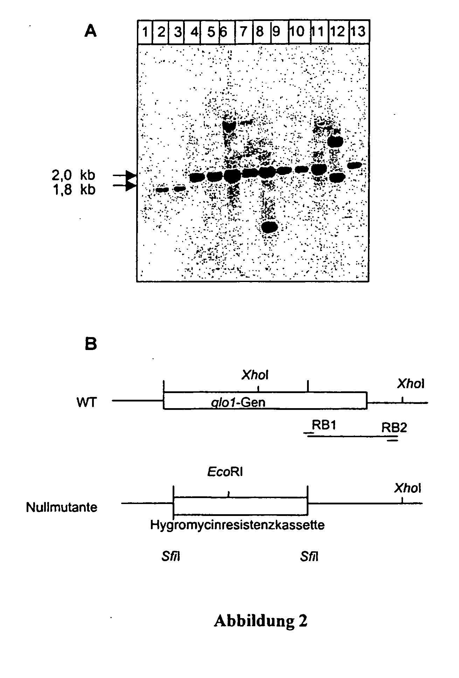

Im Rahmen der vorliegenden Erfindung konnten nun durch ein sogenanntes "plasmid rescue" Experiment (siehe Beispiel 1) die Sequenzen reisoliert werden, die die Insertionsstelle flankieren. Auf diese Weise werden die für die Glyoxaloxidase kodierenden Sequenzen, hier *glo1,* isoliert. Dabei ergab sich nach Sequenzierung, dass die Insertion 770 bp nach dem Startkodon eines putativen ORFs stattgefunden hatte. Dessen abgeleitete Aminosäuresequenz zeigt Ähnlichkeit zur Glyoxaloxidase aus *Phanerochaete chrysosporium.* Das Ustilago Gen wurde *glo1* (Glyoxaloxidase1) genannt. Da die Korrelation einer REMI-Insertion mit dem beobachteten Phänotyp der Mutanten nicht immer gelingt, wurde im Rahmen der vorliegenden Erfindung zusätzlich das *glol-Gen* in den beiden haploiden Stämmen Um518 und Um521 deletiert, um Phänotyp und Gen in eine eindeutige Beziehung zu setzten (siehe Beispiel 2). Zunächst wurde je ein 1151bp und ein 1249bp großes DNA-Fragment, das 5' bzw. 3' des putativen *glo1*-ORFs lag, durch PCR amplifiziert. Anschließend wurden die Fragmente mit dem Restriktionsenzym *Sfi*I geschnitten und so mit der *Sfi*I geschnittenen HygromycinB Kassette (1884 bp Fragment aus pBShhn) ligiert, dass 1931 Nukleotide aus dem ORF des *glol-Gens* deletiert wurden (siehe Abb. 2B sowie Kämper und Schreier, 2001). Die Amplifikation dieser Knock-out Kassette erfolgte ebenfalls durch PCR (siehe Beispiel 2). Bei einer homologen Rekombination wird so der N-terminale Teil von *glo1* durch die HygromycinB-Kassette ersetzt. Die Auswahl der Nullmutanten erfolgte durch Southernanalyse der Transformanten mit einer *glo1*-spezifischen DNA-Sonde (s. Abbildung 2A). Dabei zeigte sich, dass acht von 10 Transformanten das erwartete Restriktionmuster in der Southemanalyse aufwiesen. Die Stämme 518*□glo1*#1, 518*□glo1*#4 bzw. 521*□glo1#*7 und 521*□glo1*#9 wurden für weitere Analysen ausgewählt.

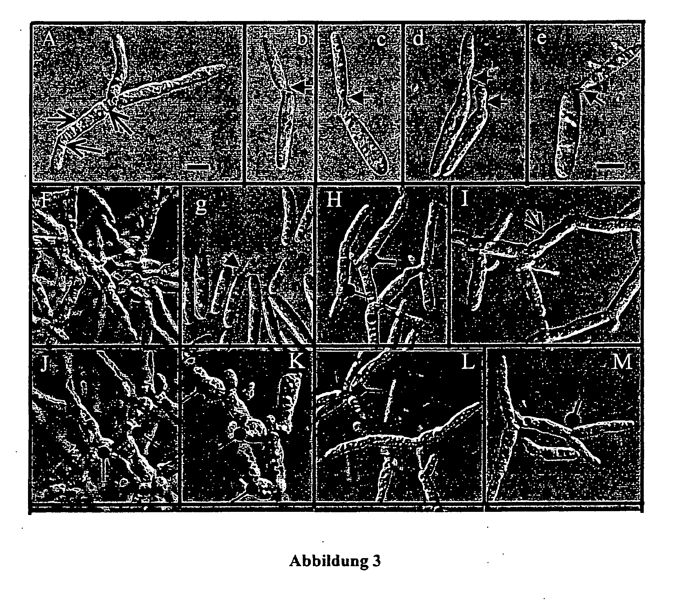

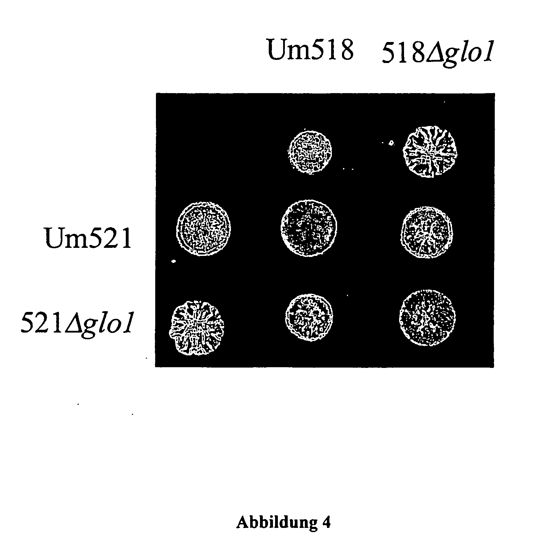

Wie in Abbildung 4 zu erkennen ist, weisen die *glo1*-Nullmutanten einen pleiotropen Morphologiedefekt auf. So zeigt sich bei der Handhabung der *glo1*-Nullmutanten außerdem, dass die Zellen, wenn sie auf Plattenmedien gezogen werden, im Vergleich zu Wildtypstämmen eine wesentlich geringere Adhäsion zueinander aufweisen. Um diesen Phänotyp näher zu charakterisieren, können z.B. mikroskopische Untersuchungen durchgeführt werden. Dazu werden die Zellen auf Objektträger aufgebracht und durch differentielle Interferenzkontrastmikroskopie beobachtet (Abb. 4). Dabei zeigt sich im Vergleich zu Wildtypstämmen, dass die Zellen elongiert sind. Zudem ist eine verstärkte Vakuolisierung zu beobachten. Mutante Zellen sind zudem in der Zytokinese beeinträchtigt und weisen eine verstärkte Septenbildung auf (siehe ebenfalls Abb. 3). Auffällig sind auch Zellen, die eine globuläre Form aufweisen und sich im Zentrum nicht separierter Zellhaufen befinden. Zusammengefasst zeigen sich bei den erfindungsgemäßen Nullmutanten alle Anzeichen eines pleiotropen Morphologiedefekts.

Bemerkenswert ist weiterhin auch, dass Mischungen kompatibler *glo1*-Nullmutanten apathogen sind. Um den Einfluss des *glo1*-Nullallels auf die Pathogenität zu untersuchen, wurden so im Rahmen der vorliegenden Erfindung Pflanzeninfektionen durchgeführt. Dazu wurden je zwei unabhängige kompatible *glol*-Nullmutanten angezogen, gewaschen und gemischt. Anschließend wurden die Mischungen in junge Maispflanzen injiziert. Zum Vergleich wurden Maispflanzen mit Mischungen kompatibler Wildtypstämme (Um518 und Um521) infiziert. Während nach einer Woche im Kontrollexperiment bereits Tumorbildung zu beobachten war, zeigten sich in der Mischung kompatibler Mutanten keinerlei Symptome. Zwei Wochen nach Infektion hatten 97 von 102 infizierten Pflanzen in der Kontrollinfektion Tumore gebildet. Drei weitere Pflanzen zeigten die für Pilzinfektionen typische Anthocyanfärbung. Damit wiesen 100 von 102 infizierten Pflanzen (98%) Pathogenitätssymptome auf (siehe Tabelle I). Bei Infektionen mit Mischungen kompatibler Mutanten konnte weder Tumorbildung noch Anthocyanfärbung beobachtet werden (siehe Tabelle I). Daraus ergibt sich, dass kompatible Nullmutanten von *glo1* nicht in der Lage sind Maispflanzen zu infizieren, d.h. sie weisen einen Pathogenitätsdefekt auf.

**Tabelle I: Mischungen kompatibler glo1-Nullmutanten**

| | Σ Pflanzen | Tumor | Anthocyan | Σ Symtome | Pathogenität (%) |
|---|---|---|---|---|---|
| Um 518 x Um 521 | 102 | 97 | 3 | 100 | 98 |
| 518Δ*glo1*-*1* x 521*Δglo1-7* | 101 | 0 | 0 | 0 | 0 |
| 518*Δglo1-4* x 521*Δglo1-9* | 106 | 0 | 0 | 0 | 0 |

Bemerkenswert ist weiterhin, dass *glo1*-Nullmutanten in ihrem Paarungsverhalten eingeschränkt sind. So ist die Bildung dikaryotischer Filamente in Mischungen kompatibler *glo1-*Mutantenstämme nicht mehr zu beobachten. Bei der Kreuzung von Mutanten mit kompatiblen Wildtypen ist bezüglich der Dikaryonbildung eine Restaktivität bezüglich des Paarungsverhaltens zu erkennen (siehe Abbildung 4), was auf einen Zellfusionsdefekt schließen lässt.

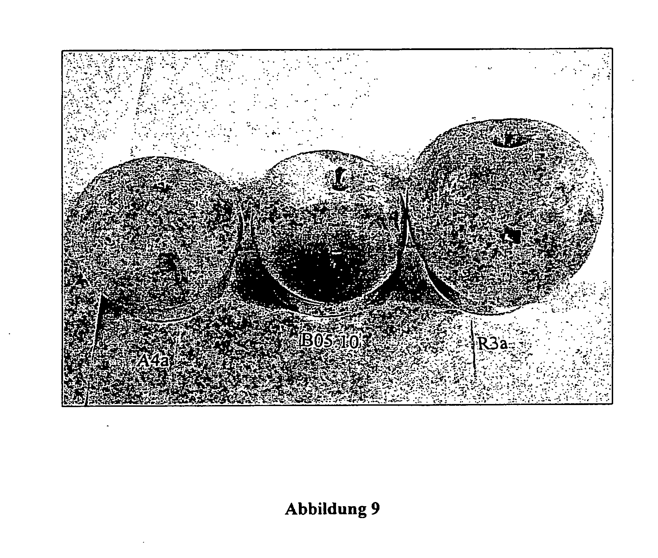

Völlig analoge Ergebnisse ergaben sich bei der Prüfung von entsprechenden Knock-out Mutanten in *B. cinerea.* Auch hier konnte klar gezeigt werden, dass die Disruption des Gens, das für die Glyoxaloxidase kodiert, zu einem Pathogenitätsdefekt bei *B. cinerea* führt (siehe Bsp. 9 und Abb. 9 bis 12).

Aus diesen Ergebnissen konnte nunmehr geschlossen werden, dass die Glyoxaloxidase nicht nur für einen spezifischen Pilz, sondern für pflanzenpathogene Pilze an sich eine besondere Rolle bei der Ausbildung der Pathogenität spielt. Die Bedeutung der Glyoxaloxidase für die Pathogenität, die Lebensfähigkeit im Wirt und den Lebenszyklus der pflanzenpathogenen Pilze wurde damit erstmals erkannt und erstmals als ein optimales Target für die Suche nach neuen, spezifischen Fungiziden identifiziert. Damit wird erstmals die Möglichkeit gegeben, mit Hilfe dieses Targets gegebenenfalls völlig neue Leitstrukturen zu identifizieren. Ausgehend von solchen Verbindungen, die die Glyoxaloxidase inhibieren, können somit neue Fungizide zur Verfügung gestellt werden.

Weiterhin werden hier anhand der genomischen als auch der cDNA-Sequenz und der Angabe von Verfahren zu deren Gewinnung Glyoxaloxidasen aus zwei unterschiedlichen Unterabteilungen pflanzenpathogener Pilze bereitgestellt, die geeignet sind, in Verfahren zur Identifizierung von Fungiziden verwendet zu werden, wobei identifizierte Fungizide dann auch mit Hilfe des zugehörigen Targets, der Glyoxaloxidase, charakterisiert und weiterentwickelt werden können.

Im Rahmen der vorliegenden Erfindung werden damit zum ersten Mal vollständige genomische Sequenzen bzw. die cDNA von Glyoxaloxidasen pathogener Pilze zur Verfügung gestellt und deren Verwendung bzw. die Verwendung des davon kodierten Polypeptids zur Identifizierung von Inhibitoren des Enzyms sowie deren Verwendung als Fungizide beschrieben.

Für die vorliegende Erfindung können die hierin beschriebenen Nukleinsäuren verwendet werden, die für vollständige pilzliche Glyoxaloxidasen kodieren, einschließlich der für die Glyoxaloxidase kodierenden Nukleinsäuresequenzen aus *Phanerochaete chrysosporium* (Kersten et al., 1995), PCGLX1G_1 PRT mit 559 Aminosäuren (zugänglich bei EMBL unter der Accession No. L47286 oder bei SPTREMBL unter der Accession No. Q01772; (Protein ID = AAA87594.1)), sowie PCGLX2G_1 PRT mit 559 Aminosäuren (zugänglich bei EMBL unter der Accession No. L47287 oder bei SPTREMBL unter der Accession No. Q01773 (Protein ID = AAA87595.1)). Die Proteinsequenzen sind identisch, bis auf einen Aminosäure Austausch Lys 308 zu Thr 308. Die Nucleotid-Sequenzen sind zu 98 % identisch.

Mit Hilfe dieser Nukleinsäuren konnten ebenfalls weitere für die Glyoxaloxidase kodierende Nukleinsäuresequenzen aus anderen Pilzen identifiziert werden, die zwar als Ergebnisse im Rahmen von Genomprojekten bereits öffentlich zugänglich waren, jedoch noch keiner Funktion oder biologischen Bedeutung zugeordnet werden konnten. Es handelt sich dabei um Sequenzen aus *Cryptococcus neoformans,* einem humanpathogenen Pilz, der für Cryptococcosis (Cryptococcal meningitis) und Pneumonia verantwortlich gemacht werden kann (siehe CRYNE_cneo 001022. contig 6786 (4064 bp), Homologiebereich: 2704-1393, CRYNE_cneo 001022.contig 7883 (13487 bp); Homologiebereiche: 916-1695, 468-2185, 2100-2345, CRYNE b6f10cnf1; Homologiebereich: 1-564, CRYNE_4_contig 456; Homologiebereich: 930-19, und CRYNE_cneo001022. contig 6828 (4546 bp); Homologiebereich: 4364-3840), aus dem als Brotschimmel bekannten Ascomyceten *Neurospora crassa* (siehe NEUCR_ contig 1887 (supercontig 127); Homologiebereich: 14411-15889) und aus dem pflanzenpathogenen Reisbrennerpilz *Magnaporthe grisea.* Es wurde damit gefunden, dass die Glyoxaloxidase auch in humanpathogenen Pilzen vorkommt. Es ist davon auszugehen, dass das Enzym auch in diesen humanpathogenen Pilzen eine nicht unerhebliche physiologische Rolle spielt und deshalb auch in diesen Pilzen ein interessantes Target für Modulatoren des Enzyms ist bzw. als Wirkort für Antimycotica eine Rolle spielt.

Für die vorliegende Erfindung können insbesondere Nukleinsäuren verwendet werden, die für Glyoxaloxidasen aus pflanzenpathogenen Pilzen kodieren, bevorzugt aus Pilzen der Unterabteilung der Ascomyceten und der Basidiomyceten, wobei die Gattungen *Botrytis* und *Ustilago* besonders bevorzugt sind.

Für die vorliegende Erfindung können ganz besonders bevorzugt Nukleinsäuren verwendet werden, die für Glyoxaloxidasen aus *Ustilago maydis* und *Botrytis cinerea* kodieren.

Für die vorliegende Erfindung können insbesondere bevorzugt die für die Glyoxaloxidasen aus *Ustilago maydis* kodierenden Nukleinsäuren mit der SEQ ID NO:1 bzw. SEQ ID NO:3 sowie SEQ ID NO:5 und SEQ ID NO:7 und die für Glyoxaloxidasen aus *Botrytis cinerea* kodierenden Nukleinsäuren mit der SEQ ID NO:9 bzw. SEQ ID NO:11 sowie die für Polypeptide gemäß SEQ ID NO:2 bzw. SEQ ID NO:4 sowie SEQ ID NO:6, SEQ ID NO:8, SEQ ID NO:10 und SEQ ID NO:12 oder aktive Fragmente davon kodierende Nukleinsäuren verwendet werden.

Bei diesen Nukleinsäuren handelt es sich insbesondere um einzelsträngige oder doppelsträngige Desoxyribonukleinsäuren (DNA) oder Ribonukleinsäuren (RNA). Bevorzugte Ausführungsformen sind Fragmente genomischer DNA, die Introns enthalten können, und cDNAs.

Bevorzugt handelt es sich bei diesen Nukleinsäuren um DNA-Fragmente, die der cDNA phytopathogener Pilze entsprechen.

Besonders bevorzugt umfassen diese Nukleinsäuren eine Sequenz ausgewählt aus
a) einer Sequenz gemäß SEQ ID NO: 1, SEQ ID NO: 3, SEQ ID NO:5, SEQ ID NO:7, SEQ ID NO:9 und SEQ ID NO:11,
b) Sequenzen, die für ein Polypeptid kodieren, welches die Aminosäuresequenz gemäß SEQ ID NO: 2, SEQ ID NO: 4, SEQ ID NO:6, SEQ ID NO: 8, SEQ ID NO:10 oder SEQ ID NO:12 umfasst,
c) Sequenzen, die für ein Polypeptid kodieren, welches die für die Cu²⁺-Koordination geeigneten Aminosäuren Tyrosin 1 und 2, Histidin 1 und 2 und Cystein gemäß Formel (I) umfasst,
d) zumindest 14 Basenpaare lange Teilsequenzen der unter a) bis c) definierten Sequenzen,
e) Sequenzen, welche eine 50 %ige, besonders bevorzugt eine 70 %ige Identität, ganz besonders bevorzugt eine 90 %ige Identität mit den unter a) bis c) definierten Sequenzen aufweisen,
f) Sequenzen, welche zu den unter a) bis c) definierten Sequenzen komplementär sind, und
g) Sequenzen, welche aufgrund der Degeneriertheit des genetischen Codes für dieselbe Aminosäuresequenz kodieren wie die unter a) bis c) definierten Sequenzen.

Eine ganz besonders bevorzugte Ausfiihrungsform dieser Nukleinsäuren stellt ein cDNA-Molekül mit der Sequenz gemäß SEQ ID NO:1 bzw. 3 bzw. mit der Sequenz SEQ ID NO:5 oder SEQ ID NO:7 kodierend für eine Glyoxaloxidase aus *Ustilago maydis* dar.

Eine weitere ganz besonders bevorzugte Ausführungsform dieser Nukleinsäuren stellt ein cDNA-Molekül mit der Sequenz gemäß SEQ ID NO:9 bzw. 11 kodierend für eine Glyoxaloxidase aus *Botrytis cinerea* dar.

Der Ausdruck "vollständige" Glyoxaloxidase wie er hierin verwendet wird, beschreibt die Glyoxaloxidasen, für die eine vollständige kodierende Region einer Transkriptionseinheit beginnend mit dem ATG-Startcodon und umfassend alle informationstragenden Exonbereiche des in den Herkunftsorganismen vorliegenden, für Glyoxaloxidasen kodierenden Genes, sowie die für eine korrekte Termination der Transkription nötigen Signale vorliegen.

Der Ausdruck "aktives Fragment" wie er hierin verwendet wird, beschreibt nicht mehr vollständige Nukleinsäuren kodierend für Glyoxaloxidase, die noch für Polypeptide mit der biologischen Aktivität einer Glyoxaloxidase kodieren, die also die Reaktion

RCHO + O₂ + H₂O → RCO₂H + H₂O₂

katalysieren können. Ob diese biologische Funktion tatsächlich noch vorliegt, kann durch einen Aktivitätstest bestimmt werden, der z.B. auf dem Nachweis von H₂O₂ z.B. durch Ansäuern mit H₂SO₄ und Zugabe von TiOSO₄-Lösung (die Bildung von [TiO₂*aq]SO₄ führt zu einer gelborangen Färbung) beruht. Eine Glyoxaloxidaseaktivität kann auch bei bekannten Glucoseoxidasen beobachtet werden. Diese Aktivität ist im Vergleich zu Glyoxaloxidasen, deren Hauptaktivität die Katalyse der gezeigten Reaktion ist, jedoch deutlich herabgesetzt. Der Ausdruck "biologische Aktivität" soll sich deshalb nicht auf solche Polypeptide wie die Glucoseoxidase erstrecken, deren Hauptaktivität nicht die Katalyse dieser Reaktion ist. "Aktive Fragmente" sind kürzer als die oben beschriebenen vollständigen, für die Glyoxaloxidase kodierenden Nukleinsäuren. Dabei können sowohl an den 3'- und/oder 5'-Enden der Sequenz Nukleinsäuren entfernt worden sein, es können aber auch Teile der Sequenz deletiert, d.h. entfernt worden sein, die die biologische Aktivität der Glyoxaloxidase nicht entscheidend beeinträchtigen. Eine geringere oder gegebenenfalls auch eine erhöhte Aktivität, die aber noch die Charakterisierung bzw. Verwendung des resultierenden Glyoxaloxidasefragments gestattet, wird dabei als ausreichend im Sinne des hier verwendeten Ausdrucks verstanden. Der Ausdruck "aktives Fragment" kann sich ebenso auf die Aminosäuresequenz der Glyoxaloxidase beziehen und gilt dann analog den obigen Ausführungen für solche Polypeptide, die im Vergleich zur oben definierten vollständigen Sequenz bestimmte Teile nicht mehr enthalten, wobei die biologische Aktivität des Enzyms jedoch nicht entscheidend beeinträchtigt ist.

Die bevorzugte Länge dieser Fragmente beträgt 1200 Nukleobasen, bevorzugt 900 Nukleobasen, ganz besonders bevorzugt 300 Nukleobasen, bzw. 400 Aminosäuren, bevorzugt 300 Aminosäuren, ganz besonders bevorzugt 100 Aminosäuren.

Der Ausdruck "Gen", wie er hierin verwendet wird, ist die Bezeichnung für einen Abschnitt aus dem Genom einer Zelle, der für die Synthese einer Polypeptid-Kette verantwortlich ist.

Der Ausdruck "hybridisieren", wie er hierin verwendet wird, beschreibt den Vorgang, bei welchem ein einzelsträngiges Nukleinsäuremolekül mit einem komplementären Strang eine Basenpaarung eingeht. Diese ist vor allem relevant für kurze, die Konsensussequenzen oder andere bekannte Bereiche der hierin beschriebenen Nukleinsäuren überspannenden Bereiche, die vorteilhafterweise für die Durchführung von PCR-Experimenten zur Identifizierung weiterer für Glyoxaloxidasen kodierende Nukleinsäuren verwendet werden. Auf diese Weise können ausgehend von der hierin offenbarten Sequenzinformation beispielsweise DNA-Fragmente von weiteren homologen Genen oder aus anderen Pilzen als *Ustilago maydis* oder *Botrytis cinerea* isoliert werden, welche für Glyoxaloxidasen kodieren, welche dieselben oder ähnliche Eigenschaften wie die Glyoxaloxidasen mit der Aminosäuresequenz gemäß SEQ ID NO:1 bzw. SEQ ID NO:3, SEQ ID NO:5, SEQ ID NO: 7, SEQ ID NO:9 bzw. SEQ ID NO:11 aufweisen.

Der Ausdruck "cDNA", wie er hierin verwendet wird, ist die Bezeichnung für die einzel- bzw. doppelsträngige Kopie eines RNA-Moleküls und ist deshalb als Kopie biologisch aktiver mRNA intronfrei, d.h. alle kodierenden Regionen eines Gens sind in zusammenhängender Form enthalten.

Hybridisierungsbedingungen, wie sie in erster Linie für die vorstehend genannten PCR-Verfahren zur Identifizierung von weiteren Glyoxaloxidasen aus Pilzen verwendet werden können, werden nach folgender Formel näherungsweise berechnet:

Die Schmelztemperatur Tm = 81.5 °C + 16.6 log[c(Na⁺)] + 0.41(%G + C)) - 500/n (Lottspeich und Zorbas, 1998).

Dabei ist c die Konzentration und n die Länge des hybridisierenden Sequenzabschnitts in Basenpaaren. Für eine Sequenz >100 bp entfällt der Ausdruck 500/n. Mit höchster Stringenz wird bei einer Temperatur 5-15°C unterhalb Tm und einer Ionenstärke von 15 mM Na⁺ (entspricht 0.1 x SSC) gewaschen. Wird eine RNA-Probe zur Hybridisierung verwendet, so ist der Schmelzpunkt um 10-15 °C höher.

Der Grad der Identität der Nukleinsäuren wie vorstehend beschrieben wird vorzugsweise bestimmt mit Hilfe des Programms CLUSTALW oder des Programms BLASTX Version 2.0.4 (Altschul *et al.,* 1997).

Für die vorliegende Erfindung können weiterhin DNA-Konstrukte verwendet werden, die eine hierin beschriebene Nukleinsäure und einen homologen oder heterologen Promotor umfassen.

Der Ausdruck "homologer Promotor", wie er hierin verwendet wird, bezieht sich auf einen Promotor, der im Ursprungsorganismus die Expression des betreffenden Gens kontrolliert.

Der Ausdruck "heterologer Promotor", wie er hierin verwendet wird, bezieht sich auf einen Promotor, der andere Eigenschaften als derjenige Promotor aufweist, der im Ursprungsorganismus die Expression des betreffenden Gens kontrolliert.

Die Auswahl von heterologen Promotoren ist davon abhängig, ob zur Expression pro- oder eukaryotische Zellen oder zellfreie Systeme verwendet werden. Beispiele für heterologe Promotoren sind der 35S Promoter des Blumenkohlmosaikvirus für pflanzliche Zellen, der Promoter der Alkoholdehydrogenase für Hefezellen, die T3-, T7- oder SP6-Promotoren für prokaryotische Zellen oder zellfreie Systeme.

Bevorzugt sollten pilzliche Expressionssysteme wie z.B. das *Pichia pastoris-*System verwendet werden, wobei hier die Transkription durch den Methanol induzierbaren AOX-Promotor angetrieben wird. Für dieses System konnte bereits für die Glyoxaloxidase aus *Phanerochaete chrysosporium,* heterologe Expression gezeigt werden (Whittaker, M. et al., 1999).

Für die vorliegende Erfindung können ferner Vektoren verwendet werden, die eine hierin beschriebene Nukleinsäure, eine hierin beschriebene regulatorische Region oder ein hierin beschriebenes DNA-Konstrukt enthalten. Als Vektoren können alle in molekularbiologischen Laboratorien verwendete Phagen, Plasmide, Phagmide, Phasmide, Cosmide, YACs, BACs, künstliche Chromosomen oder Partikel, die für einen Partikelbeschuss geeignet sind, verwendet werden.

Bevorzugte Vektoren sind pBIN (Bevan, 1984) und seine Derivate für pflanzliche Zellen, pFL61 (Minet *et al.,* 1992) oder z.B. die p4XXprom. Vektorserie (Mumberg et al., 1995) für Hefezellen, pSPORT-Vektoren (Fa. Life Technologies) für bakterielle Zellen, oder die Gateway Vektoren (Fa. Life Technologies) für verschiedene Expressionssysteme in bakteriellen Zellen, Pflanzen, *P*. *pastoris, S. cerevisiae* oder Insektenzellen.

Für die vorliegende Erfindung können auch Wirtszellen verwendet werden, die eine hierin beschriebene Nukleinsäure, ein hierin beschriebenes DNA-Konstrukt oder einen hierin beschriebenen Vektor enthalten.

Der Ausdruck "Wirtszelle", wie er hierin verwendet wird, bezieht sich auf Zellen, die natürlicherweise die hierin beschriebenen Nukleinsäuren nicht enthalten.

Als Wirtszellen eignen sich sowohl prokaryotische Zellen, vorzugsweise *E. coli,* als auch eukaryotische Zellen, wie Zellen von *Saccharomyces cerevisiae, Pichia pastoris,* Insekten, Pflanzen, Froschoozyten und Zelllinien von Säugern.

Bevorzugt für die Expression werden pilzliche Zellen wie z.B. von *Saccharomyces cerevisiae, Aspergillus nidulans* und *Pichia pastoris* verwendet. Die Expression der Glyoxaloxidase aus *Phanerochaete chrysosporium* wurde z.B. in *A. nidulans* und *P. pastoris* erfolgreich durchgeführt (Kersten et al., 1995; Whittaker et al., 1999).

Insbesondere können auch Zellen von *Ustilago maydis* zur Expression der hierin beschriebenen Polypeptide verwendet werden. Insbesondere sind dazu Zellen eines Stammes von *U. maydis* geeignet, der bei der Deutschen Sammlung von Mikroorganismen und Zellkulturen GmbH (DSMZ), Mascheroder Weg 1 b in 38124 Braunschweig unter der Nummer DSM 14 509 am 13. 09. 2001 hinterlegt wurde.

Diese hinterlegten Zellen wurden wie in Beispiel 3 beschrieben erhalten und können z.B. anhand des in Beispiel 4 dargestellten Assays von Wildtyp-Zellen des Ausgangsstammes unterschieden werden. Der Stamm mit der Hinterlegungsnummer DSM 14 509 ist in der Lage, die hierin beschriebene Glyoxaloxidase aus *U. maydis* in ausreichender Menge und Aktivität zu exprimieren, damit eine Glyoxaloxidase-Aktivität nachgewiesen und der Stamm in einem erfindungsgemäßen Verfahren verwendet werden kann.

Der Stamm mit der Hinterlegungsnummer DSM 14 509 kann für die vorliegende Erfindung verwendet werden.

Für die vorliegende Erfindung können weiterhin Polypeptide mit der biologischen Aktivität von Glyoxaloxidasen verwendet werden, die von den hierin beschriebenen Nukleinsäuren kodiert werden.

Bevorzugt umfassen diese Polypeptide eine Aminosäuresequenz ausgewählt aus
a) der Sequenz gemäß SEQ ID NO:2, SEQ ID NO:4, SEQ ID NO:6, SEQ ID NO:8, SEQ ID NO:10 und SEQ ID NO:12,
b) Sequenzen, die die für die Cu²⁺-Koordination geeigneten Aminosauren Tyrosin 1, Tyrosin 2, Histidin 1, Histidin 2 und Cystein gemäß Formel (I) umfassen,
c) zumindest 15 Aminosäuren lange Teilsequenzen der unter a) und b) definierten Sequenzen
d) Sequenzen, welche eine zumindest 20%ige, bevorzugt eine 25%ige, besonders bevorzugt eine 40%ige, ganz besonders bevorzugt eine 60%ige und am meisten bevorzugt eine 75%ige Identität mit den unter a) und b) definierten Sequenzen haben, und
e) Sequenzen, welche die gleiche biologische Aktivität aufweisen wie die unter a) bis d) definierten Sequenzen.

Der Ausdruck "Polypeptide", wie er hierin verwendet wird, bezieht sich sowohl auf kurze Aminosäureketten, die gewöhnlich als Peptide, Oligopeptide oder Oligomere bezeichnet werden, als auch auf längere Aminosäureketten, die gewöhnlich als Proteine bezeichnet werden. Er umfasst Aminosäureketten, die entweder durch natürliche Prozesse, wie posttranslationale Prozessierung, oder durch chemische Verfahren, die Stand der Technik sind, modifiziert sein können. Solche Modifikationen können an verschiedenen Stellen und mehrfach in einem Polypeptid vorkommen, wie beispielsweise an dem Peptid-Rückgrat, an der Aminosäure-Seitenkette, am Amino- und/oder am Carboxy-Terminus. Sie umfassen beispielsweise Acetylierungen, Acylierungen, ADP-Ribosylierungen, Amidierungen, kovalente Verknüpfungen mit Flavinen, Häm-Anteilen, Nukleotiden oder Nukleotid-Derivaten, Lipiden oder Lipid-Derivaten oder Phosphatidylinositol, Cyclisierungen, Disulfidbrückenbildungen, Demethylierungen, Cystin-Bildungen, Formylierungen, gamma-Carboxylierungen, Glycosylierungen, Hydroxylierungen, Iodierungen, Methylierungen, Myristoylierungen, Oxidationen, proteolytische Prozessierungen, Phosphorylierungen, Selenoylierungen und tRNA-vermittelte Additionen von Aminosäuren.

Die hierin beschriebenen Polypeptide können in der Form "reifer" Proteine oder als Teile größerer Proteine, z.B. als Fusionsproteine, vorliegen. Weiterhin können sie Sezemierungs- oder "Leader"-Sequenzen, Pro-Sequenzen, Sequenzen, die eine einfache Reinigung ermöglichen, wie mehrfache Histidin-Reste, oder zusätzliche stabilisierende Epitope aufweisen.

Diese Polypeptide, insbesondere die Polypeptide gemäß SEQ ID NO:2, 4, 6 8, 10 und 12 müssen nicht vollständige pilzliche Glyoxaloxidasen darstellen, sondern können auch nur Fragmente davon sein, solange sie zumindest noch die biologische Aktivität der vollständigen pilzlichen

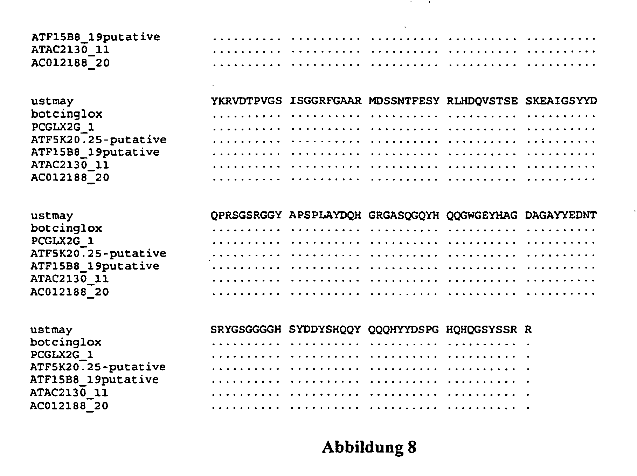

Glyoxaloxidase mit einer Aminosäuresequenz gemäß SEQ ID NO:2, 4, 6, 8 oder SEQ ID NO:10 und 12 ausüben, werden noch als für die vorliegende Erfindung verwendbar betrachtet. Dabei müssen diese Polypeptide nicht von Glyoxaloxidasen aus *Ustilago maydis* oder *Botrytis cinerea* oder aus phytopathogenen Pilzen ableitbar sein, sondern können z.B. aufgrund der Verwandtschaft zwischen den Glyoxaloxidasen aus verschiedenen Organismen wie aus humanpathogenen Pilzen und auch aus Pflanzen stammen (siehe auch Abb. 8). Als für die vorliegende Erfindung verwendbar werden vor allem auch solche Polypeptide betrachtet, die Glyoxaloxidasen beispielsweise der folgenden Pilze entsprechen oder Fragmenten davon, die noch deren biologische Aktivität haben:

Plasmodiophoromycetes, Oomycetes, Chytridiomycetes, Zygomycetes, Ascomycetes, Basidiomycetes und Deuteromycetes, z.B.

Pythium-Arten, wie beispielsweise *Pythium ultimum,* Phytophthora-Arten, wie beispielsweise *Phytophthora infestans,* Pseudoperonospora-Arten, wie beispielsweise *Pseudoperonospora humuli* oder *Pseudoperonospora cubensis,* Plasmopara-Arten, wie beispielsweise *Plasmopara viticola,* Bremia-Arten, wie beispielsweise *Bremia lactucae,* Peronospora-Arten, wie beispielsweise *Peronospora pisi* oder *P. brassicae,* Erysiphe-Arten, wie beispielsweise *Erysiphe graminis,* Sphaerotheca-Arten, wie beispielsweise *Sphaerotheca fuliginea,* Podosphaera-Arten, wie beispielsweise *Podosphaera leucotricha,* Venturia-Arten, wie beispielsweise *Venturia inaequalis,* Pyrenophora-Arten, wie beispielsweise *Pyrenophora teres* oder *P. graminea* (Konidienform: Drechslera, Syn: Helminthosporium), Cochliobolus-Arten, wie beispielsweise *Cochliobolus sativus* (Konidienform: Drechslera, Syn: Helminthosporium), Uromyces-Arten, wie beispielsweise *Uromyces appendiculatus,* Puccinia-Arten, wie beispielsweise *Puccinia recondita,* Sclerotinia-Arten, wie beispielsweise *Sclerotinia sclerotiorum,* Tilletia-Arten, wie beispielsweise *Tilletia caries;* Ustilago-Arten, wie beispielsweise *Ustilago nuda* oder *Ustilago avenae,* Pellicularia-Arten, wie beispielsweise *Pellicularia sasakii,* Pyricularia-Arten, wie beispielsweise *Pyricularia oryzae,* Fusarium-Arten, wie beispielsweise *Fusarium culmorum,* Botrytis-Arten, Septoria-Arten, wie beispielsweise *Septoria nodorum,* Leptosphaeria-Arten, wie beispielsweise *Leptosphaeria nodorum,* Cercospora-Arten, wie beispielsweise *Cercospora canescens,* Alternaria-Arten, wie beispielsweise *Alternaria brassicae* oder Pseudocercosporella-Arten, wie beispielsweise *Pseudocercosporella herpotrichoides.*

Von besonderem Interesse sind z.B. auch *Magnaporthe grisea, Cochliobulus heterostrophus, Nectria hematococca* und Phytophthora Spezies.

Wie bereits vorstehend erörtert, können die hierin beschriebenen Polypeptide auch als ein Wirkort für Antimycotica und damit zur Bekämpfung von human- bzw. tierpathogenen Pilzen verwendet werden. Dabei sind z.B. die folgenden humanpathogenen Pilze von besonderem Interesse, die die genannten Krankheitsbilder hervorrufen können:
Dermatophyten, wie z.B. Trichophyton spec., Microsporum spec., *Epidermophyton floccosum* oder *Keratomyces ajelloi,* die z.B. Fußmykosen (Tinea pedis) hervorrufen,
Hefen, wie z.B. *Candida albicans,* der z.B. Soor-Ösophagitis und Dermatitis hervorruft, *Candida glabrata, Candida krusei* oder *Cryptococcus neoformans,* die z.B. pulmonale Cryptococcose und auch Torulose hervorrufen können,
Schimmelpilze, wie z.B. *Aspergillus fumigatus, A. flavus, A. niger,* die z.B. bronchopulmonale Aspergillose oder Pilzsepsis hervorrufen, Mucor spec., Absidia spec., oder Rhizopus spec., die z.B. Zygomykosen (intravasale Mykosen) hervorrufen, *Rhinosporidium seeberi,* der z.B. chron. granulomatöse Pharyngitis und Tracheitis hervorruft, *Madurella mycetomatis,* der z.B. subkutane Myzetome hervorruft, *Histoplasma capsulatum,* der z.B. retikuloendotheliale Zytomykose und M. Darling hervorruft, *Coccidioides immitis,* der z.B. pulmonale Coccidioidomykose und Sepsis hervorruft, *Paracoccidioides brasiliensis,* der z.B. brasilianische Blastomykose hervorruft, *Blastomyces dermatitidis,* der z.B. Gilchrist-Krankheit und nordamerikanische Blastomykose hervorruft, *Loboa loboi,* der z.B. Keloid-Blastomykose und Lobo's Krankheit hervorruft, und *Sporothrix schenckii,* der z.B. Sporotrichose (granulomatöse Hautmykose) hervorruft.

Diese Polypeptide können im Vergleich zu der entsprechenden Region von natürlich vorkommenden Glyoxaloxidasen Deletionen oder Aminosäuresubstitutionen aufweisen, solange sie zumindest noch eine biologische Aktivität der vollständigen Glyoxaloxidase ausüben. Konservative Substitutionen sind bevorzugt. Solche konservativen Substitutionen umfassen Variationen, wobei eine Aminosäure durch eine andere Aminosäure aus der folgenden Gruppe ersetzt wird:
1. Kleine aliphatische, nicht-polare oder wenig polare Reste: Ala, Ser, Thr, Pro und Gly;
2. Polare, negativ geladene Reste und deren Amide: Asp, Asn, Glu und Gln;
3. Polare, positiv geladene Reste: His, Arg und Lys;
4. Große aliphatische, nicht-polare Reste: Met, Leu, Ile, Val und Cys; und
5. Aromatische Reste: Phe, Tyr und Trp.

Die folgende Liste zeigt bevorzugte konservative Substitutionen:

| **Ursprünglicher Rest** | **Substitution** |
|---|---|
| Ala | Gly, Ser |
| Arg | Lys |
| Asn | Gln, His |
| Asp | Glu |
| Cys | Ser |
| Gln | Asn |
| Glu | Asp |
| Gly | Ala, Pro |
| His | Asn, Gln |
| Ile | Leu, Val, Met |
| Leu | Ile, Val, Met |
| Lys | Arg |
| Met | Leu, Ile |
| Phe | Met, Leu, Tyr, Ile, Trp |
| Pro | Gly |
| Ser | Thr |
| Thr | Ser |
| Trp | Tyr |
| Tyr | Trp, Phe |
| Val | Ile, Leu |

Für die vorliegende Erfindung können somit auch Polypeptide verwendet werden, welche zumindest die biochemische Reaktion der Bildung von Wasserstoffperoxid durch Reduktion von Sauerstoff bei der Umsetzung von Glyoxal oder Methylglyoxal oder deren Derivate so wie die Glyoxaloxidase ausüben und eine Aminosäuresequenz umfassen, die eine zumindest 20%ige Identität, vorzugsweise 25%ige Identität, besonders bevorzugt 40%ige Identität, ganz besonders bevorzugt 60%ige, am meisten bevorzugt eine 75%ige Identität und schließlich absolut bevorzugt eine 90 %ige Identität mit der Sequenz gemäß SEQ ID NO: 2 bzw. 4 oder SEQ ID NO: 6 oder 8 sowie SEQ ID NO:10 bzw. 12 über eine Länge von 100 Aminosäuren, bevorzugt 250 Aminosäuren und besonders bevorzugt über deren Gesamtlänge aufweist.

Der Grad der Identität der Aminosäuresequenzen wird vorzugsweise bestimmt mit Hilfe des Programms BLASTP + BEAUTY (Altschul et al., 1997).

Eine besonders bevorzugte Ausführungsform der hierin beschriebenen Polypeptide sind Glyoxaloxidasen mit einer Aminosäuresequenz gemäß SEQ ID NO: 2, 4, 6 und 8 sowie SEQ ID NO: 10 und 12.

Besonders bevorzugt können für die vorliegende Erfindung solche Polypeptide verwendet werden, die die vorstehend genannten, zur Ausbildung einer Cu²⁺-Koordinationsstelle geeigneten Aminosäuren umfassen:
**Tyrosin-Ligand 1:** (z.B. Tyr 178 (*U. maydis*) bzw. Tyr 273 (*B. cinerea*)),
**Tyrosin-Ligand 2:** (z.B. Tyr 452 (*U. maydis*) bzw. Tyr 499 (*B. cinerea*)),
**Histidin-Ligand 1:** (z.B. His 453 (*U. maydis*) bzw. His 500 (*B. cinerea*)),
**Histidin-Ligand 2:** (z.B. His 555 (*U. maydis*) bzw. His 597 (*B .cinerea*)), und
**Cystein-Rest:** (z.B. Cys 105 (*U. maydis*) bzw. Cys 209 *(B. cinerea*)).

Die hierin beschriebenen Nukleinsäuren können auf die übliche Weise hergestellt werden. Beispielsweise können die Nukleinsäuremoleküle vollständig chemisch synthetisiert werden. Man kann auch kurze Stücke der hierin beschriebenen Nukleinsäuren chemisch synthetisieren und solche Oligonukleotide radioaktiv oder mit einem Fluoreszenzfarbstoff markieren. Die markierten Oligonukleotide können auch verwendet werden, um ausgehend von Pilz-mRNA hergestellte cDNA-Banken zu durchsuchen. Klone, mit denen die markierten Oliogonukleotide hybridisieren, werden zur Isolierung der betreffenden DNA-Fragmente ausgewählt. Nach der Charakterisierung der isolierten DNA erhält man auf einfache Weise die hierin beschriebenen Nukleinsäuren.

Die hierin beschriebenen Nukleinsäuren können auch mittels PCR-Verfahren unter Verwendung chemisch synthetisierter Oligonukleotide hergestellt werden.

Der Ausdruck "Oligonukleotid(e)", wie er hierin verwendet wird, bezeichnet DNA-Moleküle, die aus 10 oder mehr Nukleotiden, vorzugsweise 15 bis 30 Nukleotiden, bestehen. Sie werden chemisch synthetisiert und können als Sonden verwendet werden.

Dem Fachmann ist bekannt, dass die hierin beschriebenen Polypeptide auf verschiedenem Wege gewonnen werden können, z.B. durch chemische Methoden wie der Festphasenmethode. Zur Gewinnung größerer Proteinmengen empfiehlt sich die Verwendung rekombinanter Methoden. Die Expression eines klonierten Glyoxaloxidase-Gens oder Fragmenten davon kann in einer Reihe von passenden Wirtszellen erfolgen, die dem Fachmann bekannt sind. Zu diesem Zweck wird ein Glyoxaloxidase-Gen mit Hilfe bekannter Methoden in eine Wirtszelle eingeführt.

Die Integration des klonierten Glyoxaloxidase Gens in das Chromosom der Wirtszelle liegt im Umfang der vorliegenden Erfindung. Vorzugsweise wird das Gen oder Fragmente davon in ein Plasmid eingebracht, und die kodierenden Regionen des Glyoxaloxidase-Gens oder von Fragmenten davon mit einem konstitutiven oder induzierbaren Promotor funktionell verknüpft. Besonders geeignet für die Expression ist z.B. das *Pichia pastoris* Expressions-System der Firma Invitrogen. Als geeignete Vektoren dienen hier z.B. pPICZ und dessen Derivate. Die Expression kann hierbei mittels des AOX-Promotors durch Methanolzugabe induziert werden. Geeignet wäre drüber hinaus auch die Expression im *U. maydis* System. Dabei würde die Expression der Glyoxaloxidase-Gene oder von Fragmenten davon z.B. durch den induzierbaren crg1-Promotor oder den konstitutiven otef-Promotor erfolgen (Bottin et al., 1996, Spelling et al., 1994).

Die grundlegenden Schritte zur Herstellung rekombinanter Glyoxaloxidasen sind:
1. Gewinnung einer natürlichen, synthetischen oder semi-synthetischen DNA, die für eine Glyoxaloxidase kodiert.
2. Einbringen dieser DNA in einen Expressionsvektor, der geeignet ist Glyoxaloxidasen zu exprimieren, entweder alleine oder als Fusionsprotein.
3. Transformation einer passenden, vorzugsweise eukaryontischen Wirtszelle mit diesem Expressionsvektor.
4. Anzucht dieser transformierten Wirtszelle in einer Weise, die geeignet ist, Glyoxaloxidasen zu exprimieren.
5. Ernte der Zellen und gegebenenfalls Aufreinigung der Glyoxaloxidasen durch geeignete, bekannte Methoden.

Die kodierende Region der Glyoxaloxidasen kann dabei mit den üblichen Methoden in *E. coli* exprimiert werden. Geeignete Expressionssysteme für *E. coli* sind kommerziell erhältlich, so die Expressionsvektoren der pET-Serie, z.B. pET3a, pET23a, pET28a mit His-Tag oder pET32a mit His-Tag zur einfachen Aufreinigung und Thioredoxinfusion zur Erhöhung der Löslichkeit des exprimierten Enzyms, sowie pGEX mit Glutathionsynthetase-Fusion, sowie die pSPORT Vektoren. Die Expressionsvektoren werden in λ DE3-lysogene *E*. *coli*-Stämme, z.B. BL21(DE3), HMS 174(DE3) oder AD494(DE3) transformiert. Nach dem Anwachsen der Zellen unter dem Fachmann geläufigen Standardbedingungen wird die Expression mit IPTG induziert. Nach Induktion der Zellen wird für 3 bis 24 Stunden bei Temperaturen von 4 bis 37°C inkubiert.

Die Zellen werden durch Sonifikation in Aufschlusspuffer (10 bis 200 mM Natriumphosphat, 100 bis 500 mM NaCl, pH 5 bis 8) aufgeschlossen. Das exprimierte Protein kann über chromatographische Methoden gereinigt werden, im Fall von mit His-Tag exprimiertem Protein durch Chromatographie an einer Ni-NTA-Säule.

Die Expression des Proteins in Insektenzellkulturen (z.B. Sf9-Zellen) stellt einen anderen günstigen Ansatz dar.

Alternativ können die Proteine auch in Pflanzen exprimiert werden. So existieren z. B. auch in *Arabidopsis thaliana* mindestens 3 Glyoxaloxidase Homologe (siehe Abb. 8), was die Möglichkeit einer Expression in Pflanzen unterstreicht.

Gegenstand der vorliegenden Erfindung sind Verfahren zum Auffinden von chemischen Verbindungen, die an die hierin beschriebenen Polypeptide binden und deren Eigenschaften verändern. So stellen Modulatoren, die die Aktivität des Enzyms beeinflussen, neue fungizide Wirkstoffe dar, die in der Lage sind, die Pathogenität der Pilze zu kontrollieren. Modulatoren können Agonisten oder Antagonisten bzw. Aktivatoren oder Inhibitoren sein. Von besonderem Interesse sind im Falle der Glyoxaloxidase Inhibitoren dieses Enzyms, die durch das Ausschalten des Enzyms die Pathogenität der Pilze unterbinden können.

Glyoxaloxidasen aus Pilzen können also als Angriffspunkte für Fungizide und in Verfahren zum Auffinden von Modulatoren dieser Polypeptide verwendet werden. In solchen Verfahren können Glyoxaloxidasen direkt in einer Wirtszelle, in Extrakten oder aufgereinigt eingesetzt werden, oder mittelbar über die Expression der dafür kodierenden DNA entstehen. Für diese Verwendung sind ebenso die Polypeptide geeignet, die vorstehend beschrieben wurden (Glo 2 und Glo 3 gemäß SEQ ID NO:6 und SEQ ID NO:8). Diese besitzen unabhängig von ihrer unmittelbaren Bedeutung für die Pathogenität des Pilzes eine ausreichende Homologie zu Glo1, um ebenfalls in Verfahren zum Identifizieren von Modulatoren des Enzyms verwendet zu werden, die dann als Fungizid wirksam werden.

Die für die hierin beschriebenen Glyoxaloxidasen kodierenden Nukleinsäuren, die DNA-Konstrukte, die diese enthalten, die Wirtszellen, die diese enthalten, können in Verfahren zum Auffinden von Modulatoren der Glyoxaloxidase verwendet werden.

Der Ausdruck "Agonist", wie er hierin verwendet wird, bezieht sich auf ein Molekül, das die Aktivität der Glyoxaloxidase beschleunigt oder verstärkt.

Der Ausdruck "Antagonist", wie er hierin verwendet wird, bezieht sich auf ein Molekül, das die Aktivität der Glyoxaloxidase verlangsamt oder verhindert.

Der Ausdruck "Modulator", wie er hierin verwendet wird, stellt den Oberbegriff zu Agonist bzw. Antagonist dar. Modulatoren können kleine organisch-chemische Moleküle, Peptide oder Antikörper sein, die an die hierin beschriebenen Polypeptide binden. Weiterhin können Modulatoren kleine organisch-chemische Moleküle, Peptide oder Antikörper sein, die an ein Molekül binden, welches wiederum an die hierin beschriebenen Polypeptide bindet, und dadurch deren biologische Aktivität beeinflusst. Modulatoren können natürliche Substrate und Liganden darstellen oder strukturelle oder funktionelle Mimetika davon. Der Ausdruck "Modulator" umfasst jedoch nicht die natürlichen Substrate der Glyoxaloxidase wie z.B. Sauerstoff, Glyoxal und Methylglyoxal.

Vorzugsweise handelt es sich bei den Modulatoren um kleine organisch-chemische Verbindungen.

Die Bindung der Modulatoren an die hierin beschriebenen Glyoxaloxidasen kann die zellulären Vorgänge auf eine Weise verändern, die zur Apathogenität oder zum Absterben des damit behandelten Pilzes führt.

Die Verwendung der hierin beschriebenen Nukleinsäuren bzw. Polypeptide in einem erfindungsgemäßen Verfahren ermöglicht das Auffinden von Verbindungen, die an die hierin beschriebenen Polypeptide binden. Diese können dann als Fungizide, z.B. bei Pflanzen oder als antimycotische Wirkstoffe bei Menschen und Tieren angewandt werden. Beispielsweise werden Wirtszellen, die die hierin beschriebenen Nukleinsäuren enthalten und die entsprechenden Polypeptide exprimieren oder die Genprodukte selbst mit einer Verbindung oder einem Gemisch von Verbindungen unter Bedingungen in Kontakt gebracht, die die Wechselwirkung zumindest einer Verbindung mit den Wirtszellen, den Rezeptoren oder den einzelnen Polypeptiden erlauben.

Insbesondere ist ein Verfahren Gegenstand der vorliegenden Erfindung, das zur Identifizierung von fungiziden Wirkstoffen geeignet ist, die an Polypeptide aus Pilzen mit der biologischen Aktivität einer Glyoxaloxidase binden, bevorzugt an Glyoxaloxidasen aus phytopathogenen Pilzen, besonders bevorzugt an Glyoxaloxidasen aus *Ustilago* oder *Botrytis* und ganz besonders bevorzugt an Glyoxaloxidasen aus *U. maydis* und *B. cinerea* und dazu homologe Polypeptide, die die vorstehend genannte Konsensussequenz aufweisen. Die Verfahren können jedoch auch mit einem zu den hierin beschriebenen Glyoxaloxidasen homologen Polypeptid aus einer anderen als den hier genannten Spezies durchgeführt werden. Verfahren, die andere als die hierin beschriebene Glyoxaloxidase verwenden, sind von der vorliegenden Erfindung umfasst.

Viele Testsysteme, die die Prüfung von Verbindungen und natürlichen Extrakten zum Ziel haben, sind auf hohe Durchsatzzahlen ausgerichtet, um die Zahl der untersuchten Substanzen in einem gegebenen Zeitraum zu maximieren. Testsysteme, die auf zellfreiem Arbeiten beruhen, brauchen gereinigtes oder semi-gereinigtes Protein. Sie sind geeignet für eine "erste" Prüfung, die in erster Linie darauf abzielt, einen möglichen Einfluss einer Substanz auf das Zielprotein zu detektieren. Es können jedoch auch Testsysteme verwendet werden, die auf ganzen Zellen basieren, die das betreffende Polypeptid in ausreichender Menge produzieren. Im vorliegenden Fall gelingt die Messung der Enzymenaktivität auch mit ganzen, die Glyoxaloxidase überproduzierenden Zellen, z.B. *Ustilago maydis* Zellen in Analogie zum Aktivitätstest gemäß Beispiel 4.

Effekte wie Zelltoxizität werden in diesen *in vitro* Systemen in der Regel ignoriert. Die Testsysteme überprüfen dabei sowohl inhibierende bzw. suppressive Effekte der Substanzen, als auch stimulatorische Effekte. Die Effektivität einer Substanz kann durch konzentrationsabhängige Testreihen überprüft werden. Kontrollansätze ohne Testsubstanzen können zur Bewertung der Effekte herangezogen werden.

Um Modulatoren aufzufinden, kann ein synthetischer Reaktionsmix (z.B. Produkte der in vitro-Translation) oder ein zellulärer Bestandteil, wie eine Extrakt oder irgendeine andere Präparation, die das Polypeptid enthält, zusammen mit einem markierten Substrat oder Liganden der Polypeptide in Gegenwart und Abwesenheit eines Kandidatenmoleküls, das ein Agonist oder Antagonist sein kann, inkubiert werden. Die Fähigkeit des Kandidatenmoleküls, die Aktivität der hierin beschriebenen Polypeptide zu erhöhen oder zu hemmen, wird erkennbar an einer erhöhten oder verringerten Bindung des markierten Liganden oder an einer erhöhten oder verringerten Umsetzung des markierten Substrates. Moleküle, die gut binden und zu einer erhöhten Aktivität der hierin beschriebenen Polypeptide führen, sind Agonisten. Moleküle, die gut binden, aber der biologischen Aktivität der hierin beschriebenen Polypeptide entgegen wirken, sind wahrscheinlich gute Antagonisten.

Modulatoren des hierin beschriebenen Polypeptids können auch über enzymatische Tests aufgefunden werden. Es kann entweder die Änderung der Enzymaktivität durch entsprechende Modulatoren direkt oder in einem gekoppelten Enzymtest indirekt gemessen werden. Die Messung kann z.B. über Absorptionsänderung durch die Ab- oder Zunahme einer optisch aktiven Verbindung durchgeführt werden. So kann z.B. die Freisetzung bzw. der Verbrauch von Wasserstoffperoxid durch Entfärbung einer Phenolrot-Lösung in Gegenwart von MeerrettichPeroxidase nachgewiesen werden (siehe Bsp. 4, 10 und 11).

Eine weitere Möglichkeit zur Identifizierung von Substanzen, die die Aktivität der hierin beschriebenen Polypeptide modulieren, ist der sogenannten "Scintillation Proximity Assay" (SPA), siehe EP 015 473. Dieses Testsystem nutzt die Interaktion eines Polypeptids (z.B. Glyoxaloxidase aus *U. maydis* oder *B. cinerea*) mit einem radiomarkierten Liganden (z.B. ein kleines organisches Molekül oder ein zweites, radioaktiv markiertes Proteinmolekül). Das Polypeptid ist dabei an kleine Kügelchen ("Microspheres") oder Perlen ("Beads") gebunden, die mit szintillierenden Molekülen versehen sind. Im Verlauf des Abfalls der Radioaktivität wird die szintillierende Substanz im Kügelchen durch die subatomaren Partikel des radioaktiven Markers angeregt und ein detektierbares Photon emittiert. Die Testbedingungen werden so optimiert, dass nur jene vom Liganden ausgehenden Partikel zu einem Signal führen, die von einem an das hierin beschriebene Polypeptid gebundenen Liganden ausgehen.

In einer möglichen Ausführungsform ist Glyoxaloxidase z.B. aus *U. maydis* an die Beads gebunden, entweder zusammen oder ohne interagierende bzw. bindende Testsubstanz. Verwendet werden könnten dabei auch Fragmente des hierin beschriebenen Polypeptids. Bei der Bindung eines Liganden an die immobilisierte Glyoxaloxidase, müsste dieser Ligand eine bestehende Interaktion zwischen der immobilisiertem Glyoxaloxidase und dem markierten Liganden inhibieren oder aufheben, um selbst in den Bereich der Kontaktfläche zu gelangen. Eine erfolgte Bindung an die immobilisierte Glyoxaloxidase kann dann anhand eines Lichtblitzes detektiert werden. Entsprechend wird ein bestehender Komplex zwischen einem immobilisierten und einem freien, markierten Liganden durch die Bindung einer Testsubstanz zerstört, was zu einem Abfall der detektierten Lichtblitzintensität führt. Das Testsystem entspricht dann einem komplementären Inhibitions-System.

Ein weiteres Beispiel für ein Verfahren, mit welchem Modulatoren der hierin beschriebenen Polypeptide aufgefunden werden können, ist ein Verdrängungstest, bei dem man unter dafür geeigneten Bedingungen die hierin beschriebenen Polypeptide und einen potenziellen Modulator mit einem Molekül, das bekanntermaßen an die hierin beschriebenen Polypeptide bindet, wie einem natürlichen Substrat oder Liganden oder einem Substrat- oder Liganden-Mimetikum zusammenbringt.

Der Ausdruck "Kompetitor" wie er hierin verwendet wird, bezieht sich auf die Eigenschaft der Verbindungen, mit anderen, gegebenenfalls noch zu identifizierenden Verbindungen um die Bindung an der Glyoxaloxidase zu kompetitieren und diese vom Enzym zu verdrängen bzw. von dieser verdrängt zu werden.

Bislang ist nicht bekannt geworden, dass Glyoxaloxidasen aus pflanzenpathogenen Pilzen ein neues Target für Fungizide darstellen und dass mit Hilfe dieser Glyoxaloxidasen Verbindungen gefunden und entwickelt werden können, die als Fungizide eingesetzt werden können. In der vorliegenden Anmeldung wird diese Möglichkeit zum ersten Mal beschrieben und belegt. Weiter werden hier die dazu nötigen Glyoxaloxidasen sowie Verfahren zu deren Gewinnung und zur Identifizierung von Inhibitoren des Enzyms zur Verfügung gestellt.

Fungizide Wirkstoffe, die mit Hilfe der hierin beschriebenen Polypeptide gefunden werden, können auch mit Glyoxaloxidasen aus humanpathogenen Pilzspezies interagieren, wobei die Interaktion mit den unterschiedlichen in diesen Pilzen vorkommenden Glyoxaloxidasen nicht immer gleich stark sein muss.

Inhibitoren von Polypeptiden mit der Funktion einer Glyoxaloxidase können zum Herstellen von Mitteln zur Behandlung von durch tier- bzw. humanpathogene Pilze hervorgerufenen Erkrankungen verwendet werden.

Die Begriffe "Fungizid" bzw. "fungizid", wie sie hierin verwendet werden, umfassen im erfindungsgemäßen Sinne auch die Begriffe "Antimycoticum" bzw. "antimycotisch".

Weiterhin umfasst die vorliegende Erfindung Verfahren zum Auffinden von chemischen Verbindungen, welche die Expression der hierin beschriebenen Polypeptide verändern. Auch solche "Expressionsmodulatoren" können neue fungizide Wirkstoffe darstellen. Expressionsmodulatoren können kleine organisch-chemische Moleküle, Peptide oder Antikörper sein, die an die regulatorischen Regionen der für die hierin beschriebenen Polypeptide kodierenden Nukleinsäuren binden. Weiterhin können Expressionsmodulatoren kleine organisch-chemische Moleküle, Peptide oder Antikörper sein, die an ein Molekül binden, welches wiederum an regulatorische Regionen der für die hierin beschriebenen Polypeptide kodierenden Nukleinsäuren bindet, und dadurch deren Expression beeinflusst. Expressionsmodulatoren können auch Antisense-Moleküle sein.

Die erfindungsgemäßen Verfahren schließen Hochdurchsatz-Screening (high throughput screening; HTS) ein. Dafür können sowohl Wirtszellen als auch zellfreie Präparationen verwendet werden, die die hierin beschriebenen Nukleinsäuren und/oder die hierin beschriebenen Polypeptide enthalten.

Die hierin beschriebenen Nukleinsäuren können ebenfalls zur Herstellung transgener Organismen wie Bakterien, Pflanzen oder Pilze, bevorzugt zur Herstellung transgener Pflanzen und Pilze und besonders bevorzugt zur Herstellung transgener Pilze verwendet werden. Diese können z.B. in Testsystemen eingesetzt werden, die auf einer vom Wildtyp abweichenden Expression der hierin beschriebenen Polypeptide oder Varianten davon basieren. Ferner fallen hierunter sämtliche transgenen Pflanzen oder Pilze, bei denen durch die Modifikation anderer Gene als der vorstehend beschriebenen oder die Modifikation von Genkontrollsequenzen (z.B. Promotor) eine Veränderung der Expression der hierin beschriebenen Polypeptide oder deren Varianten eintritt.

Die transgenen Organismen sind auch zur (Über)produktion des hierin beschriebenen Polypeptids für kommerzielle bzw. gewerbliche Zwecke von Interesse, wobei z.B. Pilze (z.B. Hefe oder *Ustilago maydis*), die im Vergleich zur ihrer natürlichen Form das hierin beschriebene Polypeptid vermehrt exprimieren, sich besonders zum Einsatz in Verfahren (gerade auch HTS-Verfahren) zum Identifizieren von Modulatoren des Polypeptids eignen.

Eine Verwendung der mit den erfindungsgemäßen Verfahren identifzierten Inhibitoren von Polypeptiden mit der biologischen Funktion einer Glyoxaloxidase ist umgekehrt auch für den Materialschutz von Interesse. Pilze sind gerade bei der Erhaltung von Hölzern ein großes Problem. Da die Glyoxaloxidasen Wasserstoffperoxid für die Ligninperoxidase zur Verfügung stellen, werden mit ihrer Hilfe auch die inertesten Bestandteile von Holz abgebaut. Die Inhibition der Glyoxaloxidase mit hierin beschriebenen Inhibitoren inhibiert in der Folge jedoch auch die Ligninperoxidasen, wodurch die Zersetzung von Hölzern im Innen- und Außenbereich vermindert oder aufgehalten werden kann.

Pilze lassen sich auf unterschiedlichste Art transformieren. Neben der Protoplastentransformation (siehe Bsp. 2 und Schulz *et al.,* 1990) stehen hierfür weitere gängige Methoden zur Verfügung. Für Hefen wird gerne die Li-Acetat Methode verwendet (Gietz *et al.,* 1997). Dabei werden die Hefezellen für die DNA Aufnahme chemisch kompetent gemacht. Bei der Elektroporation wird die geladene DNA durch einen Stromimpuls in die Zellen eingebracht. Eine weitere Methode ist die Transformation durch *Agrobacterium tumefaciens.* Dieses Bakterium kann DNA ausgehend von Plasmiden in Fremdorganismen einschleusen. Werden heterologe Sequenzen auf dieses Plasmid eingebracht, so erfolgt die Transformation der Zielzelle.

Die nachfolgenden Beispiele zeigen nun, dass die hierin beschriebenen Polypeptide überraschenderweise ein für die Pathogenität essentielles Enzym in Pilzen darstellen, und zeigen weiter, dass das Enzym ein geeignetes Zielprotein für die Identifizierung von Fungiziden ist, in Verfahren zum Identifizieren von fungizid wirksamen Verbindungen verwendet werden kann und dass die in entsprechenden Verfahren identifizierten Modulatoren der Glyoxaloxidase als Fungizide verwendet werden können.

Weiterhin wird beispielhaft ein Verfahren zur Messung der enzymatischen Aktivität von Glyoxaloxidasen beschrieben, das in Verfahren zum Identifizieren von Modulatoren des Enzyms verwendet werden kann (Beispiel 10 und 22), wobei die erfindungsgemäßen Verfahren zum Identifizieren von Fungiziden nicht auf das angegebene Verfahren beschränkt sind.

Die folgenden Beispiele sind ebenfalls nicht beschränkt auf *Ustilago maydis* oder *Botrytis cinerea.* Analoge Verfahren und Ergebnisse werden auch in Zusammenhang mit anderen Pilzen erhalten.

### Beispiele

### Beispiel 1

### Isolierung der für Glyoxaloxidase aus U. maydis kodierenden Nukleinsäure ("plasmid rescue")

Der so genannte "plasmid rescue" wurde wie in Bölker *et al.,* 1995 beschrieben durchgeführt. Die genomische DNA aus *U. maydis* wurde mit *Mlu*I geschnitten, religiert und durch Elektroporation in den *E. coli* Stamm DH5□transformiert.

### Kultivierung von U. maydis

Die Stämme wurden bei 28°C in PD-Medium oder YEPS-Medium (Tsukada et al., 1988) angezogen. Die Bildung dikaryotischer Filamente wurde nach dem Auftropfen von Stämmen auf PD-Plattenmedien, die 1% Charcoal enthielten, beobachtet (Holliday, 1974). Pathogenitätstests wurden wie beschrieben durchgeführt (Gillessen et al., 1992). Übernachtkulturen der Stämme wurden in einer Konzentration von 4x10⁷ Zellen resuspendiert und jungen Maispflanzen (Gaspar Flint) injiziert. Für jeden Stamm bzw. jede Stammkombination wurden mindestens 80 Pflanzen infiziert und auf Anthocyanbildung und Tumorbildung nach 7 bis 21 Tagen untersucht.

### Bildverarbeitung

Die Morphologie einzelner Zellen von *Ustilago maydis* wurde unter Verwendung eines Zeiss Axioskop durch das so genannte "Differential Interferenz Kontrast Verfahren" analysiert. Die Zellen wurden durch das Mikroskop fotografiert (Kodak T-64, Vergrößerungsfaktor 1000).

### Beispiel 2

### Herstellung von glo1 und glo2 Knock-out Mutanten in U. maydis

### Herstellung der Knock-out Kassette

Molekularbiologische Standardmethoden wurden nach Sambrook *et al.,* 1989 durchgeführt. Um *glol*-Nullmutanten herzustellen, wurde die 5' und die 3' Flanke des glol-Gens mittels PCR amplifiziert. Als Template diente geonomische DNA des Stammes UM518. Für die 5' Flanke (1151bp) wurden die Primer LB2 mit der Sequenz 5'-cacggcctgagtggccggtgtgtaaacgatcctttctggaag-3' und LB1 mit der Sequenz 5'-cctccaagtttcgagatatcgacc-3' eingesetzt. Für die 3'-Flanke (1249bp) wurden die Primer RB1 (5'-gtgggccatctaggccgtcaacagcaccaaattcacagcc-3') und RB2 (5'-atcgtagctcgagtgtatgcttcc-3') verwendet. Mit den Primern LB2 und RB1 wurden die Schnittstellen *Sfi* I (a) und *Sfi* I (b) eingeführt. Die Amplikons wurden mit *Sfi* I restringiert und mit dem aus dem Vektor pBS-isolierten 1884 bp großen *Sfi* I-Fragment (HygromycinB-Kassette) ligiert. Durch eine PCR mit den Primern LB1 und RB2 wurde die 4300 bp große *glol*-Knock-out Kasette amplifiziert (Kämper und Schreier, 2001).

### Herstellung von Protoplasten von U. maydis

50 ml einer Kultur in YEPS Medium wurden bei 28°C bis zu einer Zelldichte von ca. 5x10⁷/ml (OD 0.6 bis 1.0) angezogen und dann für 7 min. bei 2500g (Hereaus, 3500 rpm) in 50 ml Falconröhrchen abzentrifugiert. Das Zellpellet wurde in 25 ml SCS-Puffer (20 mM NaCitrat pH 5.8, 1.0 M Sorbit, (20 mM NaCitrat/1.0 M Sorbit und 20 mM Zitronensäure/1.0 M Sorbit mischen und mit pH-Meter auf pH 5.8 einstellen)) resuspendiert, erneut 7 min. bei 2500 g (3500 rpm) zentrifugiert und das Pellet in 2 ml SCS-Puffer, pH 5,8, mit 2,5 mg/ml Novozym 234 resuspendiert. Die Protoplastierung erfolgte bei Raumtemperatur und wurde mikroskopisch alle 5 min. verfolgt. Die Protoplasten wurden dann mit 10 ml SCS-Puffer gemischt und bei 1100 g (2300 rpm) 10 min. zentrifugiert, der Überstand wurde verworfen. Das Pellet wurde vorsichtig in 10 ml SCS Puffer resuspendiert und erneut zentrifugiert. Der Waschvorgang mit SCS-Puffer wurde zweimal wiederholt, das Pellet wurde in 10 ml STC-Puffer gewaschen. Schließlich wurde das Pellet in 500 µl kaltem STC-Puffer (10 mM Tris/HCl pH 7.5, 1.0 M Sorbit, 100 mM CaCl₂) resuspendiert und auf Eis gehalten. Aliquots können mehrere Monate bei -80°C gelagert werden.

### Transformation von U. maydis

Die Transformation von *U. maydis* erfolgte nach Schulz *et al.,* 1990. Die Isolierung genomischer *U. maydis* DNA erfolgte wie bei Hoffmann und Winston 1987 beschrieben.

Dazu wurden max. 10 µl DNA (optimal 3-5 µg) in ein 2 ml Eppendorfröhrchen übertragen, 1 µl Heparin (15 µg/µl) (SIGMA H3125) zugegeben und dann 50 µl Protoplasten zugesetzt und 10 min. auf Eis inkubiert. 500 µl 40% (w/w) PEG3350 (SIGMA P3640) in STC (steril filtriert) wurden zugesetzt, vorsichtig mit der Protoplastensuspension gemischt und 15 min. auf Eis inkubiert. Das Ausplattieren erfolgte auf Gradienten-Platten (unterer Agar: 10 ml YEPS-1,5% Agar-1M Sorbitol mit Antibiotikum; kurz vor dem Ausplattieren wurde die untere Agarschicht mit 10 ml YEPS-1,5% Agar-1M Sorbitol überschichtet, die Protoplasten ausgestrichen und die Platten für 3-4 Tage bei 28°C inkubiert.

Für die Southernanlyse wurde die DNA mit *Eco*RI und *Xho*I restringiert. Die Detektion erfolgte mit einem 1249 bp langen PCR-Fragment (RB1/RB2) als DNA-Sonde, das Digoxigenin (Roche) markiert war.

### Beispiel 3

### Überproduktion von Glo1

Für die Überproduktion von Glo1 wurde ein 3400 bp Fragment, welches das *glo1-*Gen enthielt, mit den Primern 5'glol (5'-cccgggatgacgaggcacctctcctcatc-3') und 3'glolNot (5'-gcggccgcgaattggtcagacgaatccg-3') amplifiziert. Das Amplikon wurde in den Vektor pCR-Topo2.1 (Invitrogen) kloniert. Das *glo1* Fragment wurde durch die Restriktion mit *Sma*I und *Not*I reisoliert und in die entsprechenden Schnittstellen von pCA123 kloniert. pCA123 ist ein aus dem Plasmid potef-SG (Spellig et al., 1996) gewonnenes Plasmid, wobei aus potef-SG der otef promotor als 890 bp langes *Pvu*II/*Nco*I-Fragment isoliert und in den *Pvu*II/*Nco*I geschnittenen Vektor pTEF-SG (Spellig et al., 1996) ligiert wurde. Im resultierenden Plasmid wurde das SGFP Gen durch eine Restriktion mit *Nco*I/*Not*I herausgeschnitten und durch das *Nco*I/*Not*I geschnittenes EGFP-Allel aus pEGFP-N1 (Fa. Clontech) ersetzt. Das resultierende Plasmid heißt pCA123. Das schließlich aus pCA 123 resultierende Plasmid pCA929 wurde mit *Ssp*I linearisiert und in *U. maydis* transformiert. Der verwendete *U. maydis* Stamm ist in der öffentlichen Sammlung der Deutschen Sammlung von Mikroorganismen und Zellkulturen in Braunschweig unter der Stamm-Nummer UM 521 zugänglich. Die Transformanden wurden mit dem Konstrukt glol-1 transformiert und auf cbx-Resistenz selektioniert (Keon *et al.,* 1991).

Der so erhaltene Stamm *Ustilago maydis* BAY-CA95 kann zur Überproduktion des erfindungsgemäßen Polypeptids Glo1 genutzt werden. Er wurde unter der Nummer DSM 14 509 bei der DSMZ in Braunschweig hinterlegt.

### Beispiel 4

### Zellaufschluss und Fraktionierung des Extraktes und Testen der Enzymaktivität

Die Glyoxaloxidase Aktivität wurde in ganzen Zellen, in Zellextrakten und Membranfraktionen bestimmt.

Zellen des unter der Hinterlegungsnummer DSM 14 509 hinterlegten Stammes von *Ustilago maydis,* die Glyoxaloxidase exprimieren, wurden in Minimal Medium oder PD-Medium bis zu einer OD ₆₀₀ₙₘ von 0,6 bis 3 gezogen, abzentrifugiert und durch Resuspendieren auf eine OD₆₀₀ₙₘ von 20 eingestellt. Zellextrakte wurden durch Mörsern in flüssigem Stickstoff gewonnen. Alle folgenden Schritte wurden bei 4°C durchgeführt. Durch fraktionierte Zentrifugation bei 5000 rpm und 8000 rpm wurden Zellreste und Zelltrümmer abgetrennt. Membranen wurden durch Zentrifugieren bei 13000 rpm für 45 min isoliert. Der Membranniederschlag wurde in 50 mM Tris/HCl-Puffer pH 8 mit 0,5 % Tween-20 resuspendiert.

Die Messung der Aktivität von Glo1 kann durch die Kopplung der enzymatischen Reaktion mit Phenolrot und Peroxidase erfolgen. Die Glyoxaloxidase-Aktivität wurde durch die Kopplung an eine Meerrettich Peroxidase (HRP) Reaktion mit Phenolrot als Substrat nachgewiesen. Das Assayvolumen von 50 µl besteht dabei aus 10 µl Probe, 15 µl 50mM Kalium-Phosphat-Puffer pH 6, 5µl einer 100 mM Methylglyoxal-Lösung, 5 µl HRP (190 U/ml) und 5 µl einer 56 mM Phenolrot-Lösung (Kersten und Kirk 1987). Nach einer 4 stündigen Inkubation bei 28°C, wurde NaOH bis zu einer Konzentration von 0,5 M zugegeben. Die Absorption A₅₅₀ₙₘ wurde in einem "Tecan plus reader" bestimmt. Aktives Enzym wird anhand der Entfärbung von Phenolrot identifiziert.

Substanzen oder Substanzgemische, die die Aktivität des Enzyms beeinflussen, können durch einen Vergleich der Aktivität des Enzyms bei An- und Abwesenheit dieser Probensubstanz unter Einbeziehung entsprechender Kontrollen identifizieren werden.

Im vorstehend beschriebenen Verfahren, in dem das Substrat Methylglyoxal verwendet wurde, können auch andere Substrate der Glyoxaloxidase verwendet werden. Neben ganzen Zellen können wiederum Zellmembranfraktionen eingesetzt werden. Zu den nutzbaren Substraten gehören beispielsweise auch Formaldehyd, Acetaldehyd, Glycolaldehyd, Glyoxal, Glyoxalat, Glycerinaldehyd, Dihydroxyaceton, Hydroxyaceton und Glutaraldehyd, wobei die Menge des entstehenden H₂O₂ bei ansonsten gleichen Bedingungen nicht unbedingt gleich sein muss.

### Beispiel 5

### Isolierung der für Glyoxaloxidase aus B. cinerea kodierenden Nukleinsäure

### Venvendete Stämme

Der Wildtypstamm B05.10 wurde zur Analyse, Transformation und als Wildtyp-Vergleichsstamm verwendet. B05.10 ist ein Derivat des Stammes SAS56 (van der Vlugt-Bergmans et al, 1993).

### Anzucht auf Agar-Platten

*B. cinerea* wurde bei 20°C bei Dunkelheit auf Platten angezogen, die Malz Agar von Oxoid oder Czapek-Dox Agar von Oxoid (Sucrose 30.00 g, NaNO₃ 3.00 g, MgSO₄ x 7 H₂O 0.50 g, KCl 0.50 g, FeSO₄ x 7 H₂O 0.01 g, K₂HPO₄ 1.00 g, Agar 13.00 g, H₂O dest. 1000.00 ml; pH-Wert auf 7.2 einstellen), angereichert mit verschiedenen Kohlenstoffquellen, enthielten.

### Isolierung der Konidien

Zum Isolieren der Konidien (ungeschlechtliche oder asexuelle Sporen von höheren Pilzen) wurden Platten verwendet, die vollständig mit einem Myzel bewachsen waren. Um die Sporulation auf diesen Platten zu induzieren, wurden diese für 16 Stunden UV-Licht (270 nm - 370 nm) ausgesetzt. Die Konidien wurden von Platten, auf denen die Pilze sporulierten, 7 bis 14 Tage nach der Induktion mit 5 ml sterilem Wasser abgespült, das 0,05% (v/v) Tween 80 enthielt. Die Suspension wurde durch Glaswolle filtriert, einmal durch Zentrifugation (5') bei 114xg gewaschen und in sterilem Wasser resuspendiert.

### Aufbewahrung von B. cinerea Stämmen und von Knock-out Mutanten

Konidien des Wildtyps sowie von Mutanten von *B. cinerea* wurden bei -80°C in 75% (v/v) Glycerin enthaltend 12 mM NaCl eingefroren.

### Isolierung des Glyoxaloxidase Gens bcglyoxl

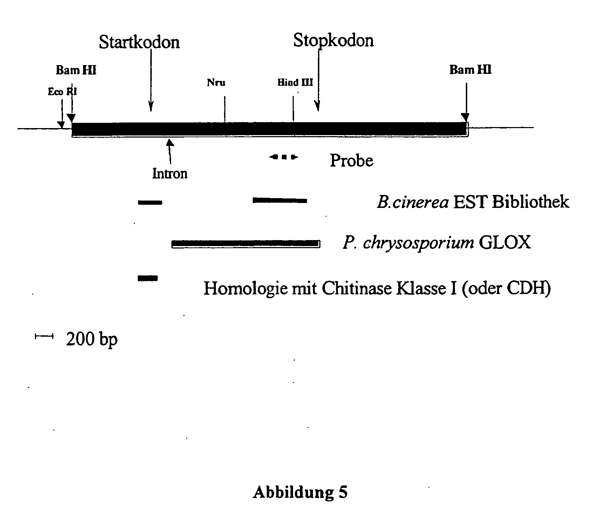

Eine genomische Bibliothek von *B. cinerea,* Stamm SAS56, in lambda EMBL3 (van der Vlugt-Bergmans et al., 1997) wurde nach Anwesenheit eines Glyoxaloxidase-Gens durchsucht. Als Sonde wurde ein 385 Basenpaare langes cDNA Fragment des Stamms T4 verwendet, das als möglicherweise homolog zur Glyoxaloxidase aus *Phanerochaete chrysosporium* identifiziert worden war. Das Fragment ist in der EMBL-Datenbank unter der Accession No. AL113811 hinterlegt. Verschiedene hybridisierende Phagen wurden gereinigt und die Phagen-DNA wurde isoliert. Von einem der Phagen wurde ein hybridisierendes 4.1 kbp *BamHI* Restriktionsfragment in ein pBluescript^{®}SKII(-) Phagemid von Stratagene kloniert und anschließend sequenziert. Die Charakteristika des klonierten Fragments sind in Abbildung 5 wiedergegeben.

### Beispiel 6

### Southern Blot Analyse der genomischen DNA

### Isolieren der genomischen DNA

Das Myzel einer Flüssigkultur wurde durch Filtration über Miracloth (Calbiochem) geerntet und gefriergetrocknet. Das getrocknete Myzel wurde in flüssigem Stickstoff homogenisiert. 3 ml TES (100mM Tris-HCl pH 8,0, 10 mM EDTA and 2% (w/v) SDS) und 60µl Proteinase K (20µg/µl) wurden zugegeben und die Suspension für eine Stunde bei 60°C inkubiert. Anschließend wurden 840µl 5M NaCl und 130µl 10% (w/v) N-Cetyl-N,N,N-trimethylarnmoniumbromid (CTAB) zugegeben und die Inkubation für 20 Minuten bei 65°C fortgesetzt. Die Suspension wurde dann durch Zugabe von 4,2 ml Chloroform/Isoamylalkohol (24:1), gefolgt von kurzem Mixen und 30 Minuten Inkubation auf Eis sowie einer anschließenden Zentrifugation für 5 Minuten bei 18 000xg weiter bearbeitet. Die obere wässrige Phase wurde abgenommen und 1350µl 7,5 M NH₄Acetat zugegeben, für eine Stunde auf Eis inkubiert und für 15 Minuten bei 18 000xg zentrifugiert. Um die DNA zu präzipitieren, wurden 0,7 Volumina Isopropanol zugegeben. Die DNA wurde mittels eines Glasstabs entnommen und in 70% (v/v) Ethanol gewaschen und getrocknet. Die genomische DNA wurde schließlich in 1 ml TE (10 mM Tris-HCl pH 7.5 und 0.1 mM EDTA, 2.5U Rnase A) gelöst, für 30 Minuten bei 50°C inkubiert und mit Ethanol gefällt.

### Southern Blot Analyse

1 µg genomische DNA wurden in einem Gesamtvolumen-von 100 µl vollständig mit dem gewünschten Restriktionsenzym geschnitten. DNA Fragmente wurden auf einem 0,8%igen (w/v) Agarosegel aufgetrennt und anschließend gemäß Protokoll für einen alkalischen Blot auf Hybond^{™}-N⁺-Membranen von Amersham geblotted. Dazu wurde das DNA enthaltende Gel zuerst in 0,25 M HCl gelegt bis die Farbstoffe ihre Farbe geändert hatten. Nach Waschen des Gels in destilliertem Wasser wurde gemäß Sambrook et al. (1989), ein Kapillar-Blot durchgeführt, wobei 0,4 M NaOH als Blotting-Lösung verwendet wurde. Nach dem Transfer der DNA wurde die Membran kurz in 2xSSC (0,3 M NaCl and 0,03 M Natriumcitrat, pH 7) gewaschen und getrocknet. Die DNA wurde durch UV-Behandlung mit der Membran vemetzt (312 nm, 0,6 J/cm²).

Mit Hilfe des "Random Primers DNA Labeling System" (Life Technologies) wurden radioaktiv markierte Sonden hergestellt. Dazu wurden 20 ng der DNA Fragmente ("Sonde", siehe Abb. 5) gemäß dem Protokoll des Herstellers markiert. Die markierten DNA Fragmente wurden über eine Sephadex G50 Säule gereinigt.

Die Hybridisierung erfolgte gemäß Church and Gilbert (1984). Dazu wurde der Blot für 30 Minuten bei 65°C in Hybridisierungspuffer (0,25 M Phosphat Puffer, pH 7,2, 1 mM EDTA, 1% (w/v) BSA and 7% (w/v) SDS) prähybridisiert. Dann wurde der Blot mit Hybridisierungspuffer, der die markierte Sonde enthielt, für 40 Stunden bei 65°C hybridisiert. Die Blots wurden drei Mal gewaschen (30 Minuten, 65°C in 2xSSC und 0,1% (w/v) SDS). Autoradiographie erfolgte mit Kodak X-OMAT AR Film.

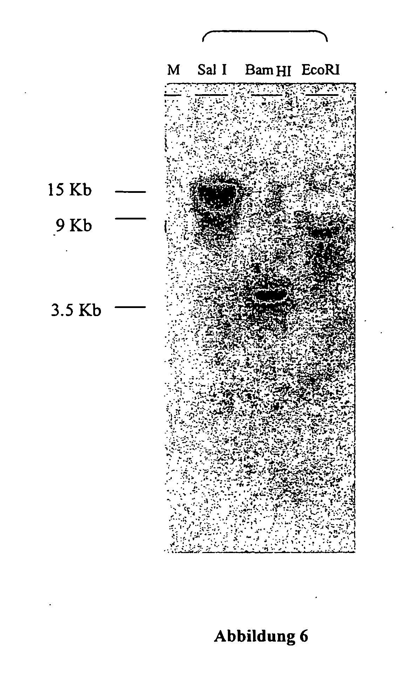

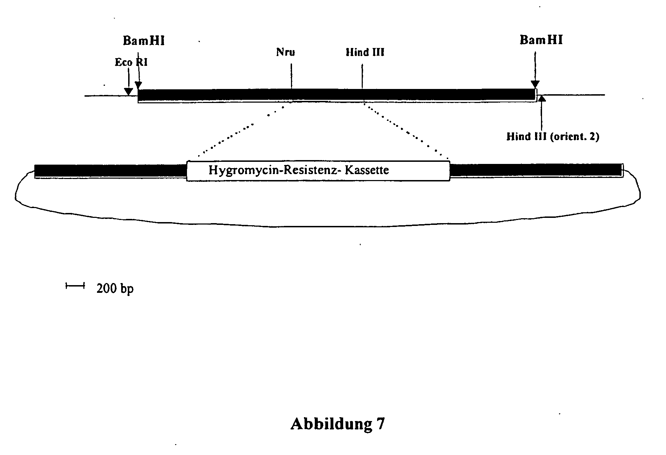

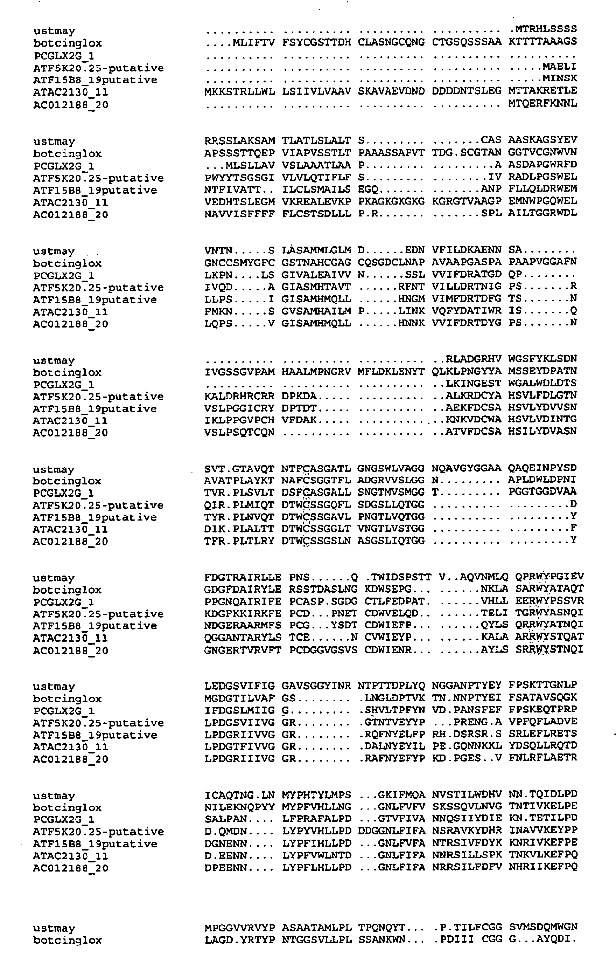

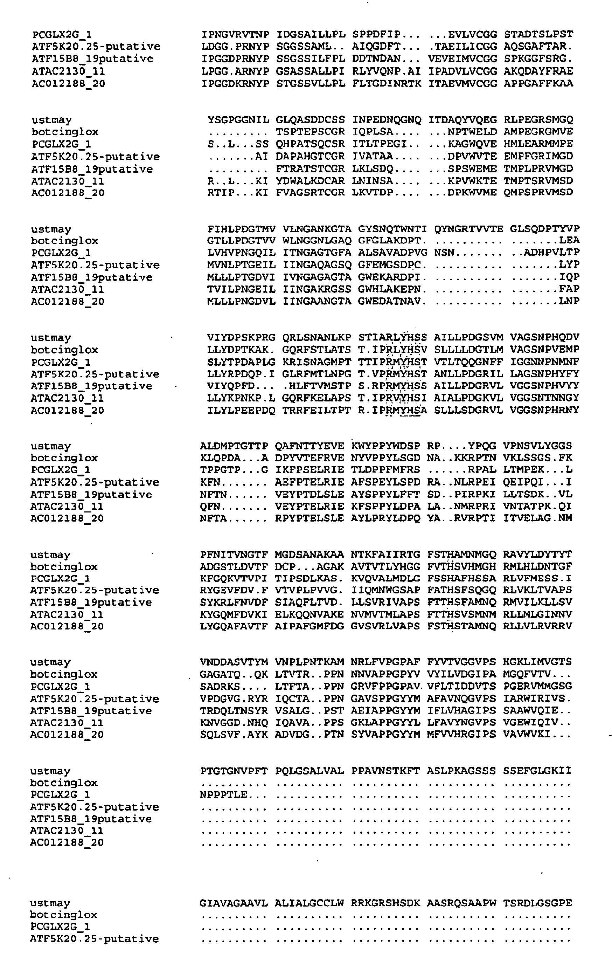

Die Ergebnisse der Hybridisierung sind in Abbildung 6 gezeigt. Mit der Sonde konnten Einzelbanden in allen drei Restriktionsansätzen identifiziert werden (*SaI*I, *Bam*HI und *Eco*RI). Das *Bam*HI-Fragment, das hybridisierte, hatte eine Größe von 4 kbp.

### Beispiel 7

### Klonierung der cDNA

Vollständige cDNA-Fragmente wurden mittels des Superscript^{™} One-Step RT-PCR Systems von Life Technologies erhalten. Dazu wurden 0,1 µg der Gesamt-RNA, die gemäß TRIzol-Protokoll unter Verwendung des TRIzol^{®} Reagenz (TRIzol Reagenzien sind monophasische Lösungen aus Phenol und Guanidiniumthiocyanat; nach der Zugabe von Chloroform und anschließender Zentrifugation wird die RNA aus der wässrigen Phase mit Isopropanol gefällt) aus *B. cinerea,* Stamm B05.10, isoliert wurde, revers transkribiert und mit Hilfe von genspezifischen Primern amplifiziert. Die cDNA wurde direkt in den Vektor pCR^{®}4-TOPO^{®} (Invitrogen) kloniert und vollständig sequenziert.

Die cDNA Sequenz bestätigt die Existenz eines Introns zwischen den Sequenzen, die für die Chitin bindende Domäne und die Glyoxaloxidase-Domäne kodieren.

### Beispiel 8

### Expression von BcGlyox1

Die Expression von *BcGlyox1* wurde anhand des zeitlichen Verlaufs der Infektion auf Tomatenblättern untersucht. Die Konidien des *B. cinerea* Stamms B05.10 wurden für 2 Stunden in B5 Medium, ergänzt mit 10 mM Glucose und 10 mM (NH₄)H₂PO₄, vorinkubiert, um die Keimung zu stimulieren. Die Blätter von Tomaten *(Lycopersicon esculentum* cultivar Moneymaker Genotyp Cf4) wurden durch Besprühen mit dem Medium das 10⁶ Sporen pro ml enthält, inokuliert. Die Blätter wurden bei 20°C und >95% Luftfeuchtigkeit inkubiert und anschließend in regelmäßigen Abständen nach der Inokulation geerntet und bei -80°C aufbewahrt.

Die RNA wurde aus dem gefriergetrockneten und in flüssigem Stickstoff homogenisierten Myzel extrahiert, indem das Blattgewebe im Mörser zu Pulver zermahlen wurde. Pro Gramm des Materials wurden 2 ml Guanidinium-Puffer pH 7,0 zugegeben. Der Puffer bestand aus 8.0 M Guanidiniumhydrochlorid, 20 mM 2-[N-morpholino]-ethansulfonsäure (MES), 20 mM EDTA und 50 mM β-Mercaptoethanol, pH 7,0. Die Suspension wurde zweimal extrahiert, einmal mit dem gleichen Volumen Phenol/Chloroform/Isoamylalkohol (IAA) (25:24:1 v/v/v) und einmal mit Chloroform/IAA (24:1 v/v). Nach Zentrifugation für 45 Minuten bei 12000xg bei 4°C wurde ein Drittel des Volumens 8 M LiCl zur wässrigen Phase zugegeben. Anschließend wurde die Suspension über Nacht auf Eis inkubiert und für 15 Minuten bei 12000xg zentrifugiert. Der Niederschlag wurde einmal mit 2 M LiCl und zweimal mit 70% (v/v) Ethanol gewaschen, an der Luft getrocknet und in 1 ml TE gelöst. Die Konzentration der RNA wurde spektrophotometrisch bei 260 nm bestimmt. Alternativ wurde auch das TRIzol^{®} Reagenz (Life Technologies) entsprechend den Angaben des Herstellers verwendet, um die RNA aus dem gefriergetrockneten Material zu gewinnen.

Zur Gelelektrophorese der Gesamt-RNA wurden die Proben wie folgt denaturiert. Zu 10 µg der Gesamt-RNA in 3,7 µl Lösung wurden 3,6 µl 6 M deionisiertes Glyoxal, 10,7 µl Dimethylsulfoxid und 2,0 µl 0,1 M Natriumphosphatpuffer, pH 7 zugegeben. Die Probe wurde für 60 Minuten bei 50°C inkubiert, kurz zentrifugiert, in flüssigem Stickstoff eingefroren und auf Eis wieder aufgetaut. Die Probe wurde auf einem 1,4 %igen (w/v) Agarosegel aufgetrennt. Gel und Laufpuffer enthielten 0,01 M Na-Phosphatpuffer, pH 7,0. Nach dem Gellauf wurden die aufgetrennten RNA-Fragmente mittels Kapillar-Blotting (Sambrook et al., 1989) auf eine Hybond^{™}-N⁺-Membran (Amersham) transferiert, wobei eine Blotting-Lösung mit 0,025 M Na-Phosphatpuffer, pH 7 verwendet wurde. Nach dem Transfer der RNA wurde die Membran getrocknet und die RNA durch UV-Behandlung (312 nm, 0,6 J/cm²) mit der Membran vemetzt. Das Protokoll für die Hybridisierung entspricht dem für die DNA-Hybridisierung angegebenen.

### Beispiel 9

### Herstellung von B. cinerea BcGlyox1 Knock-out Mutanten

### Vektorkonstruktion

Die Transformation von *B. cinerea* wurde mit einem Vektor zur homologen Rekombination, der das *BCGlyoxl-*Gen, in dem ein *Nru*I*-Hind*III Fragment deletiert und durch eine Hygromycin-Resistenz-Kassette ersetzt worden war, enthielt, durchgeführt (pHyGL YOX1, siehe Abbildung 8).

### Herstellung von Protoplasten

Um Protoplasten zur Transformation zu erhalten, wurde 1 Liter 1 % (w/v) Malz Extrakt (Oxoid) mit 2 x 10⁸ *B. cinerea* Konidien (Stamm B05.10) inokuliert. Nach 2 Stunden wurden die keimenden Konidien bei 20°C in einem Rotor-Schüttler bei 180 rpm für 24 Stunden inkubiert. Das Myzel wurde mittels eines 22,4 µm Siebes geerntet und in 50 ml KC-Lösung inkubiert, die 0,6 M KCl und 50 mM CaCl₂ enthielt, wobei 5 mg/ml Glucanex (thermostabile Beta-Glucanase zur Hydrolyse von Beta-Glucan-Polysacchariden) zugesetzt wurde. Nachdem die Protoplasten so hergestellt worden waren, wurde die Suspension durch einen 22,4 µm und einen 10 µm Sieb filtriert. Die Protoplasten wurden gewaschen und zu einer Konzentration von 10⁷ Protoplasten pro 100 µl resuspendiert.

### Transformation und Selektion von Transformanten

2 µg des Transformationsvektors pHyGLYOX1, der mit *EcoR*I geschnitten und mit Phenol extrahiert worden war, wurden in 95 µl KC verdünnt und 2 µl 5 mM Spermidin zugegeben. Nach Inkubation auf Eis für 5 Minuten wurden 100 µl der Protoplastensuspension zur DNA gegeben und alles für weitere 5 Minuten auf Eis inkubiert. 100 µl Polyethylenglycol (PEG) Lösung, enthaltend 25% (v/v) PEG 3350 in 10 mM Tris-HCl, pH 7,4, und 50 mM CaCl₂ wurden zugegeben und die Suspension gemischt. Nach 20 Minuten bei Raumtemperatur wurden 500 µl PEG zugegeben und die Gefäße für weitere 10 Minuten bei Raumtemperatur belassen. Schließlich wurden 200 µl KC-Lösung zugegeben.

Der Treansformationsansatz mit den transformierten Protoplasten wurde mit 200 ml SH-Agar gemischt und sofort auf 20 Petrischalen verteilt. SH-Agar enthält 0,6 M Saccharose, 5 mM HEPES pH 6,5, 1,2 % (w/v) gereinigten Agar und 1 mM NH₄(H₂PO₄). Nach 24 Stunden Inkubation bei 20 °C wurde ein gleiches Volumen SH-Agar enthaltend 50 µg/ml Hygromycin zugegeben. Einzelne auftauchende Kolonien wurden zur weiteren Selektion auf Malz Agar Platten transferiert, die 100 µg/ml Hygromycin enthielten. Wachsende Kolonien wurden dann auf Malz Agar Platten überführt, die kein Hygromycin enthielten, und die Sporulation durch Behandlung mit UV-Licht (nahes UV) ausgelöst. Um Monosporen-Isolate zu erhalten, wurden Konidien isoliert, verdünnt und auf Malz Agar Platten mit 100 µg/ml Hygromycin ausgestrichen. Die von diesen Platten erhaltenen Kolonien wurden isoliert und zur weiteren Analyse verwendet.

### Southern Analyse der Transformanten

Transformanten wurden einer Southern Analyse unterworfen. Die DNA wurde isoliert und mit *Eco*RV geschnitten, elektrophoretisch aufgetrennt, geblottet und mit einer Sonde hybridisiert (siehe vorne). Bei Knock-out-Transformanten sollte eine solche Hybridisierung ein 300 bp großes Fragment ergeben. Alle Transformanten mit einem langsamen Wachstumsphänotyp wiesen das 300 bp Fragment auf.

### Wachstumsanalyse der Transformanten

Alle Transformanten, die auf Platten mit hohem Hygromycin-Gehalt gewachsen waren, wuchsen auch normal auf Malz Agar Platten ohne Hygromycin. Wenn die Transformanten auf synthetischen Agarmedien angezogen wurden, die einfache Zucker als Kohlenstoffquelle enthielten, wuchsen die Transformanden langsam oder überhaupt nicht mehr. Die getesteten Zucker waren z.B. Hexosen, Pentosen und Triosen. Sowohl die Keimung als auch die Entwicklung der Hyphen waren beeinträchtigt oder völlig unterbunden. Der Wachstumsdefekt kann durch Zugabe von z.B. Trypton oder Pepton ausgeglichen werden. Die Wachstumsinhibition kann durch die Zugabe von Arginin zum Medium vollständig beseitigt werden. Konzentrationen von 100 µM Arginin und höher können das Wachstum des Pilzes, auf Medien die einfache Zucker enthalten, vollständig wiederherstellen.

### Bioassays

Ein Bioassay wurde durchgeführt, um die Virulenz von *BcGlyox1*-Mutanten mit der von Wildtyp-*B. cinerea* (Stamm B05. 10) zu vergleichen.

Abgeschnittene Blätter und Früchte von Tomaten *(Lycopersicon esculentum)* und Äpfel (Alkmene und Cox Orange) wurden mit einer Suspension von Konidien (Benito et al., 1998; ten Have et al., 1998) inokuliert. Auf die abgeschnittenen Blüten von Rosen und Gerbera-Hybriden wurden trockene Konidien aufgestäubt (van Kan et al., 1997). Das inokulierte Wirtsgewebe wurde bei 15°C bei Dunkelheit inkubiert (Tomatenblätter und -früchte, Rosen und Gerbera) oder bei 20°C und bei Licht inkubiert (Äpfel).

In allen Ansätzen waren die *BcGlyox1*-Mutanten, die getestet wurden, nicht in der Lage, primäre nekrotische Läsionen hervorzurufen, während der Wildtyp primäre Läsionen hervorrief, die sich teilweise in das Nachbargewebe ausbreiteten (siehe Abbildungen 9 bis 12).

Da die *BcGlyoxl*-Mutanten nicht wie der Wildtyp in B5-Medium bei Anwesenheit einfacher Zucker keimen (Standard-Medium), wurde die Keimung stimuliert, indem die Konidien in einem 1 %igen Malzextrakt für 2 Stunden bei Raumtemperatur vorinkubiert wurden. Dies führte zu einer effizienten Keimung von Wildtyp und Mutante. Diese präinkubierten Suspensionen wurden ebenfalls zur Inokulation verwendet, um auszuschließen, dass die Virulenz der Mutante nicht aufgrund anderer Defekte oder Mängel ausbleibt. Es zeigte sich jedoch auch in diesen Versuchen, dass die Mutanten die Testgewebe nicht infizieren können (Abb. 9 bis 12).

Schließlich wurde der Inokulationssuspension auch noch Arginin zugesetzt, um die Probleme der Mutanten mit der Nutzung einfacher Zucker auszuräumen. Die Inokulation von Äpfeln mit einer Verletzung mit Arginin-enthaltenden Suspensionen von Konidien der Mutante und des Wildtyps ergaben, dass sich in beiden Fällen nekrotisches Gewebe bildete. Die Läsionen des Wildtyps breiteten sich für einige Tage weiter aus, bis das Gewebe schließlich völlig verrottet war. Die Läsionen, die durch die Mutante hervorgerufen worden waren, breiteten sich für 2 bis 3 Tage weiter aus und kamen dann vollständig zum Erliegen.

### Beispiel 10

### Nachweis der Expression und enzymatischen Aktivität der Glyoxaloxidase

Die Aktivität der Glyoxaloxidase *in vitro* sowie *in vivo,* z.B. in den nach Beispiel 3 hergestellten erfindungsgemäßen *U. maydis-Zellen* (CA95) kann durch die Umsetzung des Substrats Methylglyoxal nachgewiesen werden, wobei folgende Reaktion genutzt wird:

### Schritt 1:

Methylglyoxal + O₂ → Pyruvat + H₂O₂

### Schritt 2:

H₂O₂ + 10-Acetyl-3,7-dihydroxyphenoxazin (Amplex Red^{®}) → Resorufin + H₂O

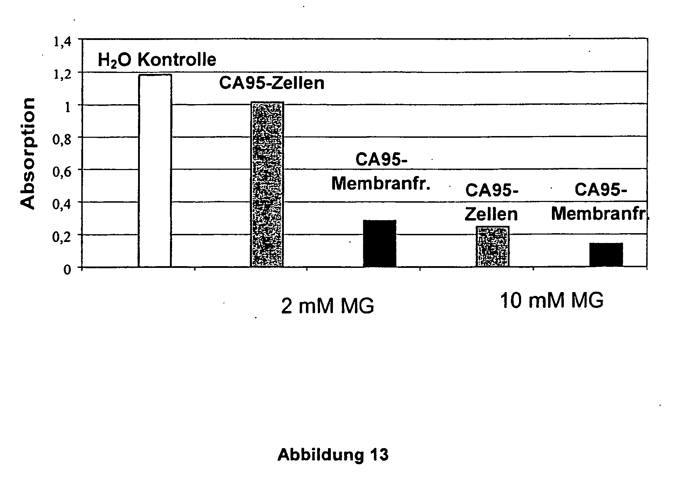

Amplex Red^{®} reagiert in einer 1:1 Stöchiometrie mit H₂O₂, wobei das stark fluoreszierende Resorufin (7-hydroxy-3H-phenoxazin-3-on, , Natriumsalz) entsteht. Die Messung der Fluoreszenz erfolgt bei einer Anregungswellenlänge von 550 nm und einer Emission von 595 nm. Im Test wurde eine Substratkonzentration von 10 mM Methylglyoxal eingesetzt. Bei der Verwendung ganzer Zellen ist für Dauer der Reaktion zu beachten, dass die Glyoxaloxidase Konzentration gering ist und die Reaktion deshalb länger laufen muss. So wurden z.B. sehr gute Messergebnisse nach einer Inkubation von 9 Stunden erzielt. Bei einer Konzentration von 1mM Methylglyoxal konnte keine Reaktion im gegebenen Zeitfenster beobachtet werden. Durch Zugabe von 100 mM Methylglyoxal wurde nur eine geringe Steigerung des Umsatzes erreicht, während die Steigerung der Umsatzrate von 2mM auf 10 mM Methylglyoxal im linearen Bereich der Kinetik liegt (Abbildung 13).

### Beispiel 11

### Enzymtest zum Identifizieren von Inhibitoren

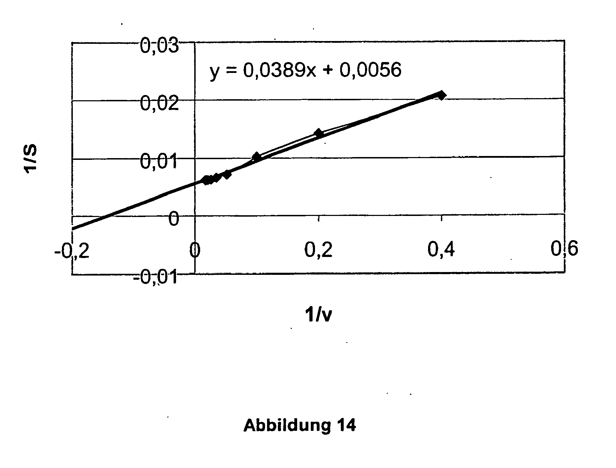

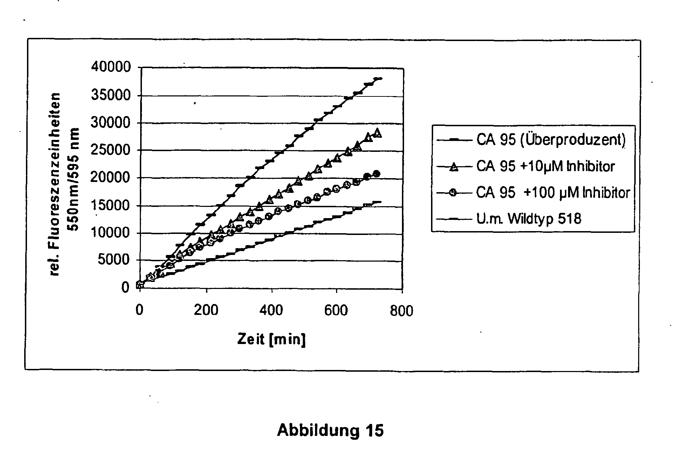

Der Enzymtest wurde in einem Gesamtvolumen von 50 µl durchgeführt. Dazu wurden die zu testenden Substanzen in 10 µl Substratlösung (50 mM Methylglyoxal, 2,5 % (v/v) DMSO) in einer 384-Mikrotiterplatte vorgelegt. Vorab war der K_{M}-Wert der Glyoxaloxidase für Methylglyoxal bestimmt worden (vgl. Abb. 14). Die Konzentration der auf eine inhibitorische Wirkung zu testenden Kandidatenverbindungen wurde so bemessen, dass die Endkonzentration der Substanzen im durchgeführten Test 10 µM betrug. Im nächsten Schritt wurden 20 µl Zell-Lösung (Zellen des Überproduzenten-Stammes Bay-CA95 (OD ₆₀₀=5); 0,2 M 2,2-Dimethylsuccinat-Puffer, pH 5, gekühlt bei 4°C) zugegeben. Zu diesem Ansatz wurden 20 µl Detektionslösung (125 µM Amplex Red^{®}-Reagens (20 mM Stammlösung in 100 % DMSO), 2,5 u/ml Horseradish peroxidase (Meerrettichperoxidase), 62,5 mM Natriumphosphat-Puffer, pH 7,4) zugegeben. Der Ansatz wurde 9 h bei 30°C inkubiert. Anschließend wurde die Zunahme der Fluoreszenz bei λ= 550 nm (Extinktion) und λ=595 nm (Emission) gemessen, wobei die Ergebnisse einer Messung in Anwesenheit von Bay-CA95-Zellen mit den Ergebnissen einer Messung in Anwesenheit der Wildtyp *U.maydis* 518-Zellen verglichen wurden (siehe auch Abbildung 15). Die im Test verwendeten Substanzen lagen in folgenden Endkonzentrationen vor: c(2,2-Dimethylsuccinat/NaOH) = 40 mM, c(Amplex Red® (Molecular Probes)) = 50 µM, c(Horseradish Peroxidase) = 0,001 u/µl, c(Methylglyoxal) = 10 mM, OD (Bay-CA95) = 1, c(Natriumphosphat-Puffer) = 25 mM. Die inhibitorische Wirkung einer Kandidatenverbindung konnte an der Abnahme der relativen Fluoreszenz festgestellt, und Inhibitoren identifiziert werden. In Tabelle II werden beispielhaft Verbindungen gezeigt, die als Inhibitoren der Glyoxaloxidase wirksam sind. In Tabell II werden auch pI50-Werte angegeben, die für die einzelnen Verbindungen bestimmt wurden. Der pI50-Wert ist der negative dekadische Logarithmus des so genannten IC50-Werts, der die molare Konzentration einer Substanz angibt, die zur 50 %igen Hemmung des Enzyms führt. Ein pI50-Wert von 8 entspricht z.B. einer halbmaximalen Hemmung des Enzyms bei einer Konzentration von 10 nM. In Abbildung 15 wird beispielhaft der Einfluss einer Verbindung (Tab. II, Bsp. 3) auf die Aktivität der Glyoxaloxidase gezeigt.

**Tabelle II**

| **Bsp.** | **Strukturformel** | **pI50** |
|---|---|---|
| 1 | 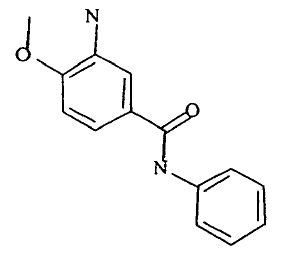 | 4,96 |
| 2 | 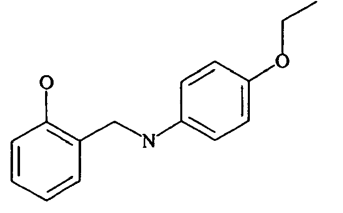 | 5,39 |
| 3 | 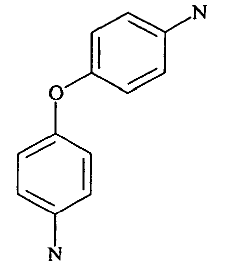 | 5,25 |
| 4 | 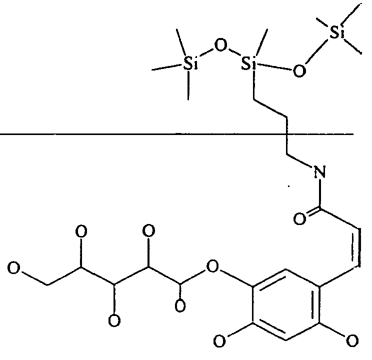 | 5,42 |
| 5 | 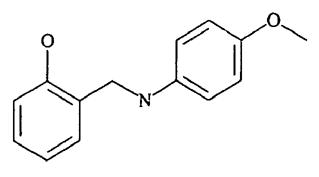 | 5,4 |

### Beispiel 12

### Nachweis der fungiziden Wirkung der identifizierten Inhibitoren der Glyoxaloxidase

Die Wirkung der Verbindungen gegen Pilze (protektive Wirkung) wurde unter anderem am Beispiel von *Venturia inaequalis* getestet. Dieser Pilz verursacht den so genannten Apfelschorf, der zu schwarzgrün gefleckten Blättern bei Kernobstbäumen führt. Die Flecken vergrößern sich und fließen zusammen. Blätter, die stark befallen sind, sterben ab, was bis zur Verkahlung der Bäume im Sommer führen kann. Eine Infektion wirkt sich auch negativ auf den Fruchtansatz aus. Schorf an Früchten äußert sich in grauen Flecken auf der Fruchthaut mit verkorkten Stellen und deformierten Früchten.

Zur Herstellung einer zweckmäßigen Wirkstoffzubereitung vermischt man 1 Gewichtsteil Wirkstoff mit z.B. 24,5 Gewichtsteilen Aceton und 24,5 Gewichtsteilen Dimethylformamid und 1,0 Gewichtsteilen Alkyl-Aryl-Polyglykolether als Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Konidiensuspension des Apfelschorferregers *Venturia inaequalis* inokuliert und verbleiben dann 1 Tag bei ca. 20°C und 100 % relativer Luftfeuchtigkeit in einer Inkubationskabine.

Die Pflanzen werden dann im Gewächshaus bei ca. 21°C und einer relativen Luftfeuchtigkeit von ca. 90 % aufgestellt.

1 bis 12 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Bei einer Konzentration von 250 ppm zeigte die Verbindung gemäß Bsp. 4 (Tab. I) einen Wirkungsgrad von 45 %.

### Abbildungen und Sequenzprotokoll

### Abbildung 1

**Bestimmung der Homologie** zwischen den hierin beschriebenen Glyoxaloxidasen Glo1, Glo2 und Glo3 aus *U. maydis* gemäß SEQ ID NO:1 bzw. SEQ ID NO:3, der Gyloxaloxidase aus *B. cinerea* und der bekannten Gyloxaloxidase aus *Phanerochaete chrysosporium* (BESTFIT). Die Ähnlichkeit von Glo1 aus *U. maydis* und der Gyloxaloxidase aus *P. chrysosporium* beträgt 44 %, die Identität 38 %. Die konservierten Positionen, die für die Koordinierung des Kupferions von Bedeutung sind, sind grau unterlegt.

### Abbildung 2

**(A) Southernanalyse zur Identifizierung von glo1-Nullmutanten.** Je 1 µg genomische DNA der jeweils angegebenen Ustilago-Stämme wurde mit Eco RI und XhoI geschnitten, auf einem 1%-igen Agarosegel getrennt und geblottet. Die Hybridisierung erfolgte mit einer Digoxigenin markierten DNA-Sonde (1200 bp; PCR-Fragment mit den Primern RB1/RB2, wie angegeben in Abb. 2B). Die in den einzelnen Spuren aufgetragene DNA wurde aus folgenden Stämmen isoliert:
   Spur 2: Wildtyp Um 518; Spur 3: WildtypUm521; Spur 4-8: Transformanten von Um 518 (518#0, 518#1, 518#4, 518#6, 518#8); Spur 9-13: Transformanten von Um 521 (521#1, 521#5, 521#7, 521#8, 521#9). Als Größenstandard diente der 1 kb plus DNA-Marker in Spur 1.
**(B) Schematische Darstellung der homologen Rekombination zur Generierung von *glol* Nullmutanten.** Die Primer RB1 und RB2 definieren das PCR-Produkt, das für die Hybridisierung als DNA-Sonde eingesetzt wurde (siehe auch Kämper und Schreier (2001)).

### Abbildung 3

***glo1*-Nullmutanten zeigen einen pleiotropen Morphologiedefekt.** Die jeweiligen Kulturen wurden bis zu einer OD₆₀₀ von 0,8 in PD-Medium hochgezogen, in H₂O gewaschen und anschließend in einer 0,2% Kelzan (Bayer-AG)-Lösung resuspendiert. Großbuchstaben kennzeichnen Nullmutanten, Kleinbuchstaben Wildtypen. A, b, c, F, G, J und K sind Um518 Stämme bzw deren Derivate; c, d, e, H, J, L und M sind Um521-Stämme und deren Derivate.
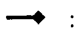 →: Budnecks in Wildtypzellen; → : zusätzliche Septen; : Y-Verbindungen, keine Zytokinese; 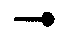 Zellen mit runder Morphologie. Auffällig ist des weiteren die starke Vakuolisierung, die Elongation und Deformation der Mutantenzellen. Der angegebene Größenstandard entspricht 3 µm.

### Abbildung 4

**Phenotyp der (Delta)*glo1* Stämme.** Das (Delta)*glo1*-Allel wurde in die *U. maydis* Stämme Um521 (alb1) und Um518 (a2B2) eingebracht. Alle Stämme wurde entweder allein oder in den angegebenen Kombinationen auf PD-Charcoal Plattenmedien getropft. Nach 48 h Inkubation kann eine erfolgreiche Paarung am Auftreten des weißen Luftmycels erkannt werden.

### Abbildung 5

**Die Hauptcharakteristika der *BcGlyox1* Sequenz aus *B. cinerea.*** Die Proteinsequenz von BcGLYOX1 enthält eine putative Signalpeptid-Spaltstelle, gefolgt von einer kurzen Sequenz mit einer Homologie mit einer Polysaccharid-Bindungsdomäne, die in Pflanzenproteinen gefunden werden kann (z.B. in Typ I Chitinasen, Lektinen). Diese Domäne geht der katalytischen Domäne voran, die eine Homologie zum *P. chrysosporium* Gen kodierend für die Glyoxaloxidase sowie zum Gen kodierend für Galactoseoxidasen (aus *Dactylium dendroides*) aufweist. Das *BcGlyoxl* Gen enthält auch die ungewöhnliche Cu²⁺-Bindungsstelle, die für die Glyoxaloxidase aus *P. chrysosporium* typisch ist. Die Schnittstellen, die bei der Isolierung des Gens verwendet wurden, sind ebenfalls angegeben. Ein aufgefundenes Intron wurde ebenso markiert wie die Position des bei der Isolierung verwendeten Fragments aus *B. cinerea* und der für die Southernanalyse verwendeten DNA-Sonde.

### Abbildung 6

**Southern Blot mit genomischer DNA** von *B. cinerea* (Stamm B05.10), geschnitten mit drei unterschiedlichen Restriktionsenzymen wie in der Abbildung angegeben. Die restringierte DNA wurde mit einem radioaktiv markierten 385 bp langen Fragment aus *B. cinerea* hybridisiert.

### Abbildung 7

**Herstellung des für die Generierung von Knock-out Mutanten verwendeten Vektors pHyGLYOX1** enthaltend eine Hygromycin-Resistenz-Kassette, die ein *Nru*I*-Hind*III Fragment des Ausgangsvektors ersetzt.

### Abbildung 8

**Sequenzvergleich (Alignment) zwischen den für Glyoxaloxidase kodierenden Sequenzen** bzw. Sequenzfragmenten aus *Ustilago maydis* (Ustmay), *Botrytis cinerea* (botcinglox), *Phanerochaete chrysosporium* (PCGLX2G_1) sowie verschiedener putativer ORFs (kodierende für Glyoxaloxidase) aus *Arabidopsis thaliana* (ATF5K20.25-putative, ATF15B8_19putative, ATAC2130_11, AC012188_20). Interessante konservierte Aminosäuren sind beispielhaft grau unterlegt.

### Abbildung 9

### Apathogenität der Knock-out Mutanten.

Abgeschnittene Äpfel (Alkmene und Cox Orange) wurden mit einer Suspension von *B. cinerea* Konidien (siehe Beispiel 9) inokuliert. Das inokulierte Wirtsgewebe wurde bei 20°C und Licht inkubiert. Die *BcGlyoxl*-Mutanten (Knock-out Mutanten), die getestet wurden, waren nicht in der Lage, primäre nekrotische Läsionen hervorzurufen (Abb. 9, A4a und R3a), während der Wildtyp primäre Läsionen hervorrief (Abb. 9, B05.10), die sich teilweise in das Nachbargewebe ausbreiteten. Bei den mit Malzextrakt präinkubierten Suspensionen (vgl. Bsp. 9) zeigte sich ebenfalls, dass die Mutanten die Testgewebe nicht infizieren können.

### Abbildung 10

### Apathogenität der Knock-out Mutanten.

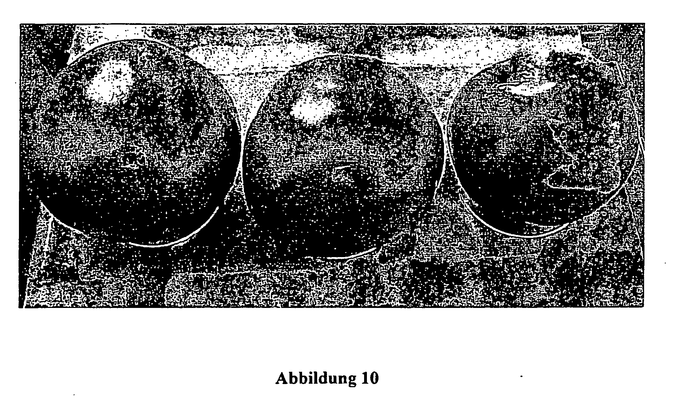

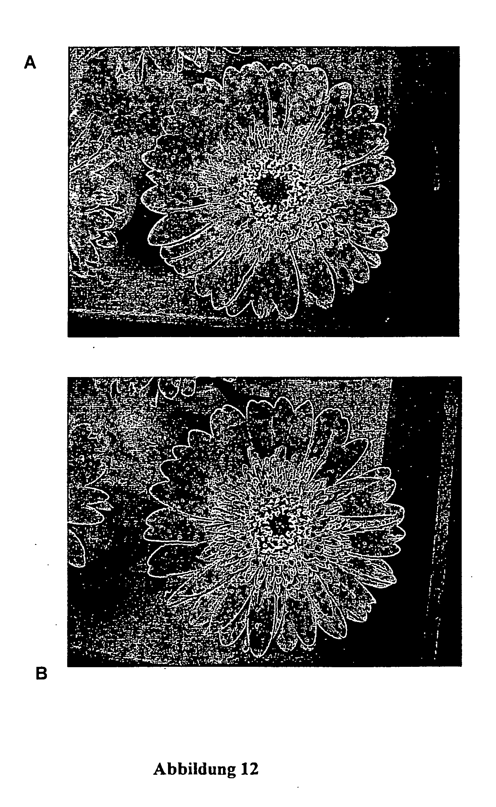

Abgeschnittene Tomaten *(Lycopericon esculentum)* wurden mit einer Suspension von *B. cinerea* Konidien (siehe Bsp. 9) inokuliert. Das inokulierte Wirtsgewebe wurde bei 15°C bei Dunkelheit inkubiert. Die *BcGlyoxl-*Mutanten (Knock-out Mutanten), die getestet wurden, waren nicht in der Lage, primäre nekrotische Läsionen hervorzurufen (Abb. 10, Tomate links, A4a, und in der Mitte, R3a), während der Wildtyp B05.10 primäre Läsionen hervorrief (Abb. 12, rechte Tomate), die sich teilweise in das Nachbargewebe ausbreiteten.

### Abbildung 11

### Apathogenität der Knock-out Mutanten.

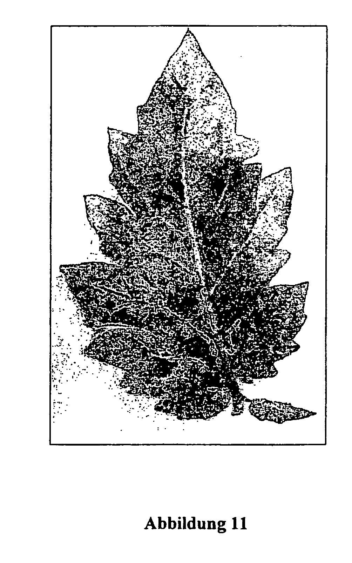

Ein abgeschnittenes Blatt von Tomaten *(Lycopericon esculentum)* wurde auf jeweils einer Seite mit einer Suspension von *B. cinerea* Konidien (siehe Bsp. 9) inokuliert. Das inokulierte Wirtsgewebe wurde bei 15°C bei Dunkelheit inkubiert. Die *BcGlyox1*-Mutanten (Knock-out Mutanten), die getestet wurden, waren nicht in der Lage, primäre nekrotische Läsionen hervorzurufen (Abb. 11, rechte Blatthälfte), während der Wildtyp primäre Läsionen hervorrief (Abb. 11, linke Blatthälfte), die sich in das Nachbargewebe ausbreiteten.

### Abbildung 12

### Apathogenität der Knock-out Mutanten.

Auf die abgeschnittenen Blüten von Gerbera-Hybriden wurden trockene *B. cinerea* Konidien aufgestäubt (siehe Bsp. 9). Das inokulierte Wirtsgewebe wurde bei 15°C bei Dunkelheit inkubiert. In allen Ansätzen waren die *BcGlyoxl*-Mutanten, die getestet wurden, nicht in der Lage, primäre nekrotische Läsionen hervorzurufen (Abb. 12A), während der Wildtyp primäre Läsionen hervorrief, die sich teilweise in das Nachbargewebe ausbreiteten (Abb. 12B).

### Abbildung 13

### Vergleich des Umsatzes von Methylglyoxal durch die Glyoxaloxidase in Abhängigkeit von verschiedenen Substratkonzentrationen.

In CA95-Zellen (*U. maydis* Stamm BAY-CA95, vgl. Bsp. 3), in denen es zur Überproduktion von Glo1 kommt, konnte die Expression von Glo1 an ganzen Zellen durch die enzymatische Umsetzung von Methylglyoxal (MG) nachgewiesen werden (vgl. Bsp. 10). Im Test wird eine Substratkonzentration von 10 mM Methylglyoxal eingesetzt. Bei einer Konzentration von 1mM Methylglyoxal konnte keine Reaktion im gegebenen Zeitfenster beobachtet werden. Durch Zugabe von 100 mM Methylglyoxal wurde nur eine geringe Steigerung des Umsatzes erreicht, während die Steigerung der Umsatzrate von 2mM auf 10 mM Methylglyoxal im linearen Bereich der Kinetik liegt. Der Test wurde sowohl mit ganzen Zellen als auch mit Zellfragmenten (Membranfraktion) durchgeführt.

### Abbildung 14

### Lineweaver-Burk-Diagramm zur K_{M}-Wert-Bestimmung der Glyoxaloxidase für Methylglyoxal.

Der Test wurde kontinuierlich durchgeführt, durch Kopplung der Reaktion mit MeerrettichPeroxidase (vgl. Bsp. 10). Die Umsetzung von Amplex Red® (Molecular Probes) wurde fluorimetrisch verfolgt (λ(exc)=550 nm; λ(em)=595 nm). Das Reaktionsvolumen betrug 50 µl. Die Geschwindigkeit der Umsetzung wurde nach ca. 180 min Inkubationszeit (Lag-Phase) und nach Abzug des Blindwertes bestimmt.

### Abbildung 15

### Inhibition von Glo1 durch Zusatz eines erfindungsgemäßen Inhibitors.

Die Glo1-Aktivität wurde mittels eines gekoppelten Testsystems mit dem Nachweisreagens Amplex Red® wie in Bsp. 10 beschrieben durchgeführt. Als Kontrolle wurden anstelle von Bay-CA95-Zellen (CA95) *U.maydis* Wildtyp 518 Zellen verwendet. Eine der im erfindungsgemäßen Verfahren identifizierte Verbindungen (Tab. II, Bsp. 3) (Inhibitor) wurde in zwei verschiedenen Konzentrationen 10 µM und 100 µM eingesetzt.

### SEQ ID NO:1

Nlukleinsäuresequenz kodierend für die Glyoxaloxidase Glo1 aus *U. maydis* (cDNA).

### SEQ ID NO:2

Aminosäuresequenz der von der Sequenz gemäß SEQ ID NO:1 kodierten Glyoxaloxidase Glo1 aus *U. maydis.*

### SEQ ID NO:3

Nukleinsäuresequenz kodierend für die Glyoxaloxidase Glo1 aus *U. maydis* (genomische DNA).

### SEQ ID N0:4

Aminosäuresequenz der von der Sequenz gemäß SEQ ID NO:3 kodierten Glyoxaloxidase Glo 1 aus *U. maydis.*

### SEQ ID NO:5

Nukleinsäuresequenz kodierend für die Glyoxaloxidase Glo2 aus *U. maydis* (cDNA).

### SEQ ID NO:6

Aminosäuresequenz der von der Sequenz gemäß SEQ ID NO:5 kodierten Glyoxaloxidase Glo2 aus *U. maydis.*

### SEQ ID NO:7

Nukleinsäuresequenz kodierend für die Glyoxaloxidase Glo3 aus *U. maydis* (cDNA).

### SEQ ID NO:8

Aminosäuresequenz der von der Sequenz gemäß SEQ ID NO:7 kodierten Glyoxaloxidase Glo3 aus *U. maydis.*

### SEQ ID NO:9

Nukleinsäuresequenz kodierend für die Glyoxaloxidase aus *B. cinerea* (cDNA).

### SEQ ID NO:10

Aminosäuresequenz der von der Sequenz gemäß SEQ ID NO:9 kodierten Glyoxaloxidase aus *B*. *cinerea*

### SEQ ID NO:11

Nukleinsäuresequenz kodierend für die Glyoxaloxidase aus *B. cinerea* (genomische DNA enthaltend zwei Exons, Exon 1 und Exon 2, sowie ein Intron).

### SEQ ID NO:12

Aminosäuresequenz der von der Sequenz gemäß SEQ ID NO:11 kodierten Glyoxaloxidase aus *B*. *cinerea* (die Exons 1 und 2 wurden hier zusammengefügt).

### Literatur

1. Altschul, S.F., Madden, T.L., Schaffer, A.A., Zhang, J.Z.; Miller W. und Lipman, D.J. (1997), Gapped BLAST und PSI-BLAST generation of protein database search programs. Nucleic Acids Res. 25: 3389-3402.
2. Benito, E.P., ten Have, A., van't Klooster, J.W. und van Kan, J.A.L. (1998), Fungal and plant gene expression during synchronized infection of tomato leaves by *Botrytis cinerea.* European Journal Plant Pathalogy 104, 207-200.
3. Bevan, M. (1984), Binary Agrobacterium vectors for plant transformation. Nucleic Acids Res 12(22): 8711-8721.
4. Bevan, M., Flavell, R.B., and Chilton, M.-D. (1983), A chimeric antibiotic resistance gene as a selectable marker for plant cell transformation. Nature 304, 184.
5. Bölker, M., Böhnert, H., U., Braun, K-H., Görl, J. und Kahmann, R. (1995), Tagging pathogenicity genes in *Ustilago maydis* by restriction enzyme-mediated integration (REMI). Mol.Gen.Genet. 248: 547-552.
6. Bottin, A., Kämper, J., Kahmann, R. (1996), Isolation of a carbon source-regulated gene from *Ustilago maydis.* Mol. Gen. Genet. 253, 342-352.
7. Gietz RD, Triggs-Raine B, Robbins A, Graham KC, Woods RA. (1997), Identification of proteins that interact with a protein of interest: applications of the yeast two-hybrid system. Mol Cell Biochem. 172(1-2):67-79.
8. Gillissen, B., Bergemann, J., Sandmann, C. Schroeer, M., Bölker, M., und Kahmann, R. (1992), A two-component regulatory system for self/nonself recognition in *Ustilago maydis.* Cell, 68: 647-657.
9. Gruber, M.Y und Grosby, W.L. (1993) Meth. in Plant Mol. Biol. and Biotechnology (Glick, B. und Thompson, J.eds), CRC Press Inc., Boca Raton, Florida.
10. Holliday, R. (1974), *Ustilago maydis.* In King, R. C. (ed), Handbook of Genetics. Plenum, New York, pp. 575-595.
11. Hollomon, D.W. (1979), Specificity of ethirimol in relation to inhibition of the enzyme adenosine deaminase. Proc. Br. Crop Prot. Conf. Pests Dis., p. 251.
12. Kahmann, R. und Basse, C. (1999), REMI and its impact on the isolation of pathogenicity genes in fungi attacking plants. Europ. J. Plant Pathology 105, 221-229.
13. Kämper und Schreier (2001), Verfahren zur Herstellung von Deletionsmutanten; Deutsche Patentanmeldung Nr. 10 133 926.3.
14. Keon, J. P. R., White, G. A. und Hargraves, J. A. (1991), Isolation, characterization and sequence of a gene conferring resistance to the systemic fungicide carboxin from the maize smut pathogen *Ustilago maydis.* Curr. Genet. 19, 475-481.
15. Kersten, J.P. (1990), Glyoxal oxidase of *Phanerochaete chrysosporium:* its characterization and activation by lignin, Proc. Natl. Acad Sci U S A 87 (8), 2936-2940.
16. Kersten J. P. und Cullen D. (1993): Cloning and Characterization of a cDNA encoding glyoxal oxidase, a H₂O₂-producing enzyme from the lignin-degrading basidiomycete *Phanerochaete chrysosporium.* PNAS (90), 7411-7413.
17. Kersten, J. P., Witek, C., Vanden Wymelenberg, A., Cullen, D. (1995) *Phanerochaete chrysosporium* Glyoxal Oxidase Is Encoded by Two Allelic Variants: Structure, Genomic Organization, and Heterologous Expression of *glx1* and *glx2,* J. Bact. 172, 6106-6110.
18. Kersten, J. P. und Kirk, K. (1987): Involvement of a New Enzyme, Glyoxal Oxidase, in Extracellular H₂O₂ Production by *Phanerochaete chrysosporium.* J. Bact. 169, 2195-2201.
19. Lottspeich, F., Zorbas H. (Hrsg.). (1998), Bioanalytik. Spektrum Akademischer Verlag, Heidelberg, Berlin.
20. Miki et al. (1993), Procedure for Introducing Foreign DNA into plants, in Methods in Plant Molecular Biology and Biotechnology (Glick, B. and Thompson, J., eds.), CRC Press Inc., Boca Raton, Florida.
21. Minet, M., Dufour, M.-E. und Lacroute, F. (1992), Complementation of *S. cerevisiae* auxotrophic mutants by *A. thaliana* cDNAs. Plant J. 2, 417-422.
22. Mumberg, D., Müller, R., Funk, M. (1995), Yeast vectors for the controlled expression of heterologous proteins in different genetic backgrounds. Gene 156, 119-122.
23. Sambrook, J., Fritsch, E., F. und Maniatis, T. (1989), Molecular cloning: A laboratory manual. Cold spring harbor laboratory press, Cold spring harbor, New York.
24. Schulz, B., Banuett, F., Dahl, M., Schlesinger, R., Schäfer, W., Martin, T., Herskowitz, I. und Kahmann, R. (1990), The *b* alleles of *Ustilago maydis,* whose combinations program pathogenic development, code for polypeptides containing a homeodomain-related motif. Cell 60, 295-306.
25. Spellig, T., Bölker, M., Lottspeich, F., Frank, R., W. und Kahmann, R. (1994): Pheromones trigger filamentous growth in *Ustilago maydis.* EMBO J., 13, 1620-1627.
26. Spellig, T., Bottin, A., Kahmann, R. (1996): Green fluorescent protein (GFP) as a new vital marker in the phytopathogenic fungus *Ustilago maydis.* Mol. Gen. Genet. 252, 503-509.
27. ten Have, Mulder, W., Visser, J. und van Kan, J.A.L. (1998), The endopolygalacturonasegene *Bcpgl* is required for full virulence of *Botrytis cinerea.* Molecular Plant-Microbe Interactions 11, 1009-1016.
28. Thomson, J., D., Higgins, D., G. and Gibson, T., J., (1994), CLUSTAL W: Improving the sensitivity of a progressive multiple sequence alignment through sequence weighting, position specific gap penalties and weight matrix choice. Nucleic Acids Res., 22, 4673-4680.
29. Thomalley P.J., Jahan I., Ng R. (2001), Suppression of the Accumulation of Triosephosphates and Increased Formation of Methylglyoxal in Human Red Blood Cells during Hyperglycaemia by Thiamine in Vitro. J. Biochem.129(4), 543-549.
30. Thornalley, J. P. (1996), Pharmacology of Methylglyoxal: Formation, Modification of Proteins and Nucleic Acids, and Enzymatic Detoxification: A Role in Pathogenesis and Antiproliferative Chemotherapy. Gen. Pharmac. 27(4), 565-563.
31. Tsukuda, T. et al. (1988) Isolation and characterization of an autonomously replicating sequence from *Ustilago maydis.* Mol. Cell. Biol. 8: 3703-3709.
32. Van der Vlugt-Bergmans, C.J.B., Wagemakers, C.A.M. und van Kan, J.A.L. (1997), Cloning and expression of the cutinase A gene of *Botrytis cinerea.* Molecular Plant-Microbe Interactions 10, 21-29.
33. Van der Vlugt-Bergmans, C.J.B., Brandwagt, B.F., Wagemakers, C.A.M., Van't Klooster, J.W. und van Kan, J.A.L. (1993), Genetic variation and segregation of DNA polymorphisms in *Botrytis cinerea,* Mycological Research 97, 1193-1200.
34. Van Kan, J.A.L., Van't Klooster, J. W., Dees, D.C.T., Wagemakers, C.A.M., und van der Vlugt-Bergmans, C.J.B. (1997), Cutinase A of *Botrytis cinerea* is expressed, but not essential, during penetration of gerbera and tomato. Molecular Plant-Microbe Interactions 10, 30-38.
35. Whittaker, M., Kersten, P.J., Cullen, D. and Whittaker, J.W. (1999), Identification of catalytic residues in Glyoxaloxidase by targeted mutagenesis. J. Biol. Chem. 274, 36226-36232.
36. Whittaker, M., Kersten, P.J., Nakamura, N. Sanders-Loehr, J. (1996) Glyoxal Oxidase from *Phanerochaete chrysosporium* Is an New Radical-Copper Oxidase. J. Biol. Chem. 271(2) 681-687.

## Patentansprüche

1. Verfahren zum Auffinden von Fungiziden, umfassend die folgenden Schritte:
a) Inkontaktbringen einer Wirtszelle, die eine Glyoxaloxidase aus Pilzen exprimiert, mit einer chemischen Verbindung oder einem Gemisch von chemischen Verbindungen unter Bedingungen, die die Interaktion der chemischen Verbindung oder des Gemisches von chemischen Verbindungen mit der Glyoxaloxidase erlauben,
b) Bestimmen der chemischen Verbindung, die spezifisch an die Glyoxaloxidase bindet, und
c) Bestimmen der chemischen Verbindung, die die Aktivität der Glyoxaloxidase beeinflusst.

2. Verfahren gemäß Anspruch 1, **dadurch gekennzeichnet, dass** es sich bei den Wirtszellen um Zellen des Stammes der Hinterlegungsnummer DSM 14 509 handelt.

3. Verfahren zum Auffinden von Fungiziden, umfassend die folgenden Schritte:
a) Inkontaktbringen einer Glyoxaloxidase aus Pilzen, mit einer chemischen Verbindung oder einem Gemisch von chemischen Verbindungen unter Bedingungen, die die Interaktion der chemischen Verbindung oder des Gemisches von chemischen Verbindungen mit der Glyoxaloxidase erlauben,
b) Bestimmen der chemischen Verbindung, die spezifisch an die Glyoxaloxidase bindet, und
c) Bestimmen der chemischen Verbindung, die die Aktivität der Glyoxaloxidase beeinflusst.

4. Verfahren gemäß einem der Ansprüche 1 bis 3, **dadurch gekennzeichnet, dass** man die Aktivität der Glyoxaloxidase bestimmt, indem man die enzymatische Reaktion der Glyoxaloxidase mit der Reaktion einer Meerrettich Peroxidase koppelt.

5. Verfahren gemäß einem der Ansprüche 1 bis 3, **dadurch gekennzeichnet, dass** man die Aktivität der Glyoxaloxidase bestimmt, indem man das bei der Umsetzung des Substrats Methylglyoxal entstehende H₂O₂ mit 10-Acetyl-3,7-dihydroxyphenoxazin reagieren lässt und das dabei entstehende Resorufin nachweist.

6. Verfahren gemäß einem der Ansprüche 1 bis 5, **dadurch gekennzeichnet, dass** man die fungizide Wirkung der identifizierten Verbindungen an Pilzen testet.

7. Verfahren zum Auffinden einer Verbindung, welche die Expression von Glyoxaloxidasen aus Pilzen verändert, umfassend die folgenden Schritte:
(a) Inkontaktbringen einer Wirtszelle, enthaltend eine Nukleinsäure, die für eine Glyoxaloxidase aus einem Pilz kodiert und eine Sequenz umfasst, die ausgewählt ist aus
(i) einer Sequenz gemäß SEQ ID NO: 1, SEQ ID NO:3, SEQ ID NO: 7, SEQ ID NO:9 und SEQ ID NO:11
(ii) Sequenzen, die für ein Polypeptid kodieren, welches eine Aminosäuresequenz gemäß SEQ ID NO: 2, SEQ ID NO:4, SEQ ID NO: 8, SEQ ID NO: 10 oder SEQ ID NO:12 umfasst, und
(iii) Sequenzen, welche eine 50 %ige Identität mit den unter i) bis ii) definierten Sequenzen aufweisen,
oder von Zellen des Ustilago maydis Stammes der Hinterlegungsnummer DSM 14 509 mit einer chemischen Verbindung oder einem Gemisch von chemischen Verbindungen,
(b) Bestimmen der Glyoxaloxidasenkonzentration in der Wirtszelle oder den Zellen der Hinterlegungsnummer DSM 14 509, und
(c) Bestimmen der Verbindung, welche die Expression der Glyoxaloxidase spezifisch beeinflusst.

## Claims

1. Method of finding fungicides, comprising the following steps:
a) bringing a host cell which expresses a fungal glyoxal oxidase into contact with a chemical compound or a mixture of chemical compounds under conditions which permit the interaction of the chemical compound or the mixture of chemical compounds with the glyoxal oxidase,
b) identifying the chemical compound which binds specifically to the glyoxal oxidase, and
c) identifying the chemical compound which influences the activity of the glyoxal oxidase.

2. Method according to Claim 1, **characterized in that** the host cells are cells of the strain with the deposit number DSM 14 509.

3. Method of finding fungicides, comprising the following steps:
a) bringing a fungal glyoxal oxidase into contact with a chemical compound or a mixture of chemical compounds under conditions which permit the interaction of the chemical compound or the mixture of chemical compounds with the glyoxal oxidase,
b) identifying the chemical compound which binds specifically to the glyoxal oxidase, and
c) identifying the chemical compound which influences the activity of the glyoxal oxidase.

4. Method according to one of Claims 1 to 3, **characterized in that** the activity of the glyoxal oxidase is determined by coupling the enzymatic reaction of the glyoxal oxidase with the reaction of a horseradish peroxidase.

5. Method according to one of Claims 1 to 3, **characterized in that** the activity of the glyoxal oxidase is determined by allowing the H₂O₂, which is generated in the conversion of the substrate methyl glyoxal, with 10-acetyl-3,7-dihydroxyphenoxazine and detecting the resorufin generated in this process.

6. Method according to one of Claims 1 to 5, **characterized in that** the fungicidal activity of the identified compounds is tested on fungi.

7. Method of finding a compound which modifies the expression of fungal glyoxal oxidases, comprising the following steps:
(a) bringing a host cell comprising a nucleic acid which codes for a fungal glyoxal oxidase and which comprises a sequence selected amongst
(i) a sequence as shown in SEQ ID NO: 1, SEQ ID NO: 2, SEQ ID NO: 7, SEQ ID NO: 9 and SEQ ID NO: 11
(ii) sequences which code for a polypeptide which comprises an amino acid sequence as shown in SEQ ID NO: 2, SEQ ID NO: 4, SEQ ID NO: 8, SEQ ID NO: 10 or SEQ ID NO: 12, and
(iii) sequences which have 50% identity with the sequences defined under (i) and (ii),
or from cells of the Ustilago maydis strain of deposit number DSM 14 509 into contact with a chemical compound or a mixture of chemical compounds,
(b) identifying the glyoxal oxidase concentration in the host cell or the cells of deposit number DSM 14 509, and
(c) identifying the compound which specifically influences the expression of the glyoxal oxidase.

## Revendications

1. Méthode d'identification de fongicides, comprenant les étapes suivantes :
(a) mise en contact d'une cellule hôte, qui exprime une glyoxal-oxydase de champignons, avec un composé chimique ou un mélange de composés chimiques dans des conditions qui permettent l'interaction du composé chimique ou du mélange de composés chimiques avec la glyoxal-oxydase,
(b) identification du composé chimique qui se lie spécifiquement à la glyoxal-oxydase, et
(c) identification du composé chimique qui influe sur l'activité de la glyoxal-oxydase.

2. Méthode suivant la revendication 1, **caractérisée en ce que** les cellules hôtes sont des cellules de la souche déposée sous le numéro DSM 14 509.

3. Méthode d'identification de fongicides, comprenant les étapes suivantes :
(a) mise en contact d'une glyoxal-oxydase de champignons avec un composé chimique ou un mélange de composés chimiques dans des conditions qui permettent l'interaction du composé chimique ou du mélange de composés chimiques avec la glyoxal-oxydase,
(b) identification du composé chimique qui se lie spécifiquement à la glyoxal-oxydase, et
(c) identification du composé chimique qui influe sur l'activité de la glyoxal-oxydase.

4. Méthode suivant l'une des revendications 1 à 3, **caractérisée en ce qu'**on détermine l'activité de la glyoxal-oxydase en couplant la réaction enzymatique de la glyoxal-oxydase avec la réaction d'une peroxydase de raifort.

5. Méthode suivant l'une des revendications 1 à 3, **caractérisée en ce qu'**on détermine l'activité de la glyoxal-oxydase en faisant réagir avec la 10-acétyl-3,7-dihydroxyphénoxazine le peroxyde d'hydrogène H₂O₂ produit au cours de la réaction du méthylglyoxal formant le substrat et en détectant la résorufine qui est alors produite.

6. Méthode suivant l'une des revendications 1 à 5, **caractérisée en ce qu'**on teste l'action fongicide exercée par les composés identifiés sur des champignons.

7. Méthode d'identification d'un composé qui modifie l'expression de glyoxal-oxydases de champignons, comprenant les étapes suivantes :
(a) mise en contact, avec un composé chimique ou un mélange de composés chimiques, d'une cellule hôte contenant un acide nucléique qui code une glyoxal-oxydase d'un champignon et comprend une séquence qui est choisie entre
(i) une séquence selon SEQ ID NO: 1, SEQ ID NO: 2, SED ID NO : 7, SEQ ID NO : 9 et SEQ ID NO : 11,
(ii) des séquences codant un polypeptide qui comprend une séquence d'acides aminés selon SEQ ID NO : 2, SEQ ID NO : 4, SEQ ID NO : 8, SEQ ID NO : 10 ou SEQ ID NO : 12,
(iii) des séquences qui présentent une identité de 50 % par rapport aux séquences définies en (i) et (ii), ou de cellules de la souche d'Ustilago maydis déposée sous le numéro DSM 14 509,
(b) détermination de la concentration de glyoxal-oxydase dans la cellule hôte ou dans les cellules déposées sous le numéro DSM 14 509, et
(c) identification du composé qui influe spécifiquement sur l'expression de la glyoxal-oxydase
